# EUROPEAN PATENT APPLICATION

(11) **EP 3 722 278 A1**
(43) Date of publication of application: **14.10.2020**
(21) Application number: 18885204.0
(22) Date of filing: 03.12.2018
(51) Int. Cl.: C07C 69/732, C07C 229/30, C07C 323/58, C07F 7/18, C09K 19/12, C09K 19/14, C09K 19/16, C09K 19/18, C09K 19/20, C09K 19/30, C09K 19/32, C09K 19/34, C09K 19/38, C09K 19/54, C09K 19/56, G02F 1/13

(54) **POLYMERIZABLE POLAR COMPOUND, LIQUID CRYSTAL COMPOSITION, AND LIQUID CRYSTAL DISPLAY ELEMENT**

(30) Priority: 05.12.2017 JP 2017233831
(71) Applicant: JNC Corporation, Chiyoda-ku Tokyo 100-8105 (JP); JNC Petrochemical Corporation, Tokyo 100-0004 (JP)
(72) Inventor: YANO, Masakazu, Ichihara-shi Chiba 290-8551 (JP); YANO, Tomohiro, Ichihara-shi Chiba 290-8551 (JP); TANAKA, Hiroyuki, Ichihara-shi Chiba 290-8551 (JP); MORI, Ayako, Ichihara-shi Chiba 290-8551 (JP); TAKATA, Akihiro, Ichihara-shi Chiba 290-8551 (JP); SHIMIZU, Eiji, Ichihara-shi Chiba 290-8551 (JP)
(74) Representative: Becker, Eberhard
(86) International application number: PCT/JP2018/044354
(87) International publication number: WO 2019/111845

(57) **Abstract**

Provided is a polar compound having high chemical stability, high capability of aligning liquid crystal molecules, high solubility in a liquid crystal composition, and a large voltage holding ratio in a liquid crystal display device. A means to solve the problem is a compound represented by the following formula (1).

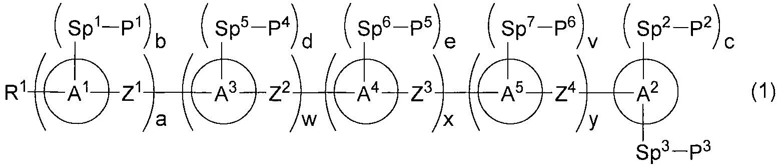

For example, R¹ is alkyl having 1 to 15 carbons; rings A¹ to A⁵ are independently cyclohexyl or phenyl; a, w, x and y are independently 1 or 2; b, d, e, v and c are independently 0 or 1, and a sum of b, d, e, v and c is 1; Z¹ to Z⁴ are independently alkylene having 1 to 6 carbons; Sp¹ to Sp³, and Sp⁵ to Sp⁷ are independently alkylene having 1 to 5 carbons; P¹ to P⁶ are independently a group represented by formula (1a); M¹ and M² are hydrogen; R² is methyl; P³ is a group represented by formula (1b); M³ and M⁴ are hydrogen; Sp⁴ is alkylene having 1 to 5 carbons; and X¹ is -OH.

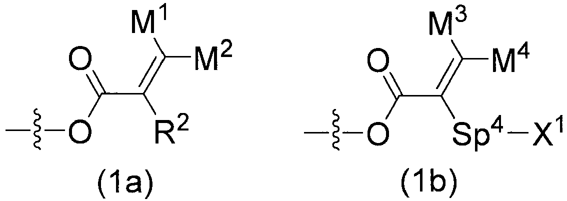

## Description

### Technical Field

The invention relates to a compound having a polymerizable group and a polar group, a liquid crystal composition and a liquid crystal display device. More specifically, the invention relates to: a compound that has a mesogen moiety formed of at least one ring, one polar group and a plurality of polymerizable groups; a liquid crystal composition that has positive or negative dielectric anisotropy and contains the compound; and a liquid crystal display device including the composition.

### Background Art

In a liquid crystal display device, a classification based on an operating mode of liquid crystal molecules includes a mode such as a phase change (PC) mode, a twisted nematic (TN) mode, a super twisted nematic (STN) mode, an electrically controlled birefringence (ECB) mode, an optically compensated bend (OCB) mode, an in-plane switching (IPS) mode, a vertical alignment (VA) mode, a fringe field switching (FFS) mode and a field-induced photo-reactive alignment (FPA) mode. A classification based on a driving mode in the device includes a passive matrix (PM) and an active matrix (AM). The PM is classified into static, multiplex and so forth, and the AM is classified into a thin film transistor (TFT), a metal insulator metal (MIM) and so forth. The TFT is further classified into amorphous silicon and polycrystal silicon. The latter is classified into a high temperature type and a low temperature type based on a production process. A classification based on a light source includes a reflective type utilizing natural light, a transmissive type utilizing backlight, and a transflective type utilizing both the natural light and the backlight.

The liquid crystal display device includes a liquid crystal composition having a nematic phase. The composition has suitable characteristics. An AM device having good characteristics can be obtained by improving characteristics of the composition. Table 1 below summarizes a relationship between two characteristics. The characteristics of the composition will be further described based on a commercially available AM device. A temperature range of the nematic phase relates to a temperature range in which the device can be used. A preferred maximum temperature of the nematic phase is about 70°C or higher, and a preferred minimum temperature of the nematic phase is about -10°C or lower. Viscosity of the composition relates to a response time in the device. A short response time is preferred for displaying moving images on the device. A shorter response time even by one millisecond is desirable. Accordingly, small viscosity in the composition is preferred. The small viscosity at low temperature is further preferred.

### Table 1

**Table 1. Characteristics of composition and AM device**

| No. | Characteristics of composition | Characteristics of AM device |
|---|---|---|
| 1 | Wide temperature range of a nematic phase | Wide usable temperature range |
| 2 | Small viscosity¹⁾ | Short response time |
| 3 | Suitable optical anisotropy | Large contrast ratio |
| 4 | Large positive or negative dielectric anisotropy | Low threshold voltage and small electric power consumption Large contrast ratio |
| 5 | Large specific resistance | Large voltage holding ratio and large contrast ratio |
| 6 | High stability to ultraviolet light and heat | Long service life |
| 7 | Large elastic constant | Large contrast ratio and short response time |

| | | |
|---|---|---|
| 1) A composition can be injected into a liquid crystal display device in a short time. | | |

Optical anisotropy of the composition relates to a contrast ratio in the device. According to a mode of the device, large optical anisotropy or small optical anisotropy, more specifically, suitable optical anisotropy is required. A product (Δn × d) of the optical anisotropy (Δn) of the composition and a cell gap (d) in the device is designed so as to maximize the contrast ratio. A suitable value of the product depends on a type of the operating mode. The value is about 0.45 micrometer in a device having a mode such as a TN mode. The value is in the range of about 0.30 micrometer to about 0.40 micrometer in a device having the VA mode, and in the range of about 0.20 micrometer to about 0.30 micrometer in a device having the IPS mode or the FFS mode. In the above case, a composition having large optical anisotropy is preferred for a device having a small cell gap. Large dielectric anisotropy in the composition contributes to low threshold voltage, small electric power consumption and a large contrast ratio in the device. Accordingly, large positive or negative dielectric anisotropy is preferred. Large specific resistance in the composition contributes to a large voltage holding ratio and the large contrast ratio in the device. Accordingly, a composition having the large specific resistance at room temperature and also at a temperature close to the maximum temperature of the nematic phase in an initial stage is preferred. The composition having the large specific resistance at room temperature and also at a temperature close to the maximum temperature of the nematic phase even after the device has been used for a long period of time is preferred. Stability of the composition to ultraviolet light and heat relates to a service life of the device. In the case where the stability is high, the device has a long service life. Such characteristics are preferred for an AM device use in a liquid crystal projector, a liquid crystal television and so forth.

In a liquid crystal display device having a polymer sustained alignment (PSA) mode, a liquid crystal composition comprising a polymer is used. First, a composition to which a small amount of a polymerizable compound is added is injected into the device. Next, the composition is irradiated with ultraviolet light while voltage is applied between substrates of the device. The polymerizable compound is polymerized to form a network structure of the polymer in the composition. Here, a polymerizable compound that has a plurality of polymerizable groups is generally used. In the composition, alignment of the liquid crystal molecules can be controlled by the polymer, and therefore the response time in the device is shortened and also image persistence is improved. Such an effect of the polymer can be expected for a device having the mode such as the TN mode, the ECB mode, the OCB mode, the IPS mode, the VA mode, the FFS mode and the FPA mode.

In a general-purpose liquid crystal display device, a vertical alignment of liquid crystal molecules is achieved by a polyimide alignment film. On the other hand, as a liquid crystal display device having no alignment film, a polar compound is added to a liquid crystal composition, and the mode in which liquid crystal molecules are made to align is proposed. First, a composition to which a small amount of the polar compound and a small amount of the polymerizable compound are added is injected into the device. Herein, the polymerizable compound which has a plurality of polymerizable groups is used in general. Here, liquid crystal molecules are aligned by action of the polar compounds. Next, the composition is irradiated with ultraviolet light while voltage is applied between substrates of the device. Here, the polymerizable compound is polymerized to stabilize alignment of the liquid crystal molecules. In the composition, alignment of liquid crystal molecules can be controlled by the polar compound and the polymer, and therefore the response time of the device is shortened, and image persistence is improved. Further, in the device having no alignment film, a step of forming an alignment film is unnecessary. The device has no alignment film, and therefore electric resistance of the device is not decreased by interaction between the alignment film and the composition. Such an effect due to a combination of the polar compound and the polymer can be expected for a device having the mode such as the TN mode, the ECB mode, the OCB mode, the IPS mode, the VA mode, the FFS mode and the FPA mode.

In a liquid crystal display device having no alignment film, the polar compound which has polymerizability has so far been synthesized as a compound which combines action of the polar compound and action of the polymerizable compound. Patent literature No. 1 describes the polymerizable compound (S-1) which has a plurality of polar groups and a plurality of polymerizable groups.

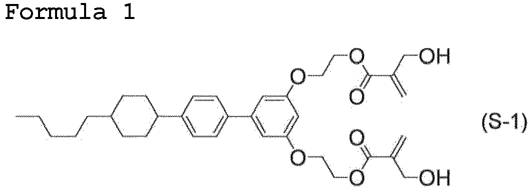

### Citation List

### Patent Literatures

Patent literature No. 1: WO 2017/047177 A.
Patent literature No. 2: WO 2016/129490 A.
Patent literature No. 3: WO 2014/090362 A.
Patent literature No. 4: WO 2014/094959 A.
Patent literature No. 5: WO 2013/004372 A.
Patent literature No. 6: WO 2012/104008 A.
Patent literature No. 7: WO 2012/038026 A.
Patent literature No. 8: JP S50-35076 A.

### Summary of Invention

### Technical Problem

A first object of the invention is to provide a compound having at least one of high chemical stability, high capability of aligning liquid crystal molecules, high polymerization reactivity by irradiation with ultraviolet light, and a large voltage holding ratio when used in a liquid crystal display device, and further having high solubility in a liquid crystal composition.

A second object is to provide a liquid crystal composition comprising the compound and satisfying at least one of characteristics such as high maximum temperature of a nematic phase, low minimum temperature of the nematic phase, small viscosity, suitable optical anisotropy, large positive or negative dielectric anisotropy, large specific resistance, high stability to ultraviolet light, high stability to heat and a large elastic constant.

A third object is to provide a liquid crystal display device including the composition and having at least one of characteristics such as a wide temperature range in which the device can be used, a short response time, a high transmittance, a large voltage holding ratio, low threshold voltage, a large contrast ratio, a long service life and good vertical alignment properties.

### Solution to Problem

The invention concerns a compound represented by formula (1), a liquid crystal composition comprising the compound, and a liquid crystal display device including the composition.

A compound, represented by formula (1): 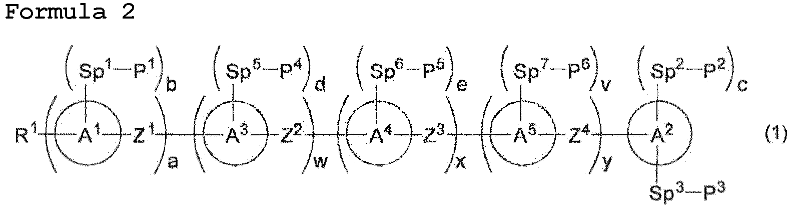 wherein, in formula (1),
R¹ is hydrogen or alkyl having 1 to 15 carbons, and in the alkyl, at least one piece of -CH₂- may be replaced by -O- or -S-, and at least one piece of -(CH₂)₂- may be replaced by -CH=CH- or -C=C-, and in the groups, at least one hydrogen may be replaced by fluorine or chlorine;
ring A¹, ring A³, ring A⁴, ring A⁵ and ring A² are independently cyclopropyl, cyclobutyl, cyclopentyl, cyclohexyl, cycloheptyl, cyclohexenyl, phenyl, 2-naphthyl, decahydronaphthalene-2-yl, 1,2,3,4-tetrahydronaphthalene-2-yl, 1,2,3,4-tetrahydronaphthalene-6-yl, tetrahydropyran-2-yl, 1,3-dioxane-2-yl, pyrimidine-2-yl or pyridine-2-yl, and in the rings, at least one hydrogen may be replaced by fluorine, chlorine, alkyl having 1 to 10 carbons, alkenyl having 2 to 10 carbons, alkoxy having 1 to 9 carbons or alkenyloxy having 2 to 9 carbons, and in the groups, at least one hydrogen may be replaced by fluorine or chlorine;
a, w, x and y are independently 0 or 1;
b, d, e, v and c are independently 0, 1, 2, 3 or 4, and a sum of b, d, e, v and c is 1, 2, 3 or 4;
Z¹, Z², Z³ and Z⁴ are independently a single bond or alkylene having 1 to 6 carbons, and in the alkylene, at least one piece of -CH₂- may be replaced by -O-, -CO-, -COO-, -OCO- or -OCOO-, and at least one piece of -(CH₂)₂- may be replaced by -CH=CH- or -C=C-, and in the groups, at least one hydrogen may be replaced by fluorine or chlorine;
Sp¹, Sp⁵, Sp⁶, Sp⁷, Sp² and Sp³ are independently a single bond or alkylene having 1 to 10 carbons, and in the alkylene, at least one piece of -CH₂- may be replaced by -O-, -CO-, -COO-, -OCO- or -OCOO-, and at least one piece of -(CH₂)₂- may be replaced by -CH=CH- or -C=C-, and in the groups, at least one hydrogen may be replaced by fluorine or chlorine; and
P¹, P⁴, P⁵, P⁶ and P² are independently a group represented by formula (1a) ; 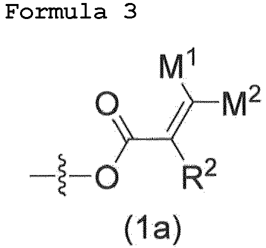
wherein, in formula (1a),
M¹ and M² are independently hydrogen, fluorine, chlorine, alkyl having 1 to 5 carbons, or alkyl having 1 to 5 carbons in which at least one hydrogen is replaced by fluorine or chlorine; and
R² is hydrogen, alkyl having 1 to 10 carbons, alkoxy having 1 to 9 carbons, alkoxyalkyl having 1 to 9 carbons, alkylthio having 1 to 9 carbons or alkylthioalkyl having 1 to 9 carbons, and in the groups, at least one piece of -(CH₂)₂- may be replaced by -CH=CH- or -C=C-, and in the groups, at least one hydrogen may be replaced by fluorine or chlorine; and
   in formula (1),
P³ is a group represented by formula (1b); 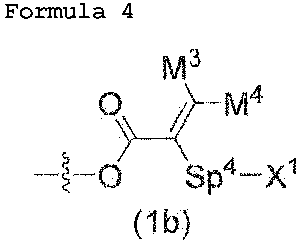
wherein, in formula (1b),
Sp⁴ is a single bond or alkylene having 1 to 10 carbons, and in the alkylene, at least one piece of -CH₂- may be replaced by -O-, -NH-, -CO-, -COO-, -OCO- or -OCOO-, and at least one piece of -(CH₂)₂- may be replaced by -CH=CH- or -C≡C-, and in the groups, at least one hydrogen may be replaced by fluorine or chlorine;
M³ and M⁴ are independently hydrogen, fluorine, chlorine, alkyl having 1 to 5 carbons, or alkyl having 1 to 5 carbons in which at least one hydrogen is replaced by fluorine or chlorine; and
X¹ is -OH, -NH₂, -OR³, -N(R³)₂, -COOH, -SH or -Si(R³)₃; and in -OR³, -N(R³)₂ and -Si(R³)₃,
R³ is hydrogen or alkyl having 1 to 10 carbons, and in the alkyl, at least one piece of -CH₂- may be replaced by -O-, and at least one piece of -(CH₂)₂- may be replaced by -CH=CH-, and in the groups, at least one hydrogen may be replaced by fluorine or chlorine, and when R³ is plural, each thereof may be identical or different.

### Advantageous Effects of Invention

A first advantage of the invention is to provide a compound having at least one of high chemical stability, high capability of aligning liquid crystal molecules, high polymerization reactivity by irradiation with ultraviolet light, and a large voltage holding ratio when used in a liquid crystal display device, and further having high solubility in a liquid crystal composition.

A second advantage is to provide a liquid crystal composition comprising the compound and satisfying at least one of characteristics such as high maximum temperature of a nematic phase, low minimum temperature of the nematic phase, small viscosity, suitable optical anisotropy, large positive or negative dielectric anisotropy, large specific resistance, high stability to ultraviolet light, high stability to heat and a large elastic constant.

A third advantage is to provide a liquid crystal display device including the composition and having at least one of characteristics such as a wide temperature range in which the device can be used, a short response time, a high transmittance, a large voltage holding ratio, low threshold voltage, a large contrast ratio, a long service life and good vertical alignment properties.

### Description of Embodiments

Hereinafter, embodiments of the invention will be described in detail. The embodiments described below are one example (representative example) of the embodiments of the invention, and the invention is not limited thereto. Moreover, the invention can be arbitrarily modified and performed within the range without departing from the spirit.

Usage of terms herein is as described below. Terms "liquid crystal compound," "liquid crystal composition" and "liquid crystal display device" may be occasionally abbreviated as "compound," "composition" and "device," respectively. "Liquid crystal compound" is a generic term for a compound having a liquid crystal phase such as a nematic phase and a smectic phase, and a compound having no liquid crystal phase but to be added for the purpose of adjusting physical properties of a composition, such as maximum temperature, minimum temperature, viscosity and dielectric anisotropy. The compound has a six-membered ring such as 1,4-cyclohexylene and 1,4-phenylene, and has rod-like molecular structure. "Liquid crystal display device" is a generic term for a liquid crystal display panel and a liquid crystal display module. "Polymerizable compound" is a compound to be added for the purpose of forming a polymer in the composition. A liquid crystal compound having alkenyl is not polymerizable in the sense. "Polar compound" helps alignment of liquid crystal molecules by interaction of a polar group with a substrate surface.

The liquid crystal composition is prepared by mixing a plurality of liquid crystal compounds. An additive is added to the composition for the purpose of further adjusting the physical properties. An additive such as a polymerizable compound, a polymerization initiator, a polymerization inhibitor, an optically active compound, an antioxidant, an ultraviolet light absorber, a light stabilizer, a heat stabilizer, a dye and an antifoaming agent is added when necessary. The liquid crystal compound and the additive are mixed in such a procedure. A proportion (content) of the liquid crystal compounds is expressed in terms of weight percent (% by weight) based on the weight of the liquid crystal composition comprising no additive, even after the additive has been added. A proportion (amount of addition) of the additive is expressed in terms of weight percent (% by weight) based on the weight of the liquid crystal composition comprising no additive. More specifically, the proportion of the liquid crystal compound or the additive is calculated based on the total weight of the liquid crystal compound. Weight parts per million (ppm) may be occasionally used. A proportion of the polymerization initiator and the polymerization inhibitor is exceptionally expressed based on the weight of the polymerizable compound.

"Clearing point" is a transition temperature between the liquid crystal phase and an isotropic phase in the liquid crystal compound. "Minimum temperature of the liquid crystal phase" is a transition temperature between a solid phase and the liquid crystal phase (the smectic phase, the nematic phase or the like) in the liquid crystal compound. "Maximum temperature of the nematic phase" is a transition temperature between the nematic phase and the isotropic phase in a mixture of the liquid crystal compound and a base liquid crystal or in the liquid crystal composition, and may be occasionally abbreviated as "maximum temperature." "Minimum temperature of the nematic phase" may be occasionally abbreviated as "minimum temperature." An expression "increase the dielectric anisotropy" means that a value of dielectric anisotropy positively increases in a composition having positive dielectric anisotropy, and the value of dielectric anisotropy negatively increases in a composition having negative dielectric anisotropy. An expression "having a large voltage holding ratio" means that the device has a large voltage holding ratio at room temperature and also at a temperature close to the maximum temperature in an initial stage, and the device has the large voltage holding ratio at room temperature and also at a temperature close to the maximum temperature even after the device has been used for a long period of time. In the composition or the device, the characteristics may be occasionally examined before and after an aging test (including an acceleration deterioration test).

A compound represented by formula (1) may be occasionally abbreviated as "compound (1)." Compound (1) means one compound, a mixture of two compounds or a mixture of three or more compounds represented by formula (1). A same rule applies also to at least one compound selected from the group of compounds represented by formula (2), or the like. A symbol of A¹, B¹, C¹ or the like surrounded by a circle or a hexagonal shape corresponds to ring A¹, ring B¹ and ring C¹ or the like, respectively. The hexagonal shape represents a six-membered ring such as a cyclohexane ring and a benzene ring, or a condensed ring such as a naphthalene ring. A straight line crossing the circle or one side of the hexagonal shape represents that arbitrary hydrogen on the ring may be replaced by a group such as -Sp¹-P¹. A subscript such as c, d and e represents the number of groups subjected to replacement. When the subscript is 0(zero), no such replacement exists. In an expression "ring A and ring C are independently X, Y or Z," a subject is plural, and therefore "independently" is used. When the subject is "ring A," the subject is singular, and therefore "independently" is not used.

A symbol of terminal group R¹¹ is used in a plurality of compounds in a chemical formula of the compound. In the compounds, two groups represented by two of arbitrary R¹¹ may be identical or different. For example, in one case, R¹¹ of compound (2) is ethyl and R¹¹ of compound (3) is ethyl. In another case, R¹¹ of compound (2) is ethyl and R¹¹ of compound (3) is propyl. A same rule applies also to a symbol of R¹², R¹³, Z¹¹ or the like. In compound (8), when i is 2, two of ring D¹ exists. In the compound, two groups represented by two of ring D¹ may be identical or different. A same rule applies also to two of arbitrary ring D¹ when i is larger than 2. A same rule applies also to other symbols.

An expression "at least one of 'A'" means that the number of 'A' is arbitrary. An expression "at least one of 'A' may be replaced by 'B'" means that, when the number of 'A' is 1, a position of 'A' is arbitrary, and also when the number of 'A' is 2 or more, positions thereof can be selected without restriction. A same rule applies also to an expression "at least one of 'A' is replaced by 'B' ." An expression "at least one of A may be replaced by B, C or D" means including a case where at least one of A is replaced by B, a case where at least one of A is replaced by C, and a case where at least one of A is replaced by D, and also a case where a plurality of A are replaced by at least two of B, C and D. For example, alkyl in which at least one piece of -CH₂- (or -CH₂CH₂-) may be replaced by -O- (or -CH=CH-) includes alkyl, alkenyl, alkoxy, alkoxyalkyl, alkoxyalkenyl and alkenyloxyalkyl. In addition, a case where two pieces of consecutive -CH₂- are replaced by -O- to form -O-O- is not preferred. In alkyl or the like, a case where -CH₂- of a methyl part (-CH₂-H) is replaced by -O- to form -O-H is not preferred, either.

An expression "R¹¹ and R¹² are independently alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine" may be occasionally used. In the expression, "in the groups" may be interpreted as described therein. In the term "the groups" means alkyl, alkenyl, alkoxy, alkenyloxy or the like. More specifically, the term "groups" expresses all the groups described before the term "in the groups." The common-sense interpretation described above applies also to "in the monovalent groups" or "in the divalent groups. " For example, "the monovalent groups" expresses all the groups described before the term "in the monovalent groups."

Halogen means fluorine, chlorine, bromine or iodine. Preferred halogen is fluorine or chlorine. Further preferred halogen is fluorine. In the liquid crystal compound, alkyl is straight-chain alkyl or branched-chain alkyl, but includes no cyclic alkyl. In general, straight-chain alkyl is preferred to branched-chain alkyl. A same rule applies also to a terminal group such as alkoxy and alkenyl. With regard to a configuration of 1, 4-cyclohexylene, trans is preferred to cis for increasing the maximum temperature of the nematic phase. Then, 2-fluoro-1,4-phenylene means two divalent groups described below. In a chemical formula, fluorine may be leftward (L) or rightward (R). A same rule applies also to a left-right asymmetrical divalent group formed by removing two hydrogens from a ring, such as tetrahydropyran-2,5-diyl.

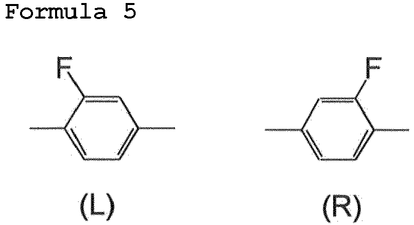

The invention includes items described below.

Item 1. A compound, represented by formula (1): 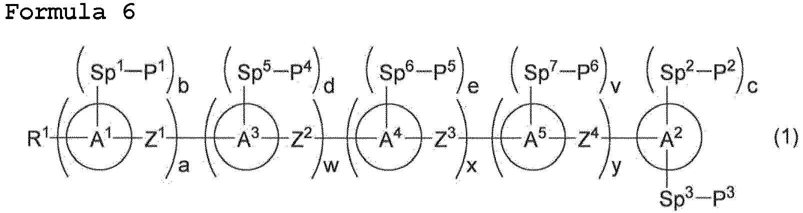 wherein, in formula (1),
R¹ is hydrogen or alkyl having 1 to 15 carbons, and in the alkyl, at least one piece of -CH₂- may be replaced by -O- or -S-, and at least one piece of -(CH₂)₂- may be replaced by -CH=CH- or -C≡C-, and in the groups, at least one hydrogen may be replaced by fluorine or chlorine;
ring A¹, ring A³, ring A⁴, ring A⁵ and ring A² are independently cyclopropyl, cyclobutyl, cyclopentyl, cyclohexyl, cycloheptyl, cyclohexenyl, phenyl, 2-naphthyl, decahydronaphthalene-2-yl, 1,2,3,4-tetrahydronaphthalene-2-yl, 1,2,3,4-tetrahydronaphthalene-6-yl, tetrahydropyran-2-yl, 1,3-dioxane-2-yl, pyrimidine-2-yl or pyridine-2-yl, and in the rings, at least one hydrogen may be replaced by fluorine, chlorine, alkyl having 1 to 10 carbons, alkenyl having 2 to 10 carbons, alkoxy having 1 to 9 carbons or alkenyloxy having 2 to 9 carbons, and in the groups, at least one hydrogen may be replaced by fluorine or chlorine;
a, w, x and y are independently 0 or 1;
b, d, e, v and c are independently 0, 1, 2, 3 or 4, and a sum of b, d, e, v and c is 1, 2, 3 or 4;
Z¹, Z², Z³ and Z⁴ are independently a single bond or alkylene having 1 to 6 carbons, and in the alkylene, at least one piece of -CH₂- may be replaced by -O-, -CO-, -COO-, -OCO- or -OCOO-, and at least one piece of -(CH₂)₂- may be replaced by -CH=CH- or -C≡C-, and in the groups, at least one hydrogen may be replaced by fluorine or chlorine;
Sp¹, Sp⁵, Sp⁶, Sp⁷, Sp² and Sp³ are independently a single bond or alkylene having 1 to 10 carbons, and in the alkylene, at least one piece of -CH₂- may be replaced by -O-, -CO-, -COO-, -OCO- or -OCOO-, and at least one piece of -(CH₂)₂- may be replaced by -CH=CH- or -C≡C-, and in the groups, at least one hydrogen may be replaced by fluorine or chlorine; and
P¹, P⁴, P⁵, P⁶ and P² are independently a group represented by formula (1a) ; 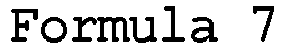 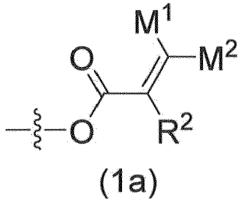
wherein, in formula (1a),
M¹ and M² are independently hydrogen, fluorine, chlorine, alkyl having 1 to 5 carbons, or alkyl having 1 to 5 carbons in which at least one hydrogen is replaced by fluorine or chlorine; and
R² is hydrogen, alkyl having 1 to 10 carbons, alkoxy having 1 to 9 carbons, alkoxyalkyl having 1 to 9 carbons, alkylthio having 1 to 9 carbons or alkylthioalkoxy having 1 to 9 carbons, and in the groups, at least one piece of -(CH₂)₂- may be replaced by -CH=CH- or -C≡C-, and in the groups, at least one hydrogen may be replaced by fluorine or chlorine; and in formula (1),
P³ is a group represented by formula (1b); 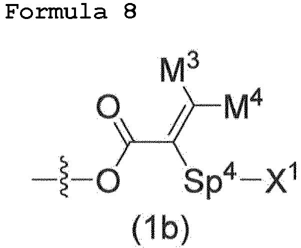
wherein, in formula (1b),
Sp⁴ is a single bond or alkylene having 1 to 10 carbons, and in the alkylene, at least one piece of -CH₂- may be replaced by -O-, -NH-, -CO-, -COO-, -OCO- or -OCOO-, and at least one piece of -(CH₂)₂- may be replaced by -CH=CH- or -C≡C-, and in the groups, at least one hydrogen may be replaced by fluorine or chlorine;
M³ and M⁴ are independently hydrogen, fluorine, chlorine, alkyl having 1 to 5 carbons, or alkyl having 1 to 5 carbons in which at least one hydrogen is replaced by fluorine or chlorine; and
X¹ is -OH, -NH₂, -OR³, -N(R³)₂, -COOH, -SH or -Si(R³)₃; and in -OR³, -N(R³) ₂ and -Si(R³)₃,
R³ is hydrogen or alkyl having 1 to 10 carbons, and in the alkyl, at least one piece of -CH₂- may be replaced by -O-, and at least one piece of -(CH₂)₂- may be replaced by -CH=CH-, and in the groups, at least one hydrogen may be replaced by fluorine or chlorine, and when R³ is plural, each thereof may be identical or different.

As a preferred aspect of the compound represented by formula (1), the items 2 to 7 are described below.

Item 2. The compound according to item 1, wherein, in formula (1), Z¹, Z², Z³ and Z⁴ are independently a single bond, - (CH₂)₂-, - (CH₂)₄-, -CH=CH-, -C≡C-, -COO-, -OCO-, -CF₂O-, -OCF₂-, -CH₂O-, -OCH₂- or -CF=CF-.

Item 3. The compound according to item 1 or 2, wherein, in formula (1), ring A¹, ring A³, ring A⁴, ring A⁵ and ring A² are independently cyclohexyl, cyclohexenyl, phenyl, 2-naphthyl, tetrahydropyran-2-yl, 1,3-dioxane-2-yl, pyrimidine-2-yl or pyridine-2-yl, and in the rings, at least one hydrogen may be replaced by fluorine, chlorine, alkyl having 1 to 10 carbons, alkenyl having 2 to 10 carbons, alkoxy having 1 to 9 carbons or alkenyloxy having 2 to 9 carbons, and in the groups, at least one hydrogen may be replaced by fluorine or chlorine.

Item 4. The compound according to any one of items 1 to 3, represented by any one of formula (1-1) to formula (1-4): 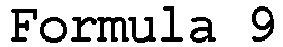 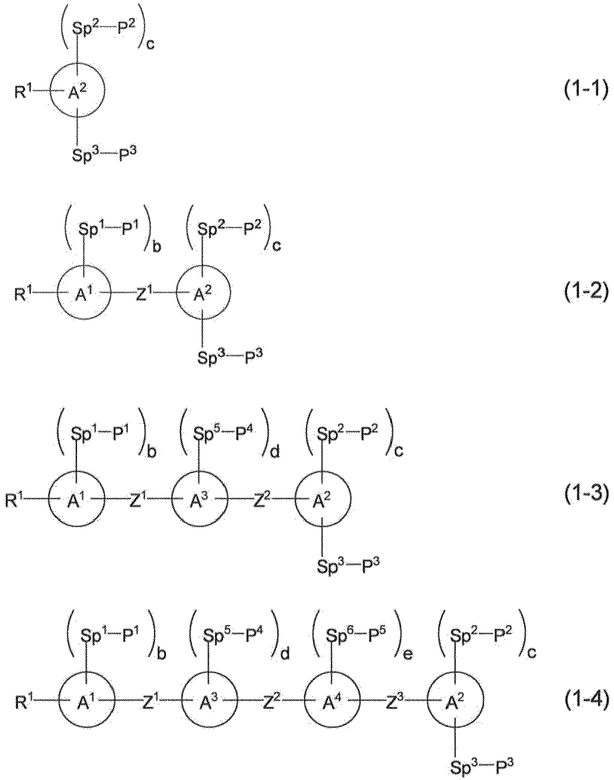 wherein, in formula (1-1) to formula (1-4),
R¹ is alkyl having 1 to 15 carbons, alkenyl having 2 to 15 carbons, alkoxy having 1 to 14 carbons, alkenyloxy having 2 to 14 carbons or alkoxyalkyl having 1 to 14, and in the groups, at least one hydrogen may be replaced by fluorine;
ring A¹, ring A³ and ring A⁴ are independently 1, 4-cyclohexylene, 1,4-cyclohexenylene, 1,4-phenylene, naphthalene-2,6-diyl, tetrahydropyran-2,5-diyl or 1,3-dioxane-2,5-diyl, and ring A² is cyclohexyl, cyclohexenyl, phenyl, 2-naphthyl, tetrahydropyran-2-yl or 1,3-dioxane-2-yl, and in the rings, at least one hydrogen may be replaced by fluorine, alkyl having 1 to 10 carbons, alkenyl having 2 to 10 carbons, alkoxy having 1 to 9 carbons or alkenyloxy having 2 to 9 carbons, and in the groups, at least one hydrogen may be replaced by fluorine;
b, c, d and e are independently 0, 1 or 2, and a sum of b, c, d and e is 1 or 2;
Z¹, Z² and Z³ are independently a single bond, - (CH₂)₂-, -CH=CH-, -C≡C-, -COO-, -OCO-, -CF₂O-, -OCF₂-, -CH₂O-, -OCH₂- or -CF=CF-;
Sp¹, Sp², Sp³, Sp⁵ and Sp⁶ are independently a single bond or alkylene having 1 to 7 carbons, and in the alkylene, at least one piece of -CH₂-may be replaced by -O-, -COO- or -OCO-, and at least one piece of - (CH₂)₂-may be replaced by -CH=CH-, and in the groups, at least one hydrogen may be replaced by fluorine; and
P¹, P², P⁴ and P⁵ are independently a group represented by formula (1a) ; 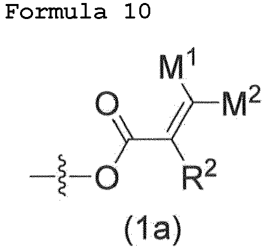
wherein, in formula (1a),
M¹ and M² are independently hydrogen, fluorine, alkyl having 1 to 5 carbons, or alkyl having 1 to 5 carbons in which at least one hydrogen is replaced by fluorine; and
R² is hydrogen, alkyl having 1 to 5 carbons, alkoxy having 1 to 4 carbons, alkoxyalkyl having 1 to 4 carbons, alkylthio having 1 to 4 carbons or alkylthioalkoxy having 1 to 4 carbons, and in the groups, at least one piece of -(CH₂)₂- may be replaced by -CH=CH-, and in the groups, at least one hydrogen may be replaced by fluorine; and
   in formula (1-1) to formula (1-4),
P³ is a group represented by formula (1b); 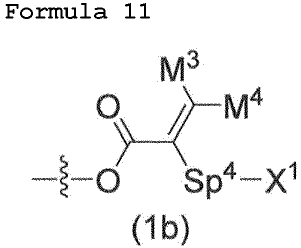
wherein, in formula (1b),
Sp⁴ is a single bond or alkylene having 1 to 7 carbons, and in the alkylene, at least one piece of -CH₂- may be replaced by -O-, -NH-, -CO-, -COO- or -OCO-, and at least one piece of - (CH₂)₂- may be replaced by -CH=CH-, and in the groups, at least one hydrogen may be replaced by fluorine;
M³ and M⁴ are independently hydrogen, fluorine, alkyl having 1 to 5 carbons, or alkyl having 1 to 5 carbons in which at least one hydrogen is replaced by fluorine; and
X¹ is -OH, -NH₂ or -SH.

Item 5. The compound according to any one of items 1 to 4, represented by any one of formula (1-5) to formula (1-16): 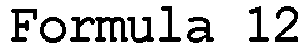 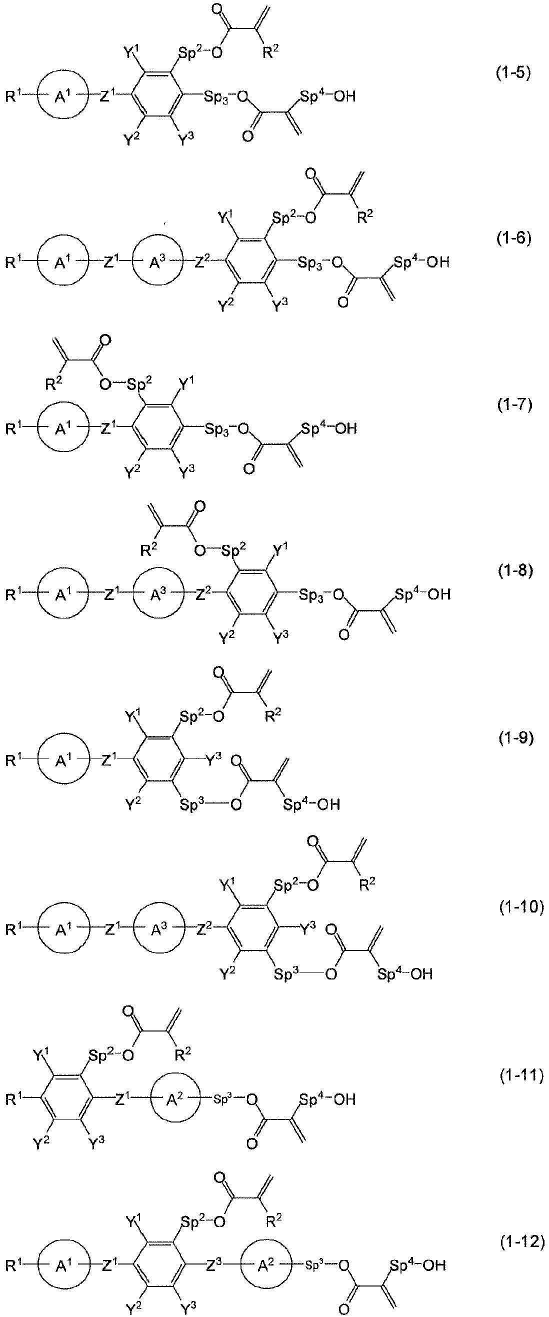 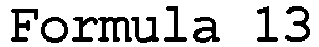 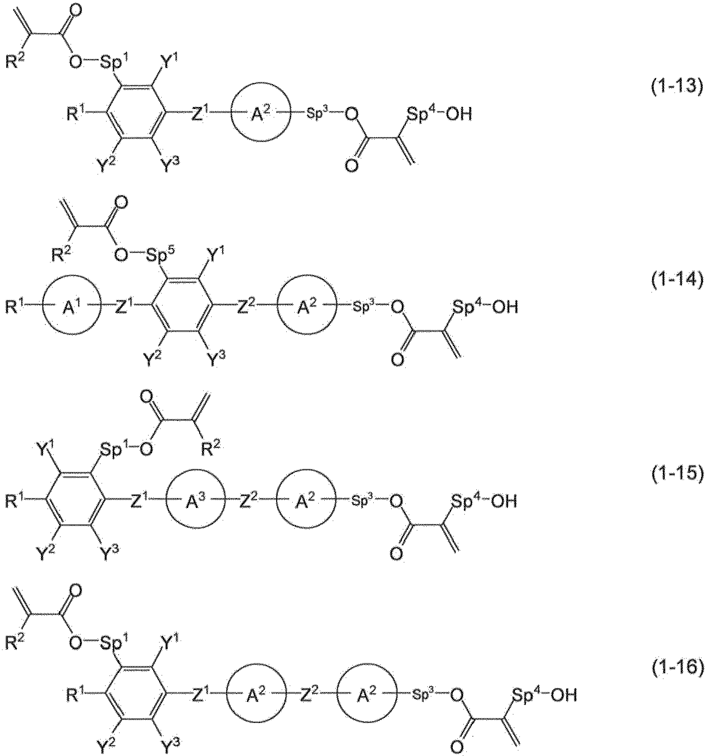 wherein, in formula (1-5) to formula (1-16),
R¹ is alkyl having 1 to 10 carbons, alkenyl having 2 to 10 carbons or alkoxy having 1 to 9 carbons;
ring A¹, ring A² and ring A³ are independently 1,4-cyclohexylene, 1,4-cyclohexenylene, 1,4-phenylene, naphthalene-2,6-diyl, tetrahydropyran-2,5-diyl or 1,3-dioxane-2,5-diyl, and in the rings, at least one hydrogen may be replaced by fluorine, alkyl having 1 to 5 carbons, alkenyl having 2 to 5 carbons or alkoxy having 1 to 4 carbons;
Y¹, Y² and Y³ are independently hydrogen, fluorine, alkyl having 1 to 5 carbons, alkenyl having 2 to 5 carbons or alkoxy having 1 to 4 carbons;
Z¹ and Z² are independently a single bond, - (CH₂)₂-, -CH=CH-, -C=C-, -CH₂O- or -OCH₂-;
Sp¹, Sp², Sp³ and Sp⁵ are independently a single bond or alkylene having 1 to 5 carbons, and in the alkylene, at least one piece of -CH₂-may be replaced by -O-, and at least one piece of - (CH₂)₂- may be replaced by -CH=CH-;
R² is hydrogen, alkyl having 1 to 5 carbons, alkoxy having 1 to 4 carbons, alkoxyalkyl having 1 to 4 carbons, alkylthio having 1 to 4 carbons or alkylthioalkoxy having 1 to 4 carbons; and
Sp⁴ is a single bond or alkylene having 1 to 5 carbons, and in the alkylene, at least one piece of -CH₂- may be replaced by -O-, -NH-, -CO-, -COO- or -OCO-, and at least one piece of - (CH₂)₂- may be replaced by -CH=CH-.

Item 6. The compound according to any one of items 1 to 5, represented by any one of formula (1-17) to formula (1-39): 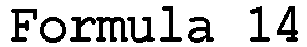 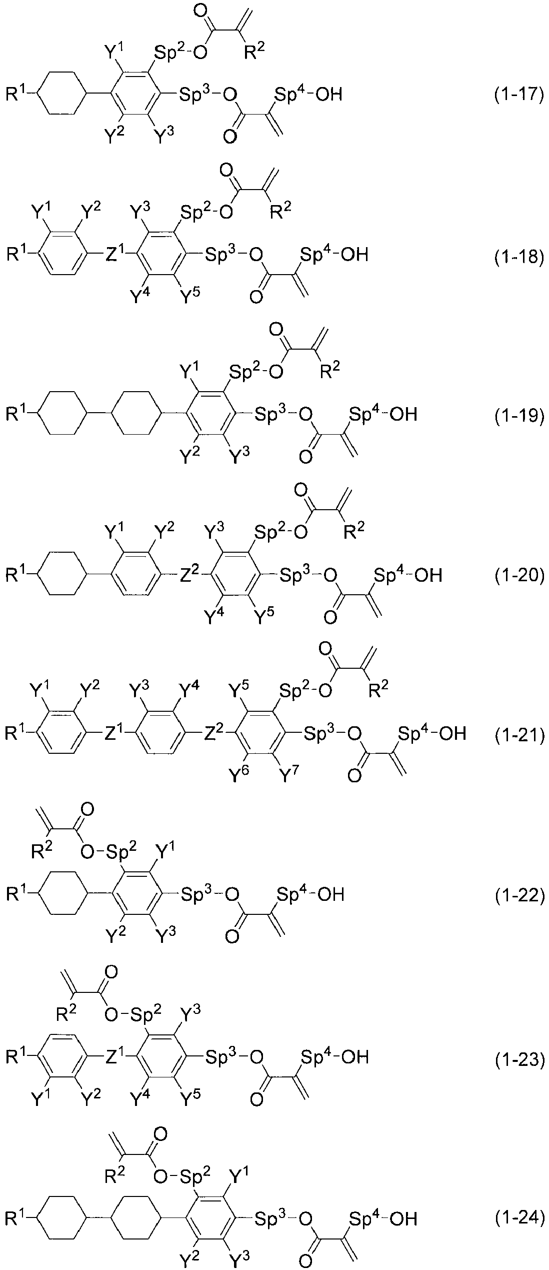 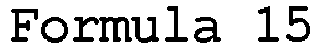 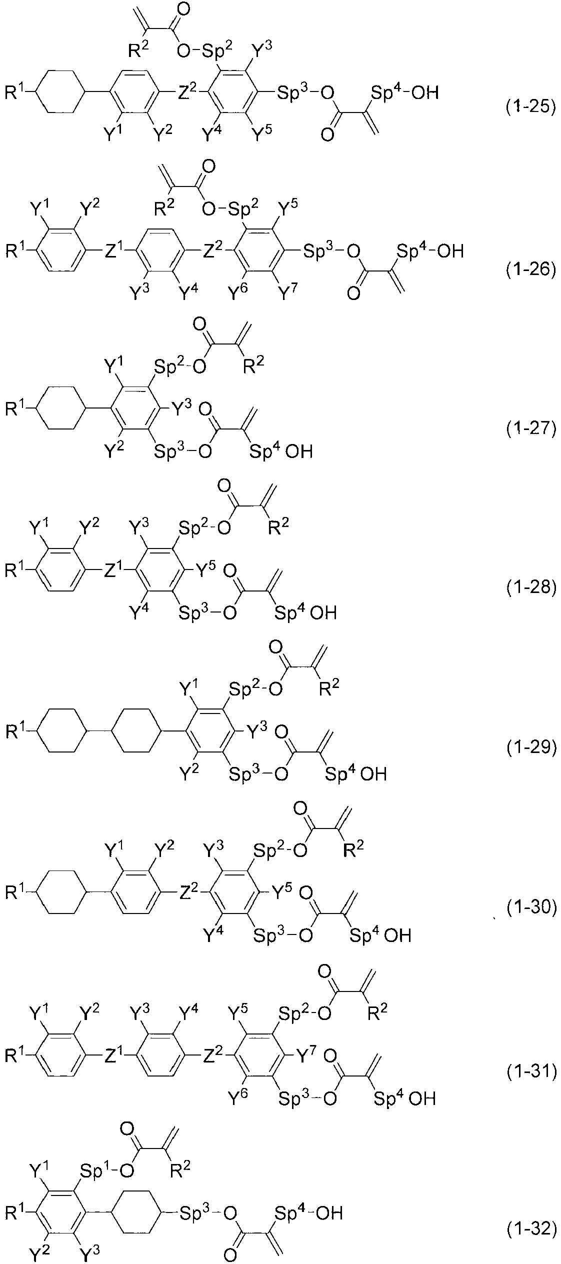 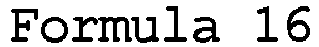 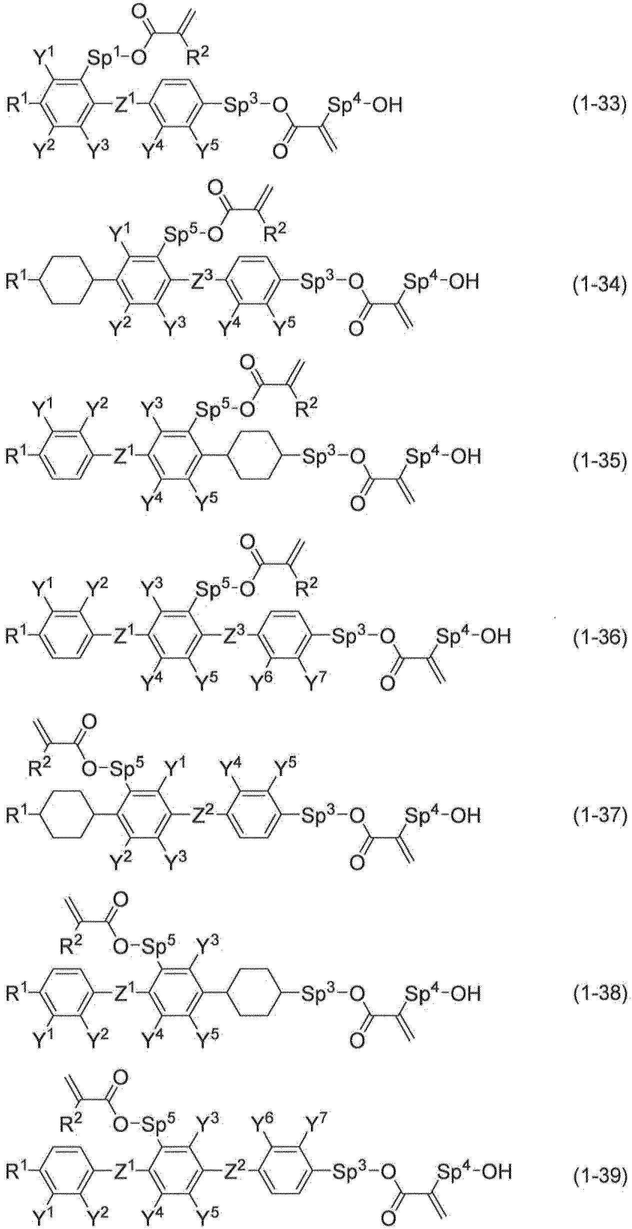 wherein, in formula (1-17) to formula (1-39),
R¹ is alkyl having 1 to 10 carbons, alkenyl having 2 to 10 carbons or alkoxy having 1 to 9 carbons;
Y¹, Y², Y³, Y⁴, Y⁵, Y⁶ and Y⁷ are independently hydrogen, fluorine, or alkyl having 1 to 5 carbons;
Z¹, Z² and Z³ are independently a single bond or -(CH₂)₂-;
Sp¹, Sp², Sp³, Sp⁴ and Sp⁵ are independently a single bond or alkylene having 1 to 5 carbons, and in the alkylene, at least one piece of -CH₂-may be replaced by -O-; and
R² is hydrogen, alkyl having 1 to 3 carbons, alkoxy having 1 or 2 carbons, alkoxyalkyl having 1 or 2 carbons, alkylthio having 1 or 2 carbons or alkylthioalkoxy having 1 or 2 carbons.

Item 7. The compound according to any one of items 1 to 6, represented by any one of formula (1-40) to formula (1-47): 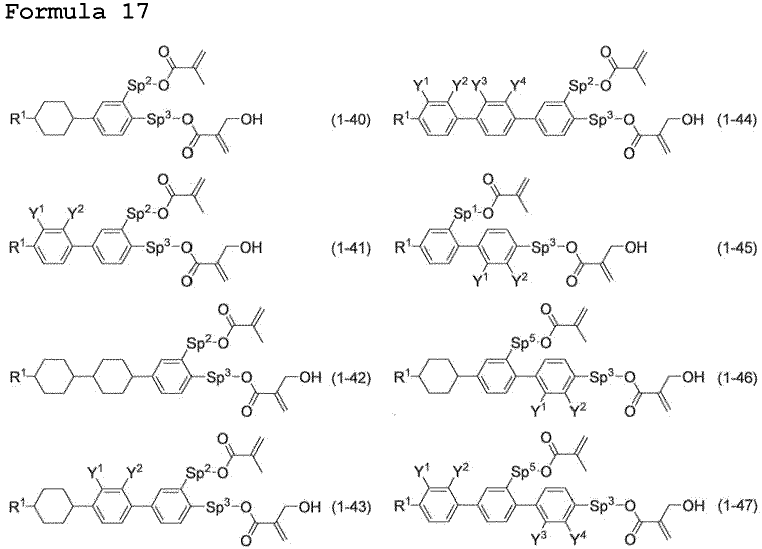 wherein, in formula (1-40) to formula (1-47),
R¹ is alkyl having 1 to 10 carbons;
Y¹, Y², Y³ and Y⁴ are independently hydrogen, fluorine, methyl or ethyl; and
Sp¹, Sp², Sp³ and Sp⁵ are independently a single bond or alkylene having 1 to 5 carbons, and in the alkylene, at least one piece of -CH₂-may be replaced by -O-.

Item 8. A liquid crystal composition, comprising at least one compound according to any one of items 1 to 7.

Item 9. The liquid crystal composition according to item 8, further comprising at least one compound selected from the group of compounds represented by formulas (2) to (4): 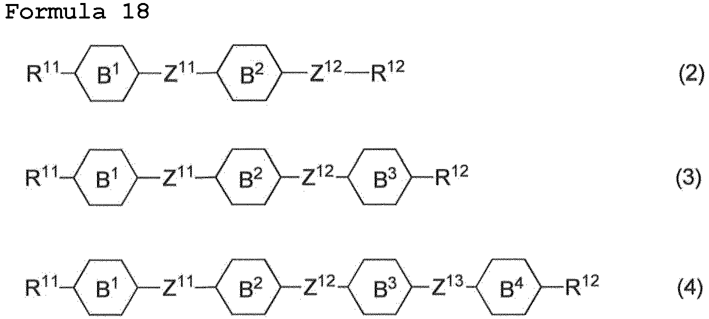 wherein, in formulas (2) to (4),
R¹¹ and R¹² are independently alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
ring B¹, ring B², ring B³ and ring B⁴ are independently 1,4-cyclohexylene, 1,4-phenylene, 2-fluoro-1,4-phenylene, 2,5-difluoro-1,4-phenylene or pyrimidine-2,5-diyl; and
Z¹¹, Z¹² and Z¹³ are independently a single bond, -COO-, -CH₂CH₂-, -CH=CH- or -C≡C-.

Item 10. The liquid crystal composition according to item 8 or 9, further comprising at least one compound selected from the group of compounds represented by formulas (5) to (7): 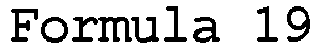 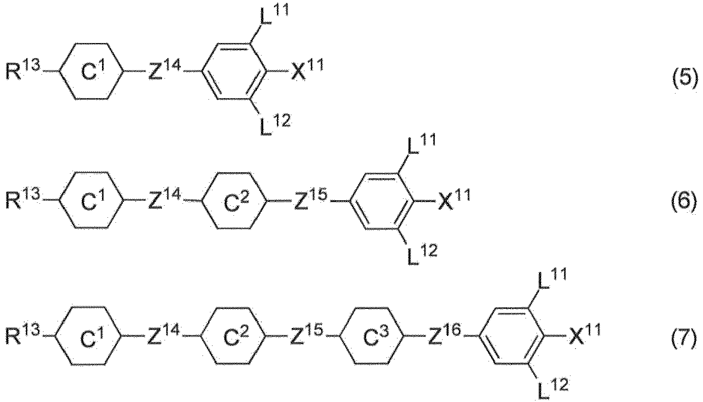 wherein, in formulas (5) to (7),
R¹³ is alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
X¹¹ is fluorine, chlorine, -OCF₃, -OCHF₂, -CF₃, -CHF₂, -CH₂F, -OCF₂CHF₂ or -OCF₂CHFCF₃;
ring C¹, ring C² and ring C³ are independently 1,4-cyclohexylene, 1,4-phenylene, 1,4-phenylene in which at least one hydrogen is replaced by fluorine, tetrahydropyran-2,5-diyl, 1,3-dioxane-2,5-diyl or pyrimidine-2,5-diyl;
Z¹⁴, Z¹⁵ and Z¹⁶ are independently a single bond, -COO-, -OCO-, -CH₂O-, -OCH₂-, -CF₂O-, -OCF₂-, -CH₂CH₂-, -CH=CH-, -C≡C- or -(CH₂)₄-; and L¹¹ and L¹² are independently hydrogen or fluorine.

Item 11. The liquid crystal composition according to any one of items 8 to 10, further comprising at least one compound represented by formula (8): 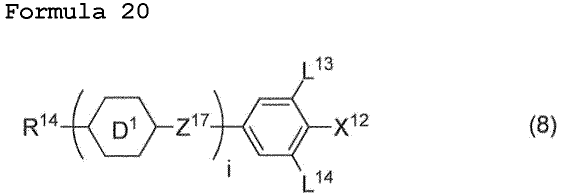 wherein, in formula (8),
R¹⁴ is alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
X¹² is -C≡N or -C≡C-C≡N;
ring D¹ is 1,4-cyclohexylene, 1,4-phenylene, 1,4-phenylene in which at least one hydrogen is replaced by fluorine, tetrahydropyran-2,5-diyl, 1,3-dioxane-2,5-diyl or pyrimidine-2,5-diyl;
Z¹⁷ is a single bond, -COO-, -OCO-, -CH₂O-,-OCH₂-, -CF₂O-, -OCF₂-, -CH₂CH₂- or -C=C-;
L¹³ and L¹⁴ are independently hydrogen or fluorine; and
i is 1, 2, 3 or 4.

Item 12. The liquid crystal composition according to any one of items 8 to 11, further comprising at least one compound selected from the group of compounds represented by formulas (11) to (19): 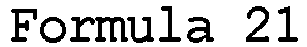 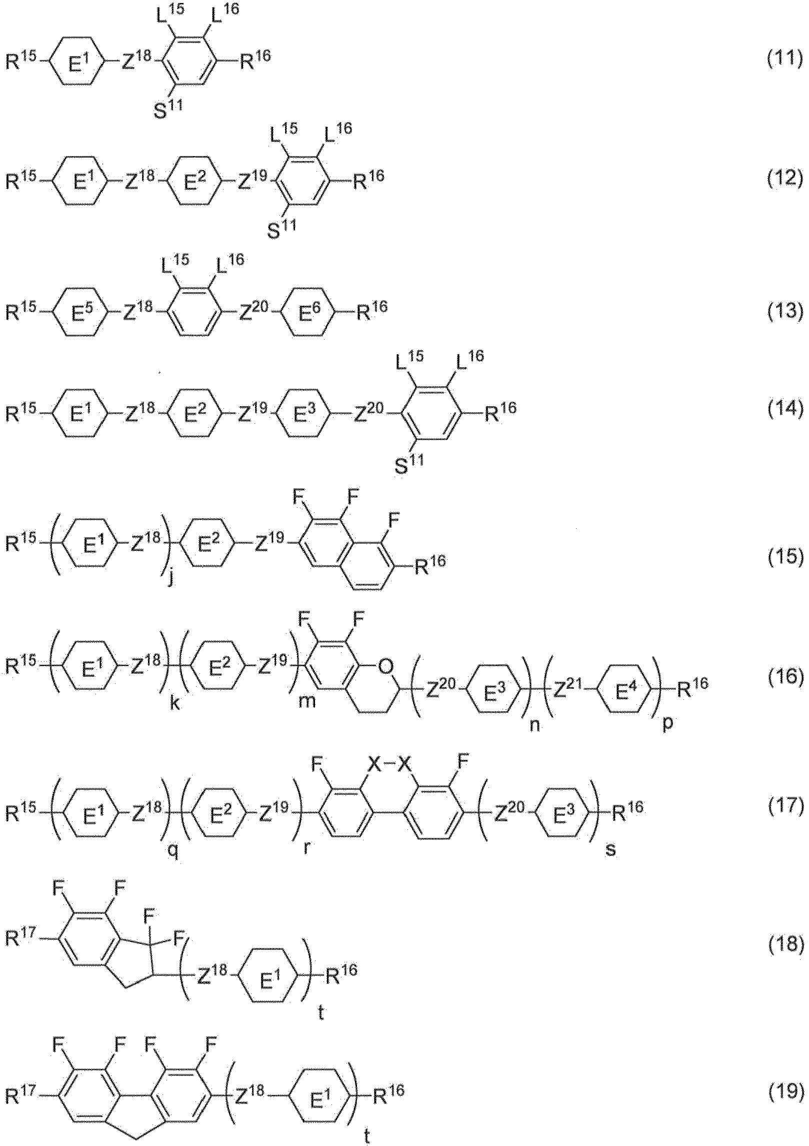 wherein, in formulas (11) to (19),
R¹⁵, R¹⁶ and R¹⁷ are independently alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine, and R¹⁷ may be hydrogen or fluorine;
ring E¹, ring E², ring E³ and ring E⁴ are independently 1,4-cyclohexylene, 1,4-cyclohexenylene, 1,4-phenylene, 1,4-phenylene in which at least one hydrogen is replaced by fluorine, tetrahydropyran-2,5-diyl or decahydronaphthalene-2,6-diyl;
ring E⁵ and ring E⁶ are independently 1,4-cyclohexylene, 1,4-cyclohexenylene, 1,4-phenylene, tetrahydropyran-2,5-diyl or decahydronaphthalene-2,6-diyl;
Z¹⁸, Z¹⁹, Z²⁰ and Z²¹ are independently a single bond, -COO-, -OCO-, -CH₂O-,-OCH₂-, -CF₂O-, -OCF₂-, -CH₂CH₂-, -CF₂OCH₂CH₂- or -OCF₂CH₂CH₂-;
L¹⁵ and L¹⁶ are independently fluorine or chlorine;
S¹¹ is hydrogen or methyl;
X is -CHF- or -CF₂-; and
j, k, m, n, p, q, r and s are independently 0 or 1, a sum of k, m, n and p is 1 or 2, a sum of q, r and s is 0, 1, 2 or 3, and t is 1, 2 or 3.

Item 13. The liquid crystal composition according to any one of items 8 to 12, further comprising at least one compound of polymerizable compounds represented by formula (20): 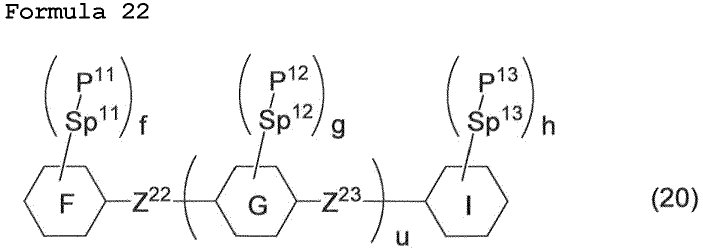 wherein, in formula (20),
ring F and ring I are independently cyclohexyl, cyclohexenyl, phenyl, 1-naphthyl, 2-naphthyl, tetrahydropyran-2-yl, 1,3-dioxane-2-yl, pyrimidine-2-yl or pyridine-2-yl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen;
ring G is 1,4-cyclohexylene, 1,4-cyclohexenylene, 1,4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl, naphthalene-2,7-diyl, phenanthrene-2,7-diyl, tetrahydropyran-2,5-diyl, 1,3-dioxane-2,5-diyl, pyrimidine-2,5-diyl or pyridine-2,5-diyl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen;
Z²² and Z²³ are independently a single bond or alkylene having 1 to 10 carbons, and in the alkylene, at least one piece of -CH₂- may be replaced by -O-, -CO-, -COO- or -OCO-, and at least one piece of -CH₂CH₂- may be replaced by -CH=CH-, -C(CH₃)=CH-, -CH=C(CH₃)- or -C(CH₃)=C (CH₃)-, and in the groups, at least one hydrogen may be replaced by fluorine or chlorine;
P¹¹, P¹² and P¹³ are independently a polymerizable group;
Sp¹¹, Sp¹² and Sp¹³ are independently a single bond or alkylene having 1 to 10 carbons, and in the alkylene, at least one piece of -CH₂- may be replaced by -O-, -COO-, -OCO- or -OCOO-, and at least one piece of -CH₂CH₂- may be replaced by -CH=CH- or -C≡C-, and in the groups, at least one hydrogen may be replaced by fluorine or chlorine;
u is 0, 1 or 2; and
f, g and h are independently 0, 1, 2, 3 or 4, and a sum of f, g and h is 1 or more.

Item 14. The liquid crystal composition according to item 13, wherein, in formula (20), P¹¹, P¹² and P¹³ are independently a group selected from the group of polymerizable groups represented by formula (P-1) to formula (P-5): 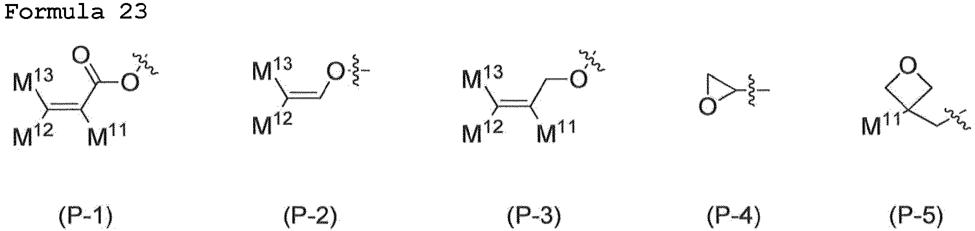 wherein, in formula (P-1) to formula (P-5),
M¹¹, M¹² and M¹³ are independently hydrogen, fluorine, alkyl having 1 to 5 carbons, or alkyl having 1 to 5 carbons in which at least one hydrogen is replaced by halogen.

Item 15. The liquid crystal composition according to item 13 or 14, wherein a polymerizable compound represented by formula (20) is at least one compound selected from the group of polymerizable compounds represented by formula (20-1) to formula (20-7): 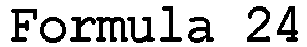 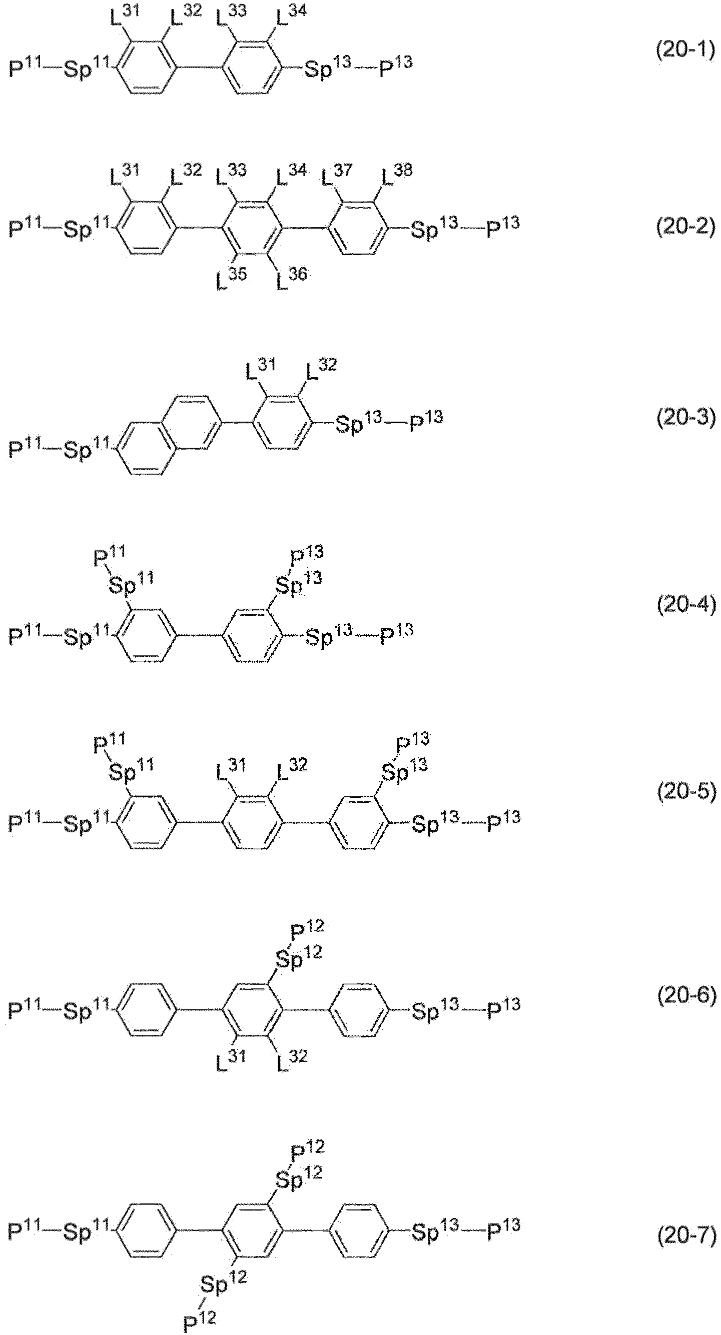 wherein, in formula (20-1) to formula (20-7),
L³¹, L³², L³³, L³⁴, L³⁵, L³⁶, L³⁷ and L³⁸ are independently hydrogen, fluorine or methyl;
Sp¹¹, Sp¹² and Sp¹³ are independently a single bond or alkylene having 1 to 10 carbons, and in the alkylene, at least one piece of -CH₂- may be replaced by -O-, -COO-, -OCO- or -OCOO-, and at least one piece of -CH₂CH₂- may be replaced by -CH=CH- or -C≡C-, and in the groups, at least one hydrogen may be replaced by fluorine or chlorine; and
P¹¹, P¹² and P¹³ are independently a group selected from the group of polymerizable groups represented by formula (P-1) to formula (P-3) ; 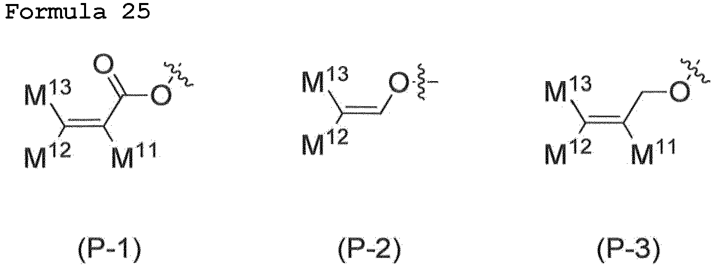
wherein, in formula (P-1) to formula (P-3),
M¹¹, M¹² and M¹³ are independently hydrogen, fluorine, alkyl having 1 to 5 carbons, or alkyl having 1 to 5 carbons in which at least one hydrogen is replaced by halogen.

Item 16. The liquid crystal composition according to any one of items 8 to 15, further comprising at least a polymerizable compound different from the compound represented by formula (1) or formula (20) described below, a polymerization initiator, a polymerization inhibitor, an optically active compound, an antioxidant, an ultraviolet light absorber, a light stabilizer, a heat stabilizer and an antifoaming agent: 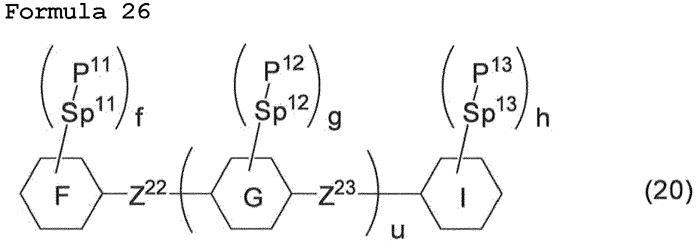 wherein, in formula (20),
ring F and ring I are independently cyclohexyl, cyclohexenyl, phenyl, 1-naphthyl, 2-naphthyl, tetrahydropyran-2-yl, 1,3-dioxane-2-yl, pyrimidine-2-yl or pyridine-2-yl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen;
ring G is 1,4-cyclohexylene, 1,4-cyclohexenylene, 1,4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl, naphthalene-2,7-diyl, phenanthrene-2,7-diyl, tetrahydropyran-2,5-diyl, 1,3-dioxane-2,5-diyl, pyrimidine-2,5-diyl or pyridine-2,5-diyl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen;
Z²² and Z²³ are independently a single bond or alkylene having 1 to 10 carbons, and in the alkylene, at least one piece of -CH₂- may be replaced by -O-, -CO-, -COO- or -OCO-, and at least one piece of -CH₂CH₂- may be replaced by -CH=CH-, -C(CH₃)=CH-, -CH=C(CH₃)- or -C (CH₃)=C (CH₃)-, and in the groups, at least one hydrogen may be replaced by fluorine or chlorine;
P¹¹, P¹² and P¹³ are independently a polymerizable group;
Sp¹¹, Sp¹² and Sp¹³ are independently a single bond or alkylene having 1 to 10 carbons, and in the alkylene, at least one piece of -CH₂- may be replaced by -O-, -COO-, -OCO- or -OCOO-, and at least one piece of -CH₂CH₂- may be replaced by -CH=CH- or -C≡C-, and in the groups, at least one hydrogen may be replaced by fluorine or chlorine;
u is 0, 1 or 2; and
f, g and h are independently 0, 1, 2, 3 or 4, and a sum of f, g and h is 1 or more.

Item 17. A liquid crystal display device, including at least one liquid crystal composition according to any one of items 8 to 16.

The invention further includes the following items:
Item (a). The liquid crystal composition, further comprising at least two of additives such as a polymerizable compound, a polymerization initiator, a polymerization inhibitor, an optically active compound, an antioxidant, an ultraviolet light absorber, a light stabilizer, a heat stabilizer and an antifoaming agent.
Item (b). A polymerizable composition, prepared by adding a polymerizable compound different from compound (1) or compound (20) to the liquid crystal composition.
Item (c). A polymerizable composition, prepared by adding compound (1) and compound (20) to the liquid crystal composition.
Item (d). A liquid crystal composite, prepared by polymerizing a polymerizable composition.
Item (e). A polymer sustained alignment mode device, including the liquid crystal composite.
Item (f). A polymer sustained alignment mode device, prepared by using a polymerizable composition prepared by adding compound (1), compound (20) and a polymerizable compound different from compound (1) or compound (20) to the liquid crystal composition.

An aspect of compound (1), synthesis of compound (1), the liquid crystal composition and the liquid crystal display device will be described in the order.

### 1. Aspect of compound (1)

Compound (1) which is one embodiment of the invention has features of having a mesogen moiety formed of at least one ring, one polar group and a plurality of polymerizable groups. The polar group noncovalently interacts with a substrate surface of glass (or metal oxide), and therefore compound (1) is useful. One of applications is the additive for the liquid crystal composition used in the liquid crystal display device. Compound (1) is added for the purpose of controlling alignment of liquid crystal molecules. Such an additive preferably has chemical stability under conditions in which the additive is sealed in the device, high capability of aligning liquid crystal molecules, a large voltage holding ratio when used in the liquid crystal display device, and further a large solubility in the liquid crystal composition. Compound (1) satisfies such characteristics to a significant extent.

Preferred examples of compound (1) will be described. Preferred examples of a symbol of R¹, A¹, Sp¹, P¹ or the like in compound (1) are applied also to a subordinate formula of formula (1) for compound (1) . In compound (1), characteristics can be arbitrarily adjusted by suitably combining kinds of the groups. Compound (1) may contain a larger amount of isotope such as ²H (deuterium) and ¹³C than an amount of natural abundance because no significant difference exists in the characteristics of the compound.

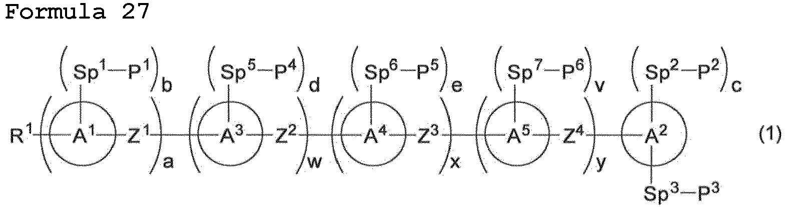

In formula (1), R¹ is hydrogen or alkyl having 1 to 15 carbons, and in the alkyl, at least one piece of -CH₂- may be replaced by -O- or -S-, at least one piece of -(CH₂)₂- may be replaced by -CH=CH- or -C≡C-, and in the groups, at least one hydrogen may be replaced by fluorine or chlorine.

Preferred R¹ is alkyl having 1 to 15 carbons, alkenyl having 2 to 15 carbons, alkoxy having 1 to 14 carbons, alkenyloxy having 2 to 14 carbons or alkoxyalkyl having 1 to 14 carbons, and in the groups, at least one hydrogen may be replaced by fluorine. Further preferred R¹ is alkyl having 1 to 10 carbons, alkenyl having 2 to 10 carbons or alkoxy having 1 to 9 carbons. Particularly preferred R¹ is alkyl having 1 to 10 carbons.

When R¹ is alkyl having 1 to 15 carbons, alkoxy having 1 to 14 carbons or alkoxyalkyl having 1 to 14 carbons, compound (1) has high chemical stability. When R¹ is alkyl having 1 to 15 carbons, alkenyl having 2 to 15 carbons or alkenyloxy having 2 to 14 carbons, compound (1) has solubility in the liquid crystal composition. When R¹ is alkyl having 1 to 15 carbons, compound (1) has high capability of aligning liquid crystal molecules.

In formula (1), ring A¹, ring A³, ring A⁴, ring A⁵ and ring A² are independently cyclopropyl, cyclobutyl, cyclopentyl, cyclohexyl, cycloheptyl, cyclohexenyl, phenyl, 2-naphthyl, decahydronaphthalene-2-yl, 1,2,3,4-tetrahydronaphthalene-2-yl, 1,2,3,4-tetrahydronaphthalene-6-yl, tetrahydropyran-2-yl, 1,3-dioxane-2-yl, pyrimidine-2-yl or pyridine-2-yl, and in the rings, at least one hydrogen may be replaced by fluorine, chlorine, alkyl having 1 to 10 carbons, alkenyl having 2 to 10 carbons, alkoxy having 1 to 9 carbons or alkenyloxy having 2 to 9 carbons, and in the groups, at least one hydrogen may be replaced by fluorine or chlorine.

Preferred ring A¹, ring A³, ring A⁴ and ring A⁵ are independently 1,4-cyclohexylene, 1,4-cyclohexenylene, 1,4-phenylene, naphthalene-2,6-diyl, tetrahydropyran-2,5-diyl or 1,3-dioxane-2,5-diyl, and in the rings, at least one hydrogen may be replaced by fluorine, alkyl having 1 to 10 carbons, alkenyl having 2 to 10 carbons, alkoxy having 1 to 9 carbons or alkenyloxy having 2 to 9 carbons, and in the groups, at least one hydrogen may be replaced by fluorine. Further preferred ring A¹, ring A³, ring A⁴ and ring A⁵ are 1,4-cyclohexylene, 1,4-cyclohexenylene, 1,4-phenylene, naphthalene-2,6-diyl, tetrahydropyran-2,5-diyl or 1,3-dioxane-2,5-diyl, and in the rings, at least one hydrogen may be replaced by fluorine, alkyl having 1 to 5 carbons, alkenyl having 2 to 5 carbons or alkoxy having 1 to 4 carbons. Particularly preferred ring A¹, ring A³, ring A⁴ and ring A⁵ are 1,4-cyclohexylene or 1,4-phenylene, and in the rings, at least one hydrogen may be replaced by fluorine or alkyl having 1 to 5 carbons.

When ring A¹, ring A³, ring A⁴ and ring A⁵ are independently 1,3-cyclopentylene, 1,4-cyclohexylene, 1,4-cycloheptylene, 1,4-phenylene, 1,4-phenylene in which at least one hydrogen is replaced by fluorine, 1,4-phenylene in which at least one hydrogen is replaced by alkyl having 1 to 5 carbons, decahydronaphthalene-2,6-diyl or tetrahydropyran-2,5-diyl, chemical stability is high. When ring A¹, ring A³, ring A⁴ and ring A⁵ are independently 1,4-cyclohexylene, 1,4-cyclohexenylene, 1,4-phenylene, 1,4-phenylene in which at least one hydrogen is replaced by fluorine, 1,4-phenylene in which at least one hydrogen is replaced by alkyl having 1 to 5 carbons, or 1, 4-phenylene in which at least one hydrogen is replaced by alkenyl having 2 to 5 carbons, solubility in a liquid crystal composition is large. When ring A¹, ring A³, ring A⁴ and ring A⁵ are independently 1, 4-cyclohexylene, 1,4-phenylene, or 1,4-phenylene in which at least one hydrogen is replaced by alkyl having 1 to 2 carbons, capability of aligning liquid crystal molecules is high. When ring A¹ is 1,4-phenylene, 1,4-phenylene in which at least one hydrogen is replaced by alkyl having 1 to 5 carbons, 1,4-phenylene in which at least one hydrogen is replaced by alkoxy having 1 to 4 carbons, naphthalene-2,6-diyl, pyrimidine-2,5-diyl or pyridine-2,5-diyl, polymerization reactivity by irradiation with ultraviolet light is high.

Preferred ring A² is cyclohexyl, cyclohexenyl, phenyl, 2-naphthyl, tetrahydropyran-2-yl, 1,3-dioxane-2-yl, pyrimidine-2-yl or pyridine-2-yl, and in the rings, at least one hydrogen may be replaced by fluorine, alkyl having 1 to 10 carbons, alkenyl having 2 to 10 carbons, alkoxy having 1 to 9 carbons or alkenyloxy having 2 to 9 carbons, and in the groups, at least one hydrogen may be replaced by fluorine. Further preferred ring A² is cyclohexyl, cyclohexenyl, phenyl, 2-naphthyl, tetrahydropyran-2-yl or 1,3-dioxane-2-yl, and in the rings, at least one hydrogen may be replaced by fluorine, alkyl having 1 to 5 carbons, alkenyl having 2 to 5 carbons or alkoxy having 1 to 4 carbons. Particularly preferred ring A² is cyclohexyl or phenyl, and in the rings, at least one hydrogen may be replaced by fluorine or alkyl having 1 to 5 carbons.

When ring A² is cyclohexyl, phenyl, phenyl in which at least one hydrogen is replaced by fluorine, phenyl in which at least one hydrogen is replaced by alkyl having 1 to 5 carbons, decahydronaphthalene-2-yl or tetrahydropyran-2-yl, chemical stability is high. When ring A² is cyclohexyl, cyclohexenyl, phenyl, phenyl in which at least one hydrogen is replaced by fluorine, phenyl in which at least one hydrogen is replaced by alkyl having 1 to 5 carbons, or phenyl in which at least one hydrogen is replaced by alkenyl having 2 to 5 carbons, solubility in a liquid crystal composition is large. When ring A² is cyclohexyl, phenyl, or phenyl in which at least one hydrogen is replaced by alkyl having 1 to 2 carbons, capability of aligning liquid crystal molecules is high. When ring A² is phenyl, phenyl in which at least one hydrogen is replaced by alkyl having 1 to 5 carbons, phenyl in which at least one hydrogen is replaced by alkoxy having 1 to 4 carbons, 2-naphthyl, pyrimidine-2-yl or pyridine-2-yl, polymerization reactivity by irradiation with ultraviolet light is high.

In formula (1), a, w, x and y are independently 0 or 1. A sum of preferred a, w, x and y is 0, 1, 2 or 3. A sum of further preferred a, w, x and y is 0, 1 or 2. A sum of particularly preferred a, w, x and y is 1 or 2.

When a sum of a, w, x and y is 0, solubility in a liquid crystal composition is large. When a sum of a, w, x and y is 3 or 4, capability of aligning liquid crystal molecules is high. When a sum of a, w, x and y is 1 or 2, solubility in a liquid crystal composition is large, capability of aligning liquid crystal molecules is high, and polymerization reactivity by irradiation with ultraviolet light is high.

In formula (1), b, d, e, v and c are independently 0, 1, 2, 3 or 4, and a sum of b, d, e, v and c is 1, 2, 3 or 4. Preferred b, d, e, v and c are independently 0, 1 or 2, and a sum of preferred b, d, e, v and c is 1, 2 or 3. A sum of further preferred b, d, e, v and c is 1 or 2. A sum of particularly preferred b, d, e, v and c is 1.

When a sum of b, d, e and v is 0 and c is 1, solubility in a liquid crystal composition is large, and capability of aligning liquid crystal molecules is high. When a sum of b, d, e and v is 1 and c is 0, solubility in a liquid crystal composition is large. When a sum of b, d, e, v and c is 2, 3 or 4, polymerization reactivity by irradiation with ultraviolet light is high.

In formula (1), Z¹, Z², Z³ and Z⁴ are independently a single bond or alkylene having 1 to 6 carbons, and in the alkylene, at least one piece of -CH₂- may be replaced by -O-, -CO-, -COO-, -OCO- or -OCOO-, and at least one piece of - (CH₂)₂- may be replaced by -CH=CH- or -C≡C-, and in the groups, at least one hydrogen may be replaced by fluorine or chlorine.

Preferred Z¹, Z², Z³ and Z⁴ are independently a single bond, - (CH₂)₂-, -CH=CH-, -C≡C-, -COO-, -OCO-, -CF₂O-, -OCF₂-, -CH₂O-,-OCH₂- or -CF=CF-. Further preferred Z¹ is a single bond or -(CH₂)₂-. Particularly preferred Z¹, Z², Z³ and Z⁴ is independently a single bond.

When Z¹, Z², Z³ and Z⁴ are independently a single bond, chemical stability is high. When Z¹, Z², Z³ and Z⁴ are independently a single bond, -(CH₂)₂-, -CF₂O- or -OCF₂-, solubility in a liquid crystal composition is large. When Z¹, Z², Z³ and Z⁴ are independently a single bond or -(CH₂)₂-, capability of aligning liquid crystal molecules is high. When Z¹, Z², Z³ and Z⁴ is independently a single bond, -CH=CH-, -C≡C-, -COO-, -OCO-, -CH₂O- or -OCH₂-, polymerization reactivity by irradiation with ultraviolet light is high.

In formula (1), Sp¹, Sp⁵, Sp⁶, Sp⁷, Sp² and Sp³ are independently a single bond or alkylene having 1 to 10 carbons, and in the alkylene, at least one piece of -CH₂- may be replaced by -O-, -CO-, -COO-, -OCO- or -OCOO-, and at least one piece of - (CH₂)₂- may be replaced by -CH=CH- or -C≡C-, and in the groups, at least one hydrogen may be replaced by fluorine or chlorine.

Preferred Sp¹, Sp⁵, Sp⁶, Sp⁷, Sp² and Sp³ are independently a single bond or alkylene having 1 to 7 carbons, and in the alkylene, at least one piece of -CH₂- may be replaced by -O-, -COO- or -OCO-, and at least one piece of -(CH₂)₂- may be replaced by -CH=CH-, and in the groups, at least one hydrogen may be replaced by fluorine. Further preferred Sp¹, Sp⁵, Sp⁶, Sp⁷, Sp² and Sp³ are independently a single bond or alkylene having 1 to 5 carbons, and in the alkylene, at least one piece of -CH₂-may be replaced by -O-, -NH-, -CO-, -COO- or -OCO-, and at least one piece of -(CH₂)₂- may be replaced by -CH=CH-. Particularly preferred Sp¹, Sp⁵, Sp⁶, Sp⁷, Sp² and Sp³ are independently a single bond or alkylene having 1 to 5 carbons, and in the alkylene, at least one piece of -CH₂-may be replaced by -O-.

When Sp¹, Sp⁵, Sp⁶, Sp⁷, Sp² and Sp³ are a single bond or alkylene having 1 to 7 carbons, chemical stability is high. When Sp¹, Sp⁵, Sp⁶, Sp⁷, Sp² and Sp³ are alkylene having 1 to 7 carbons, or alkylene having 1 to 7 carbons in which at least one piece of -CH₂- is replaced by -O-, solubility in a liquid crystal composition is large. When Sp¹, Sp⁵, Sp⁶, Sp⁷, Sp² and Sp³ are a single bond, -CH=CH- or -C≡C-, polymerization reactivity by irradiation with ultraviolet light is high. When Sp¹, Sp⁵, Sp⁶, Sp⁷ and Sp² are a single bond, capability of aligning liquid crystal molecules is high. When Sp³ is a single bond, alkylene having 1 to 5 carbons, or alkylene having 1 to 5 carbons in which at least one piece of -CH₂- is replaced by -O-, capability of aligning liquid crystal molecules is high.

In formula (1), P¹, P⁴, P⁵, P⁶ and P² are independently a group represented by formula (1a).

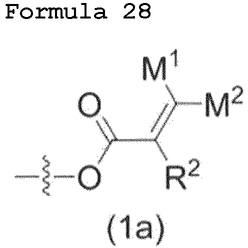

In formula (1a), M¹ and M² are independently hydrogen, fluorine, chlorine, alkyl having 1 to 5 carbons, or alkyl having 1 to 5 carbons in which at least one hydrogen is replaced by fluorine or chlorine. Preferred M¹ and M² are independently hydrogen, fluorine, alkyl having 1 to 5 carbons, or alkyl having 1 to 5 carbons in which at least one hydrogen is replaced by fluorine. Particularly preferred M¹ and M² are hydrogen. When M¹ and M² are hydrogen, polymerization reactivity by irradiation with ultraviolet light is particularly high.

In formula (1a), R² is hydrogen, alkyl having 1 to 10 carbons, alkoxy having 1 to 9 carbons, alkoxyalkyl having 1 to 9 carbons, alkylthio having 1 to 9 carbons or alkylthioalkyl having 1 to 9 carbons, and in the groups, at least one piece of -(CH₂)₂- may be replaced by -CH=CH- or -C≡C-, and in the groups, at least one hydrogen may be replaced by fluorine or chlorine. Preferred R² is hydrogen or alkyl having 1 to 5 carbons, and further preferred R² is hydrogen, alkyl having 1 to 3 carbons, alkoxy having 1 to 2 carbons or alkoxyalkyl having 1 to 2 carbons. Particularly preferred R² is methyl. When R² is methyl, chemical stability is high, and capability of aligning liquid crystal molecules is high.

In formula (1), P³ is a group represented by formula (1b).

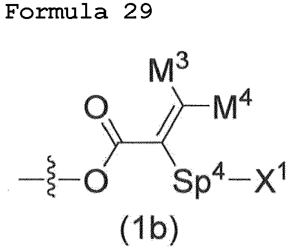

In formula (1b), Sp⁴ is a single bond or alkylene having 1 to 10 carbons, and in the alkylene, at least one piece of -CH₂- may be replaced by -O-, -NH-, -CO-, -COO-, -OCO- or -OCOO-, and at least one piece of -(CH₂)₂- may be replaced by -CH=CH- or -C≡C-, and in the groups, at least one hydrogen may be replaced by fluorine or chlorine. Preferred Sp⁴ is a single bond or alkylene having 1 to 5 carbons, and in the alkylene, at least one piece of -CH₂- may be replaced by -O-, -NH-, -CO-, -COO- or -OCO-, and at least one piece of - (CH₂)₂- may be replaced by -CH=CH-. Further preferred Sp⁴ is a single bond or alkylene having 1 to 5 carbons, and in the alkylene, at least one piece of -CH₂- may be replaced by -O-. Particularly preferred Sp⁴ is -CH₂-.

When Sp⁴ is a single bond or alkylene having 1 to 10 carbons, chemical stability is high. When Sp⁴ is alkylene having 1 to 10 carbons, or alkylene having 1 to 10 carbons in which at least one piece of -CH₂-is replaced by -O-, solubility in a liquid crystal composition is large. When Sp⁴ is a single bond, -CH=CH- or -C≡C-, polymerization reactivity by irradiation with ultraviolet light is high. When Sp⁴ is alkylene having 1 to 5 carbons, or alkylene having 1 to 5 carbons in which at least one piece of -CH₂- is replaced by -O-, capability of aligning liquid crystal molecules is high.

In formula (1b), M³ and M⁴ are independently hydrogen, fluorine, chlorine, alkyl having 1 to 5 carbons, or alkyl having 1 to 5 carbons in which at least one hydrogen is replaced by fluorine or chlorine. Preferred M³ and M⁴ are independently hydrogen, fluorine, alkyl having 1 to 5 carbons, or alkyl having 1 to 5 carbons in which at least one hydrogen is replaced by fluorine. Particularly preferred M³ and M⁴ is hydrogen. When M³ and M⁴ are hydrogen, polymerization reactivity by irradiation with ultraviolet light is particularly high.

In formula (1b), X¹ is -OH, -NH₂, -OR³, -N(R³)₂, -COOH, -SH or -Si (R³)₃, in which R³ is hydrogen or alkyl having 1 to 10 carbons, and in the alkyl, at least one piece of -CH₂- may be replaced by -O-, and at least one piece of -(CH₂)₂- may be replaced by -CH=CH-, and in the groups, at least one hydrogen may be replaced by fluorine or chlorine, and when R³ is plural, each thereof may be identical or different.

Preferred X¹ is -OH, -NH₂ or -SH. Particularly preferred X¹ is -OH. When X¹ is -OH, -NH₂ or -SH, capability of aligning liquid crystal molecules is high. When X¹ is -OH, chemical stability is high, capability of aligning liquid crystal molecules is high, and a voltage holding ratio when used in a liquid crystal display device is large.

As described above, in a liquid crystal compound having a plurality of polymerizable groups, the liquid crystal compound having polymerizable group (P3) having no polar group at a terminal in one thereof is excellent in solubility in a liquid crystal composition in comparison with polymerizable compound (S-1) having a polar group at terminals of all polymerizable groups.

Specific examples of preferred compound (1) include compounds (1-1) to (1-4) described in item 4. Specific examples of further preferred compound (1) include compounds (1-5) to (1-16) described in item 5. Specific examples of further preferred compound (1) include compounds (1-17) to (1-39) described in item 6. Specific examples of most preferred compound (1) include compounds (1-40) to (1-47) described in item 7.

Compounds (1-40), (1-41) and (1-45) are preferred from a viewpoint of large solubility in a liquid crystal composition. Compounds (1-42), (1-43), (1-44), (1-46) and (1-47) are preferred from a viewpoint of high capability of aligning liquid crystal molecules. Compounds (1-40) and (1-42) are preferred from a viewpoint of a large voltage holding ratio when used in a liquid crystal display device.

Specific examples of a component compound of the liquid crystal composition include formula (2) to formula (19) as described above. Compounds (2) to (4) have small dielectric anisotropy. Compounds (5) to (7) have large positive dielectric anisotropy. Compound (8) has a cyano group, and therefore has larger positive dielectric anisotropy. Compounds (9) to (19) have large negative dielectric anisotropy. Specific examples of the compounds are described later.

In compound (20), P¹¹, P¹² and P¹³ are independently a polymerizable group. Preferred P¹¹, P¹² or P¹³ is independently a polymerizable group selected from the group of groups represented by formula (P-1) to formula (P-5) . Further preferred P¹¹, P¹² or P¹³ is independently a group represented by formula (P-1), formula (P-2) or formula (P-3), and particularly preferably a group represented by formula (P-1) . A group represented by formula (P-1) is particularly preferably -OCO-CH=CH₂ or -OCO-C (CH₃)=CH₂. In addition, a wavy line in formula (P-1) to formula (P-5) represents a site to form a bonding.

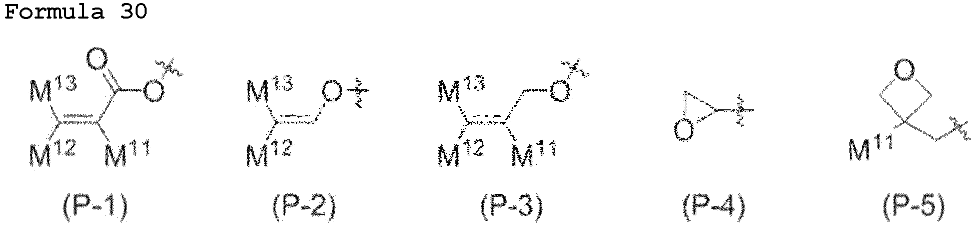

In formula (P-1) to formula (P-5) , M¹¹, M¹² and M¹³ are independently hydrogen, fluorine, alkyl having 1 to 5 carbons, or alkyl having 1 to 5 carbons in which at least one hydrogen is replaced by halogen. Preferred M¹¹, M¹² or M¹³ is independently hydrogen or methyl for increasing reactivity. Further preferred M¹¹ is methyl and further preferred M¹² or M¹³ is hydrogen.

Sp¹¹, Sp¹² and Sp¹³ are independently a single bond or alkylene having 1 to 10 carbons, and in the alkylene, at least one piece of -CH₂- may be replaced by -O-, -COO-, -OCO- or -OCOO-, and at least one piece of -(CH₂)₂- may be replaced by -CH=CH- or -C≡C-, and in the groups, at least one hydrogen may be replaced by fluorine or chlorine. Preferred Sp¹¹, Sp¹² or Sp¹³ is a single bond.

Ring F and ring I are independently cyclohexyl, cyclohexenyl, phenyl, 1-naphthyl, 2-naphthyl, tetrahydropyran-2-yl, 1,3-dioxane-2-yl, pyrimidine-2-yl or pyridine-2-yl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen. Preferred ring F or ring I is phenyl. Ring G is 1,4-cyclohexylene, 1,4-cyclohexenylene, 1,4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl, naphthalene-2,7-diyl, tetrahydropyran-2,5-diyl, 1,3-dioxane-2,5-diyl, pyrimidine-2,5-diyl or pyridine-2,5-diyl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen. Particularly preferred ring G is 1,4-phenylene or 2-fluoro-1,4-phenylene.

Z²² and Z²³ are independently a single bond or alkylene having 1 to 10 carbons, and in the alkylene, at least one piece of -CH₂- may be replaced by -O-, -CO-, -COO- or -OCO-, and at least one piece of -(CH₂)₂- may be replaced by -CH=CH-, -C(CH₃)=CH-, -CH=C(CH₃)- or -C (CH₃)=C (CH₃)-, and in the groups, at least one hydrogen may be replaced by fluorine or chlorine. Preferred Z²² or Z²³ is a single bond, - (CH₂)₂-, -CH₂O-, -OCH₂-, -COO- or -OCO-. Further preferred Z²² or Z²³ is a single bond.

Then, u is 0, 1 or 2. Preferred u is 0 or 1. Then, f, g and h are independently 0, 1, 2, 3 or 4, and a sum of f, g and h is 1 or more. Preferred f, g or h is 1 or 2.

### 2. Synthesis of compound (1)

A synthesis method of compound (1) will be described. The synthesis method described below is one example (representative example) of the embodiments of the invention, and the invention is not limited thereto. Compound (1) can be prepared by suitably combining methods in synthetic organic chemistry. Any compounds whose synthetic methods are not described are prepared according to methods described in books such as "Organic Syntheses" (John Wiley & Sons, Inc.), "Organic Reactions" (John Wiley & Sons, Inc.), "Comprehensive Organic Synthesis" (Pergamon Press) and "New Experimental Chemistry Course (Shin Jikken Kagaku Koza in Japanese)" (Maruzen Co., Ltd.).

### 2-1. Formation of a bonding group

An example of a method for forming a bonding group in compound (1) is as described in a scheme below. In the scheme, MSG¹ (or MSG²) is a monovalent organic group having at least one ring. The monovalent organic groups represented by a plurality of MSG¹ (or MSG²) may be identical or different. Compounds (1A) to (1G) correspond to compound (1) or an intermediate of compound (1).

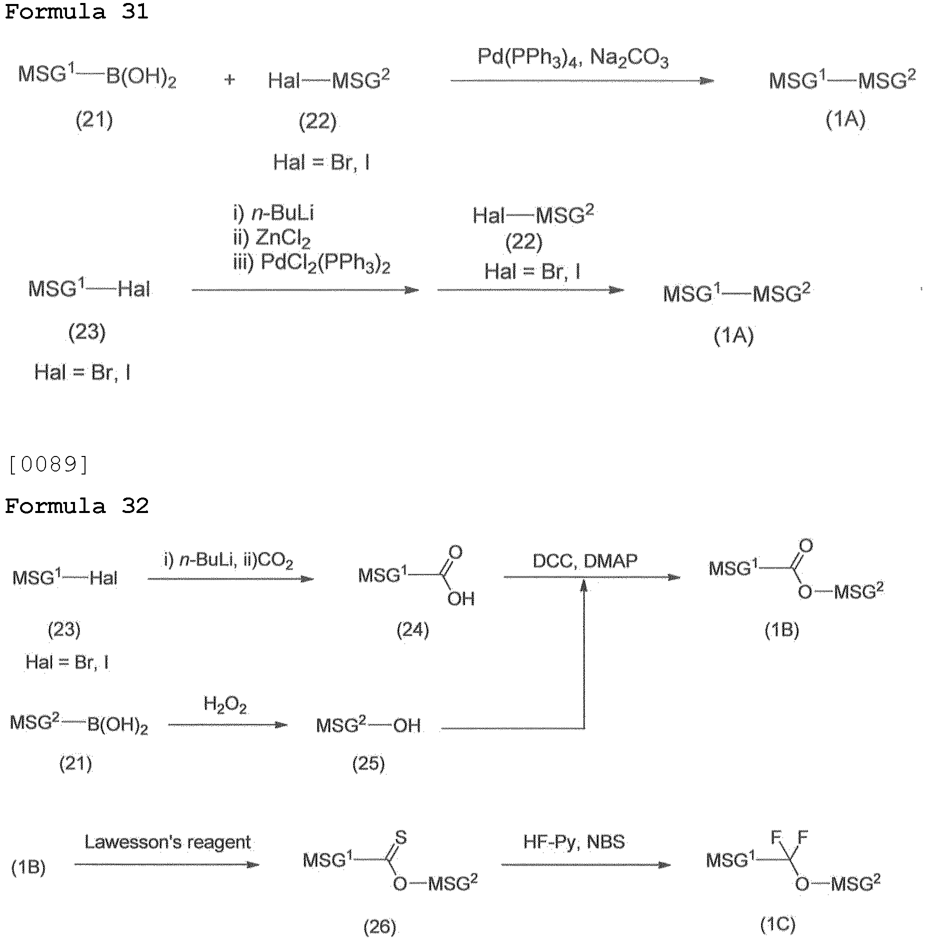

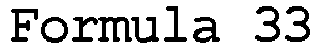

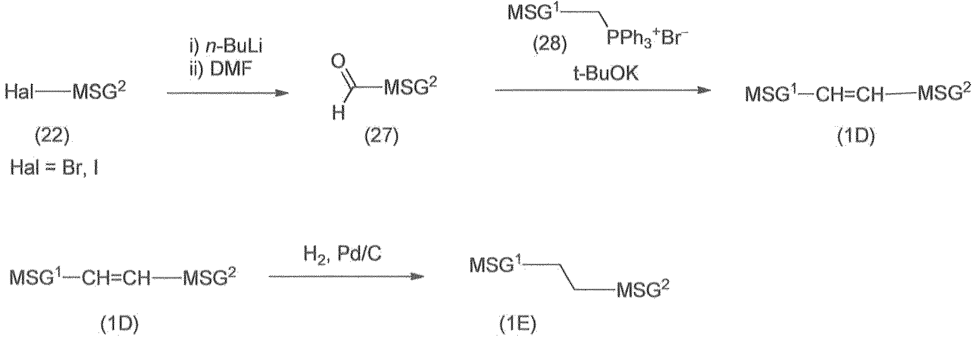

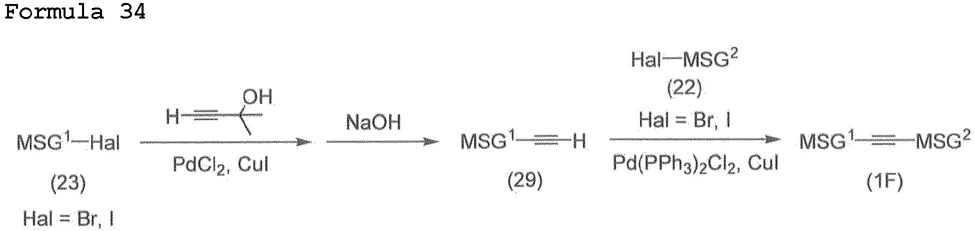

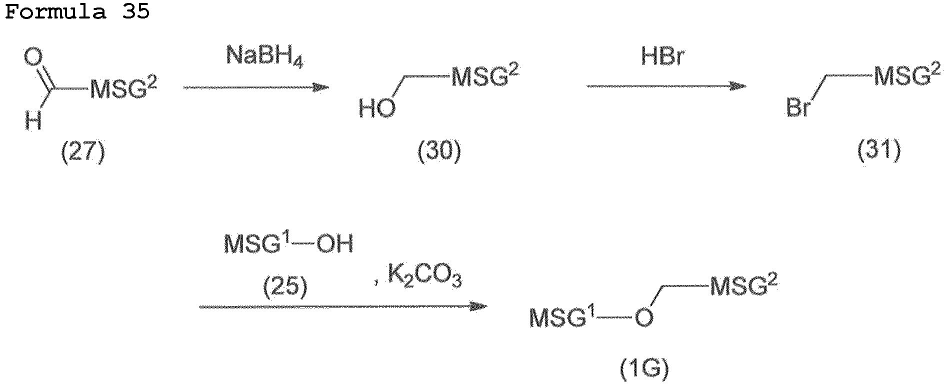

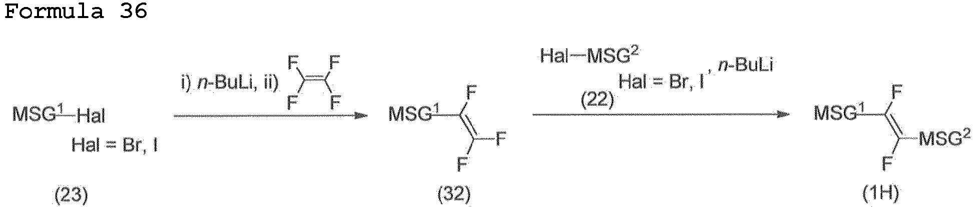

### (I) Formation of a single bond

Compound (1A) is prepared by allowing aryl boronic acid (21) to react with compound (22), in the presence of carbonate and a catalyst including tetrakis(triphenylphosphine)palladium. Compound (1A) is also prepared by allowing compound (23) to react with n-butyllithium and subsequently with zinc chloride, and further with compound (22) in the presence of a catalyst including dichlorobis(triphenylphosphine)palladium.

### (II) Formation of -COO- and -OCO-

Carboxylic acid (24) is obtained by allowing compound (23) to react with n-butyllithium and subsequently with carbon dioxide. Compound (1B) having -COO- is prepared by dehydration of carboxylic acid (24) described above and phenol (25) induced from compound (21) in the presence of 1,3-dicyclohexylcarbodiimide (DCC) and 4-dimethylaminopyridine (DMAP). A compound having -OCO- is also prepared according to the method.

### (III) Formation of -CF₂O- and -OCF₂-

Compound (26) is obtained by sulfurizing compound (1B) with a Lawesson's reagent. Compound (1C) having -CF₂O- is prepared by fluorinating compound (26) with a hydrogen fluoride-pyridine complex and N-bromosuccinimide (NBS). Refer to M. Kuroboshi et al., Chem. Lett., 1992, 827. Compound (1C) is also prepared by fluorinating compound (26) with (diethylamino)sulfur trifluoride (DAST). Refer to W. H. Bunnelle et al., J. Org. Chem. 1990, 55, 768. A compound having -OCF₂- is also prepared by the method described above.

### (IV) Formation of -CH=CH-

Aldehyde (27) is obtained by allowing compound (22) to react with n-butyllithium and subsequently with N,N-dimethylformamide (DMF). Compound (1D) is prepared by allowing phosphorus ylide, generated by allowing phosphonium salt (28) to react with potassium t-butoxide, to react with aldehyde (27). A cis isomer may be generated depending on reaction conditions, and therefore the cis isomer is isomerized into a trans isomer according to a publicly known method when necessary.

### (V) Formation of -CH₂CH₂-

Compound (1E) is prepared by hydrogenating compound (1D) in the presence of a catalyst including palladium on carbon.

### (VI) Formation of -C≡C-

Compound (29) is obtained by allowing compound (23) to react with 2-methyl-3-butyn-2-ol in the presence of a catalyst including dichloropalladium and copper iodide, and then performing deprotection under basic conditions. Compound (1F) is prepared by allowing compound (29) to react with compound (22) in the presence of a catalyst including dichlorobis(triphenylphosphine)palladium and copper halide.

### (VII) Formation of -CH₂O- and -OCH₂-

Compound (30) is obtained by reducing compound (27) with sodium borohydride. Compound (31) is obtained by brominating the obtained compound with hydrobromic acid. Compound (1G) is prepared by allowing compound (25) to react with compound (31) in the presence of potassium carbonate. A compound having -OCH₂- is also prepared by the method described above.

### (VIII) Formation of -CF=CF-

Compound (32) is obtained by treating compound (23) with n-butyllithium and then allowing the treated compound to react with tetrafluoroethylene. Compound (1H) is prepared by treating compound (22) with n-butyllithium and then allowing the treated compound to react with compound (32).

### 2-2. Formation of rings A¹ and A²

A starting material is commercially available or a synthetic method is well known with regard to a ring such as 1,2-cyclopropylene, 1,3-cyclobutylene, 1,3-cyclopentylene, 1,4-cyclohexylene, 1,4-cycloheptylene, 1,4-cyclohexenylene, 1,4-phenylene, naphthalene-2,6-diyl, decahydronaphthalene-2,6-diyl, 1,2,3,4-tetrahydronaphthalene-2,6-diyl, tetrahydropyran-2,5-diyl, 1,3-dioxane-2,5-diyl, pyrimidine-2,5-diyl and pyridine-2,5-diyl.

### 2-3. Synthesis Example

An example of a method for preparing compound (1) is as described below. In the compounds, definitions of R¹, ring A¹, M¹, M² and the like are identical to definitions described in item 1.

Compound (1-51) in which, in formula (1), ring A² is phenyl, Z¹ is a single bond, a is 1, b is 0, c is 1, Sp² is -CH₂-, Sp³ is a single bond, P² is a methacryloyl group, and P³ is a group represented by formula (1b), and in formula (1b), M³ and M⁴ are hydrogen, Sp⁴ is -CH₂-, and X¹ is -OH can be prepared according to a method described below.

Compound (52) is obtained by allowing compound (51) to react with formaldehyde in the presence of 1,4-diazabicyclo[2.2.2]octane (DABCO). Compound (53) is obtained by allowing compound (52) to react with 3,4-dihydro-2H-pyran in the presence of pyridinium p-toluenesulfonate (PPTS). Compound (54) is obtained by hydrolyzing compound (53) with lithium hydroxide. Compound (57) is obtained by allowing compound (55) and compound (56) to react with each other in the presence of tetrakis(triphenylphosphine)palladium and potassium carbonate. Compound (58) is obtained by reducing compound (57) with sodium borohydride. Compound (59) is obtained by allowing compound (58) to react with compound (54) in the presence of DCC and DMAP. Compound (60) is obtained by allowing compound (59) to react with methacrylic acid in the presence of DCC and DMAP. Compound (1-51) can be derived by performing deprotection of compound (60) by using PPTS.

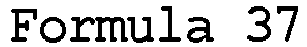

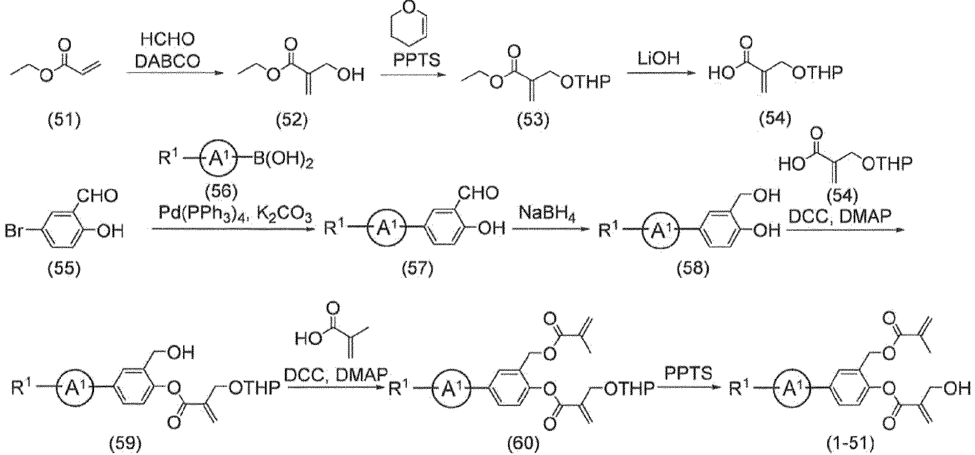

Compound (1-52) in which, in formula (1), ring A² is phenyl, Z¹ is a single bond, a is 1, b is 0, c is 1, Sp² is -CH₂-, Sp³ is -O(CH₂)₂-, P² is a methacryloyl group, and P³ is a group represented by formula (1b), and in formula (1b), M³ and M⁴ are hydrogen, Sp⁴ is -CH₂-, and X¹ is -OH can be prepared according to a method described below. Compound (62) is obtained by allowing compound (58) to react with compound (61) in the presence of potassium carbonate. Compound (63) is obtained by allowing compound (62) to react with methacrylic acid in the presence of DCC and DMAP. Compound (64) can be obtained by performing deprotection of compound (63) by using tetrabutylammonium fluoride (TBAF). Compound (65) is obtained by allowing compound (64) to react with compound (54) in the presence of DCC and DMAP. Compound (1-52) can be derived by performing deprotection of compound (65) by using PPTS.

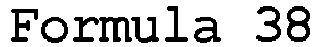

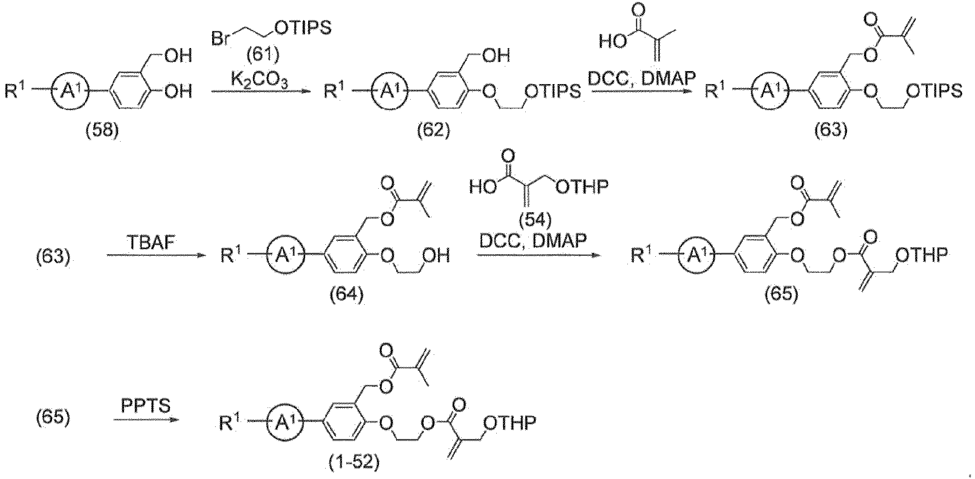

Compound (1-53) in which, in formula (1), ring A¹ is phenyl, Z¹ is a single bond, a is 1, b is 1, c is 0, Sp² is -CH₂-, Sp³ is a single bond, P² is a methacryloyl group, and P³ is a group represented by formula (1b), and in formula (1b), M³ and M⁴ are hydrogen, Sp⁴ is -CH₂-, and X¹ is -OH can be prepared according to a method described below.

Compound (68) is obtained by allowing compound (66) and compound (67) to react with each other in the presence of tetrakis(triphenylphosphine)palladium and potassium carbonate. Compound (70) is obtained by allowing compound (68) and compound (69) to react with each other in the presence of tetrakis(triphenylphosphine)palladium and potassium carbonate. Compound (71) is obtained by reducing compound (70) with sodium borohydride. Compound (72) is obtained by allowing compound (71) to react with methacrylic acid in the presence of DCC and DMAP. Compound (73) is obtained by performing deprotection of compound (72) by using TBAF. Compound (74) is obtained by allowing compound (73) to react with compound (54) in the presence of DCC and DMAP. Compound (1-53) can be derived by performing deprotection of compound (74) by using PPTS.

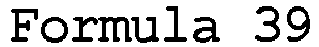

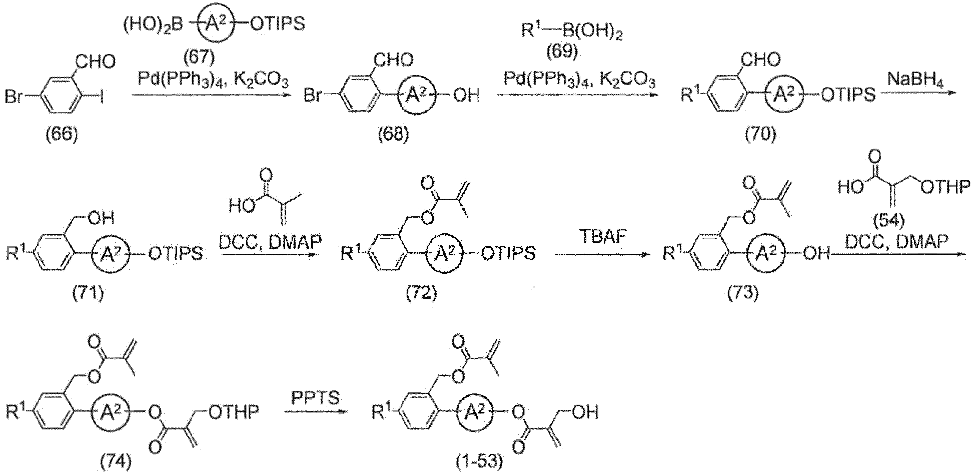

Compound (1-55) in which, in formula (1), P³ is a group represented by formula (1b), and in formula (1b), M³ and M⁴ are hydrogen, Sp⁴ is - (CH₂)₂-, and X¹ is -OH can be prepared according to a method described below.

Compound (75) is obtained by allowing phosphorus tribromide to act on compound (1-54). Next, compound (1-55) can be derived by allowing indium to act on compound (57) and then allowing the resulting compound to react with formaldehyde.

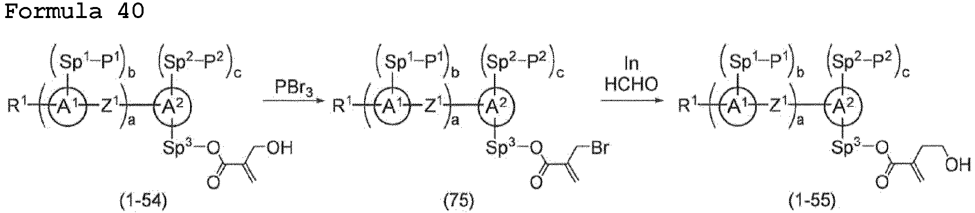

Compound (1-56) in which, in formula (1), P³ is a group represented by formula (1b), and in formula (1b), M³ and M⁴ are hydrogen, Sp⁴ is -CH₂O(CH₂)₂-, and X¹ is -OH can be prepared according to a method described below.

Compound (1-56) can be derived by allowing trifluoromethanesulfonic anhydride and triethylamine to act on compound (1-54) and then allowing the resulting compound to react with ethylene glycol.

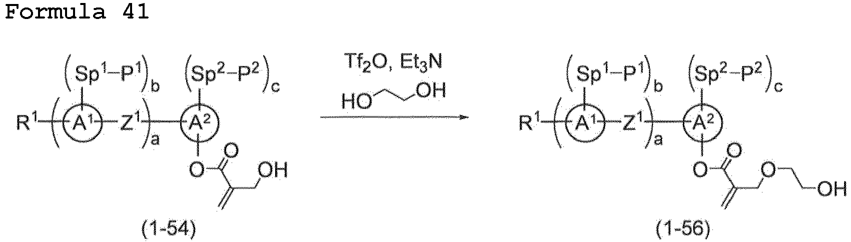

### 3. Liquid crystal composition

### 3-1. Component compound

A liquid crystal composition contains compound (1) as component A. Compound (1) noncovalently interacts with a substrate of a device, and thus can control alignment of liquid crystal molecules. The composition contains compound (1) as component A, and preferably, further contains a liquid crystal compound selected from components B, C, D and E described below. Component B includes compounds (2) to (4). Component C includes compounds (5) to (7). Component D includes compound (8). Component E includes compounds (11) to (19). The composition may contain any other liquid crystal compound different from compounds (2) to (19). When the composition is prepared, components B, C, D and E are preferably selected by considering magnitude of positive or negative dielectric anisotropy, or the like. A composition in which components thereof are suitably selected has high maximum temperature, low minimum temperature, small viscosity, suitable optical anisotropy (more specifically, large optical anisotropy or small optical anisotropy), large positive or negative dielectric anisotropy, large specific resistance, stability to heat or ultraviolet light and a suitable elastic constant (more specifically, a large elastic constant or a small elastic constant).

Compound (1) is added to a composition for the purpose of controlling alignment of liquid crystal molecules. A preferred proportion of compound (1) is ordinarily 0.05% by weight or more, preferably 0.1% by weight or more, further preferably 0.5% by weight or more, and still further preferably 1.0% by weight or more based on the total weight of the liquid crystal composition for aligning liquid crystal molecules, and is ordinarily 10% by weight or less, preferably 7% by weight or less, and further preferably 5% by weight or less based on the total weight of the liquid crystal composition for preventing poor display of the device. In the range described above, a problem of precipitation or the like can be prevented from being caused by decreasing solubility in the liquid crystal composition. The proportions are applied also to the total amount of compound (1) and compound (20).

Component B includes a compound in which two terminal groups are alkyl or the like. Specific examples of preferred component B include compounds (2-1) to (2-11), compounds (3-1) to (3-19) and compounds (4-1) to (4-7) . In a compound of component B, R¹¹ and R¹² are independently alkyl having 1 to 10 carbons or alkenyl having 2 to 10 carbons, and in the alkyl or the alkenyl, at least one piece of -CH₂- may be replaced by -O-, and at least one hydrogen may be replaced by fluorine.

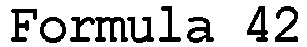

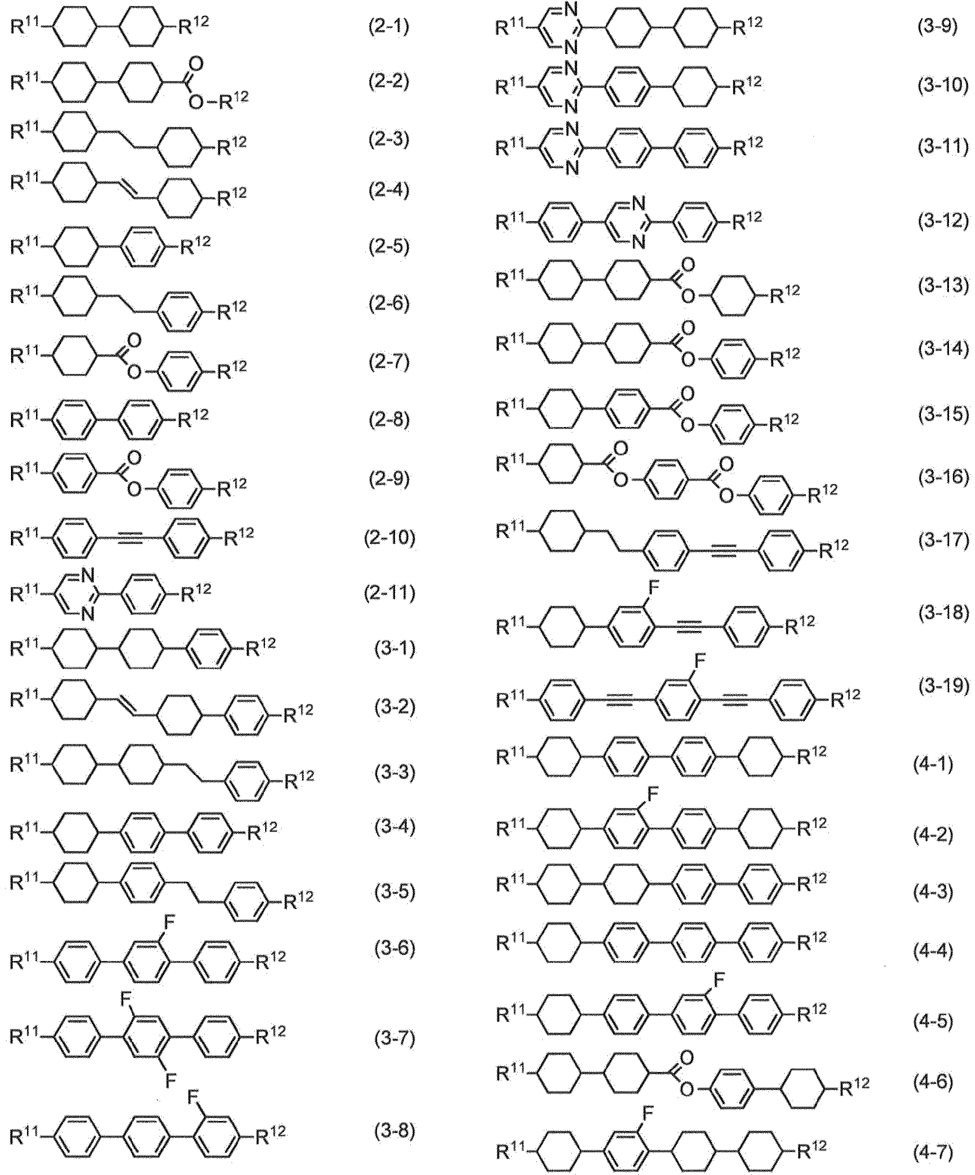

Component B has a small absolute value of dielectric anisotropy, and therefore is a compound close to neutrality. Compounds (2-1) to (2-11) are mainly effective in decreasing viscosity or adjusting optical anisotropy. Compounds (3-1) to (3-19) and (4-1) to (4-7) are effective in extending the temperature range of the nematic phase by increasing the maximum temperature, or in adjusting the optical anisotropy.

As a content of component B is increased, the dielectric anisotropy of the composition is decreased, but the viscosity is decreased. Thus, as long as a desired value of threshold voltage of the device is met, the content may be, for example, as large as possible. When a composition for the IPS mode, the VA mode or the like is prepared, the content of component B may be, for example, 30% by weight or more, and further preferably 40% by weight or more, based on the total weight of the liquid crystal composition.

Component C is a compound having a halogen-comprising group or a fluorine-comprising group at a right terminal. Specific examples of preferred component C include compounds (5-1) to (5-16), compounds (6-1) to (6-116) and compounds (7-1) to (7-59). In a compound of component C, R¹³ is alkyl having 1 to 10 carbons or alkenyl having 2 to 10 carbons, and in the alkyl and the alkenyl, at least one piece of -CH₂- may be replaced by -O-, and at least one hydrogen may be replaced by fluorine; and X¹¹ is fluorine, chlorine, -OCF₃, -OCHF₂, -CF₃, -CHF₂, -CH₂F, -OCF₂CHF₂ or -OCF₂CHFCF₃.

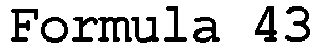

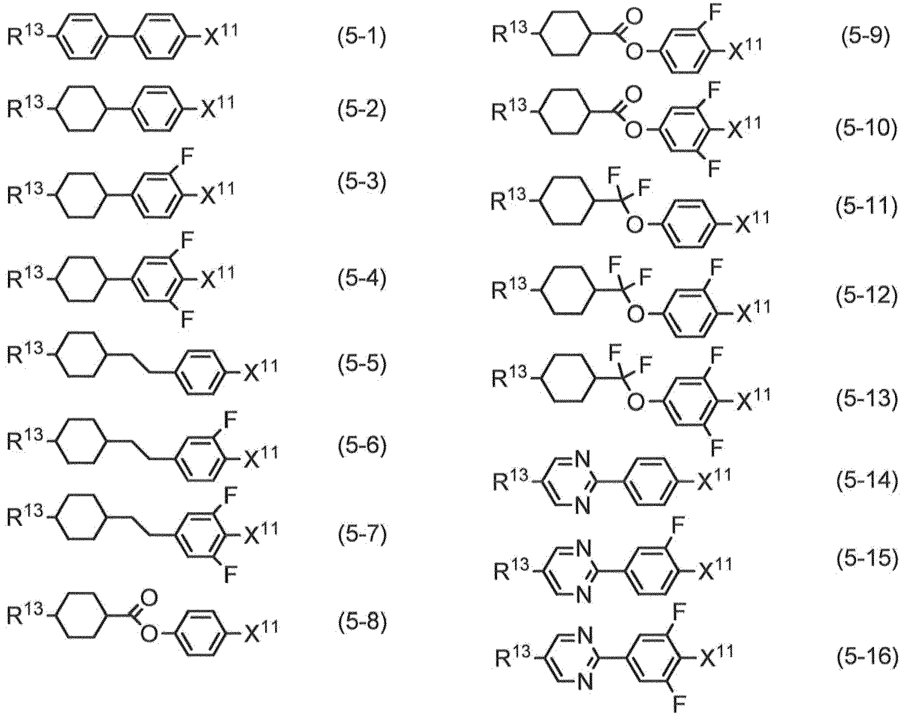

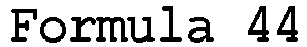

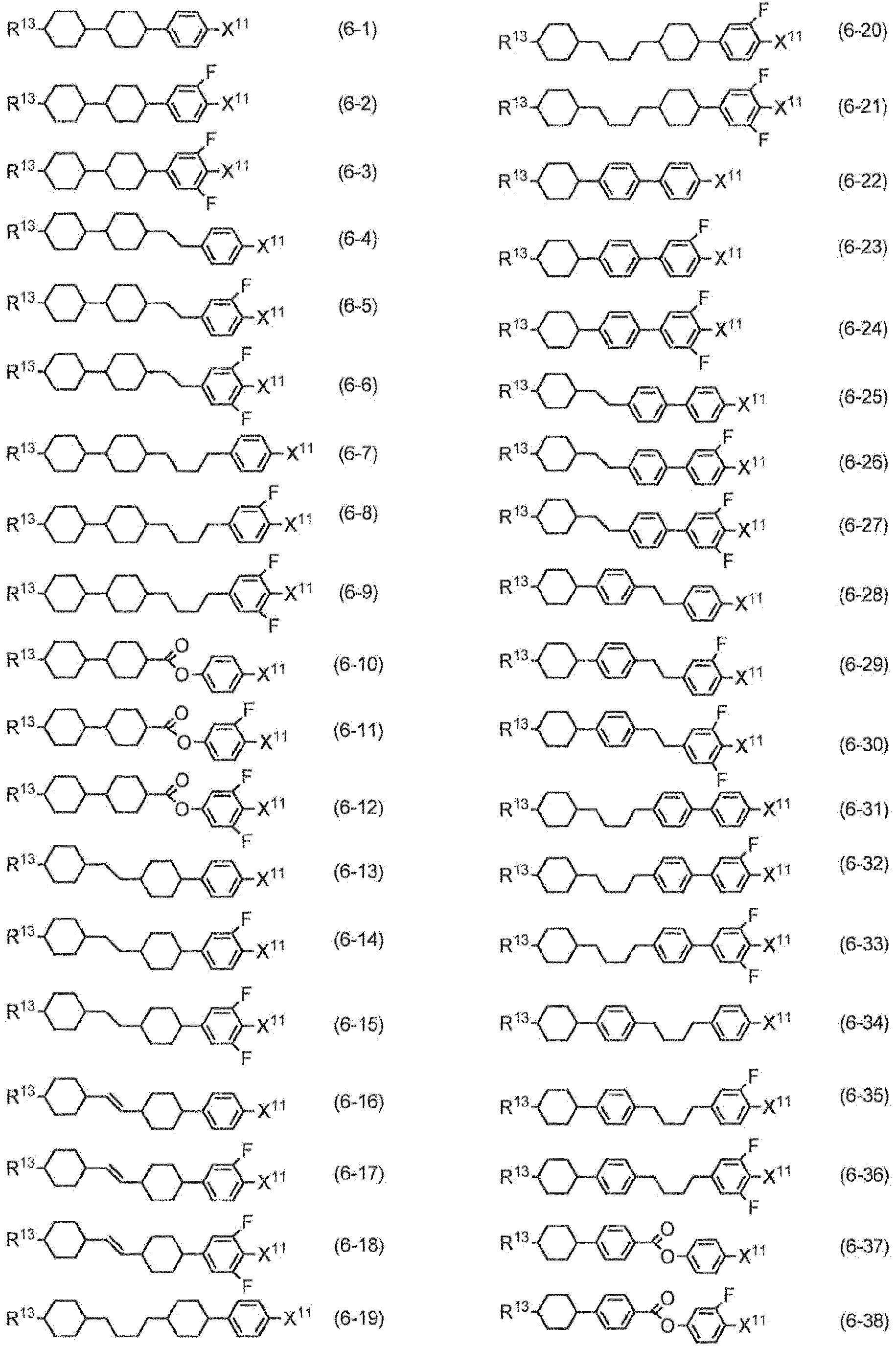

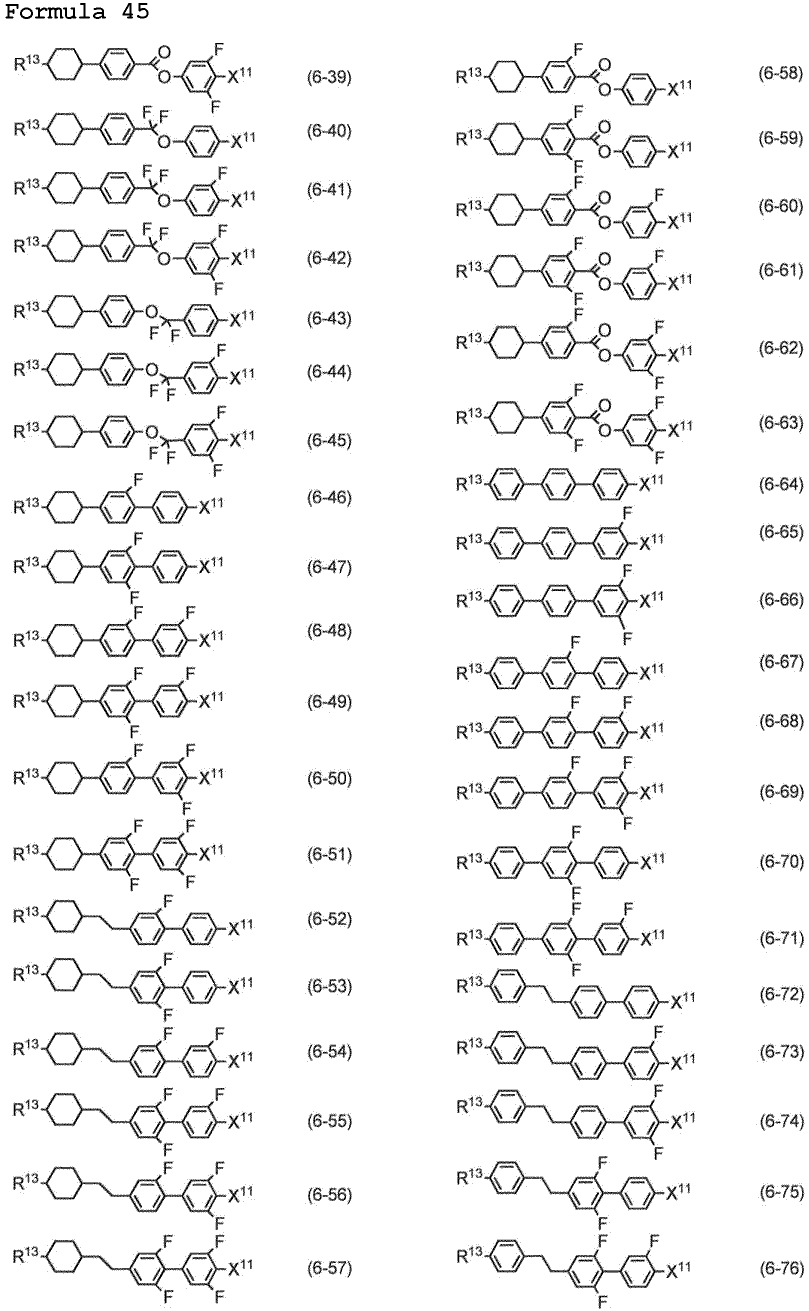

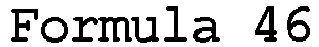

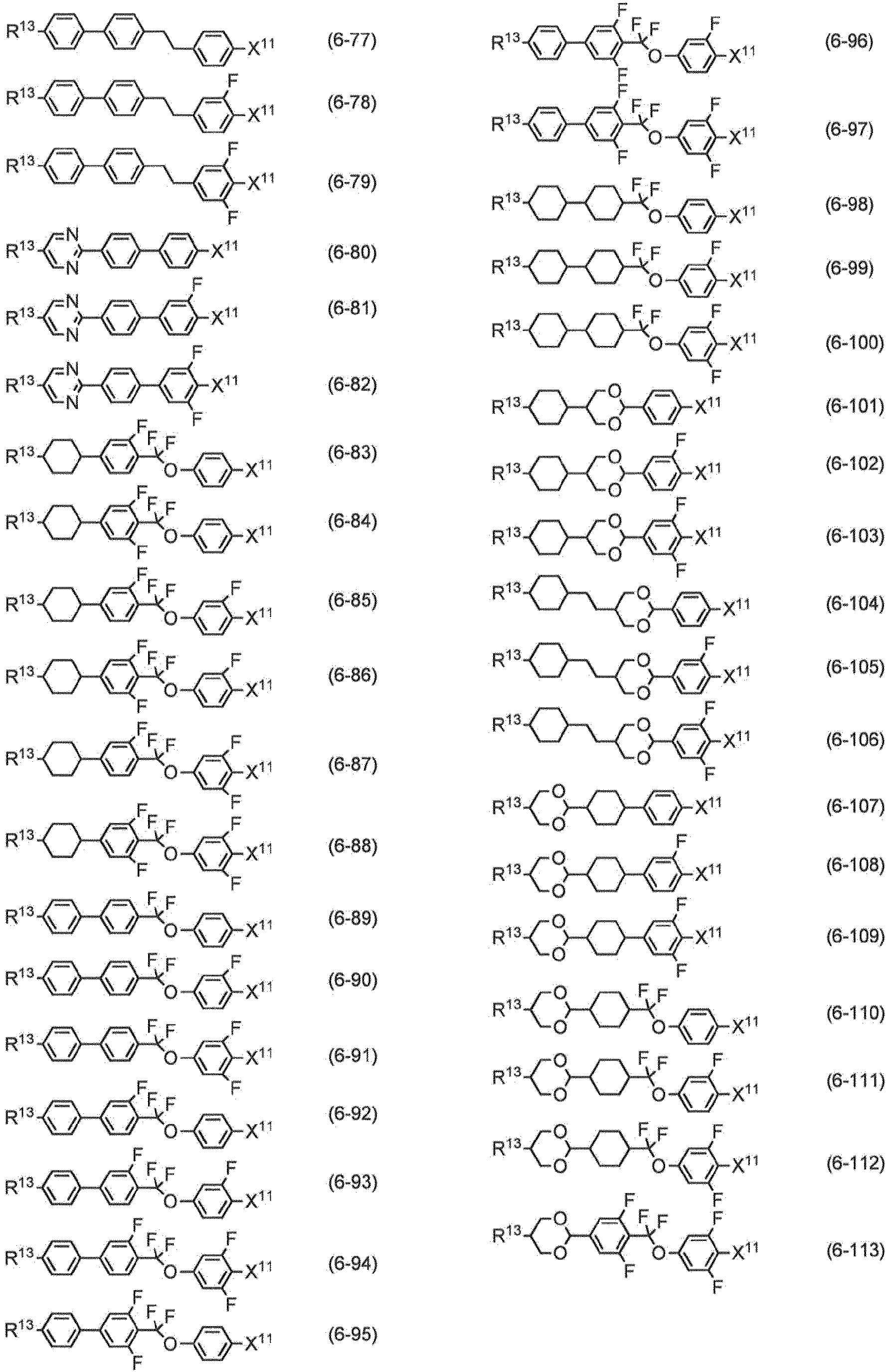

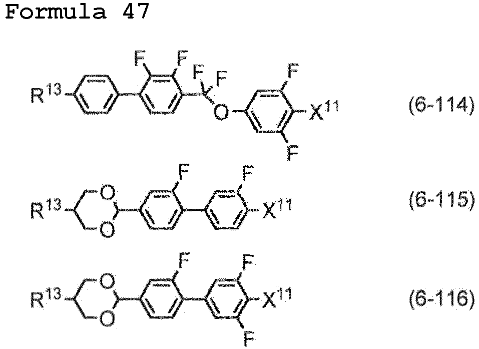

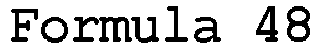

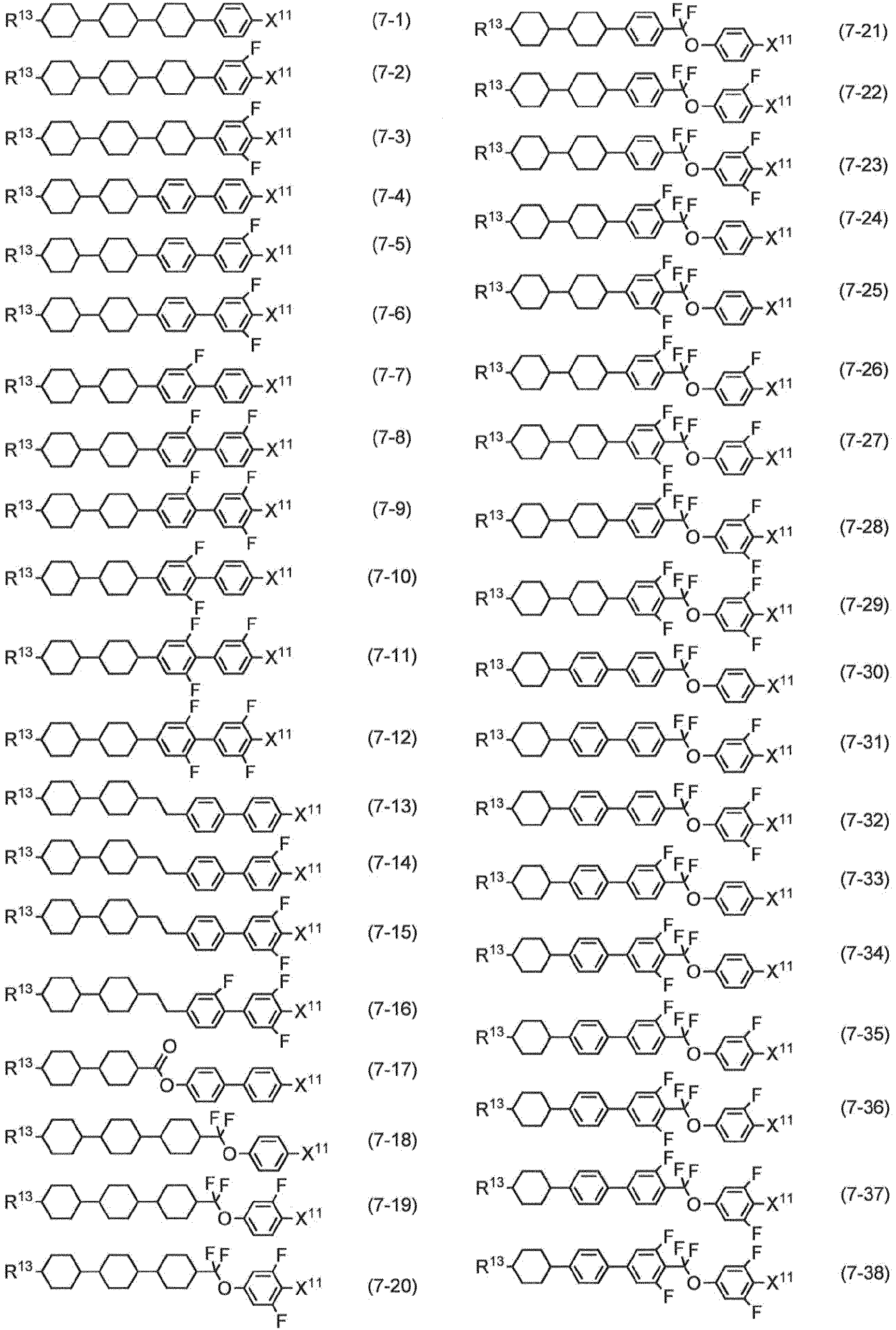

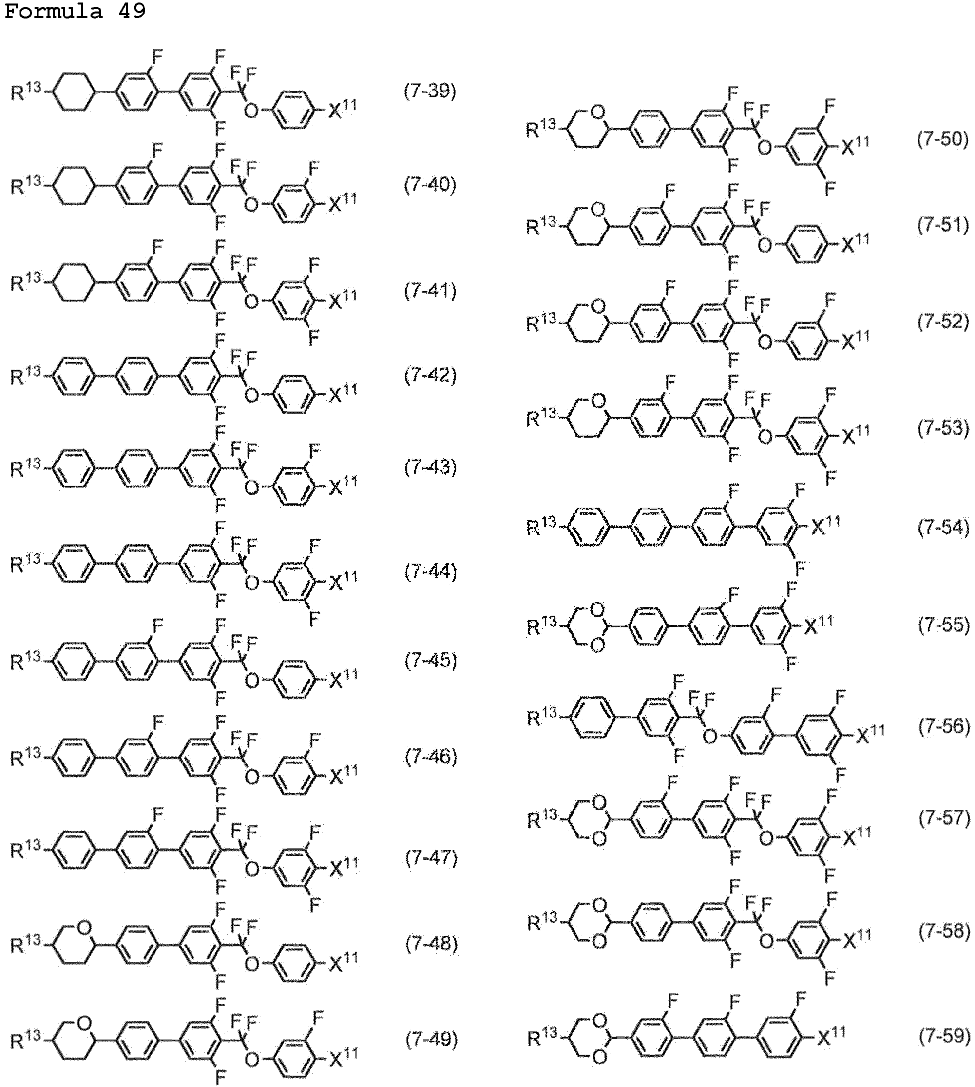

Component C has positive dielectric anisotropy, and significantly good stability to heat, light and so forth, and therefore is used when a composition for the IPS mode, the FFS mode, the OCB mode or the like is prepared. A content of component C is suitably in the range of 1% by weight to 99% by weight, preferably in the range of 10% by weight to 97% by weight, and further preferably in the range of 40% by weight to 95% by weight, based on the total weight of the liquid crystal composition. When component C is added to a composition having negative dielectric anisotropy, the content of component C may be, for example, 30% by weight or less based on the total weight of the liquid crystal composition. Addition of component C allows adjustment of the elastic constant of the composition and adjustment of a voltage-transmittance curve of the device.

Component D is compound (8) in which a right-terminal group is -C≡N or -C≡C-C≡N. Specific examples of preferred component D include compounds (8-1) to (8-64) . In a compound of component D, R¹⁴ is alkyl having 1 to 10 carbons or alkenyl having 2 to 10 carbons, and in the alkyl and the alkenyl, at least one piece of -CH₂- may be replaced by -O-, and at least one hydrogen may be replaced by fluorine; and -X¹² is -C≡N or -C≡C-C≡N.

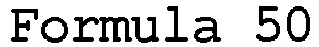

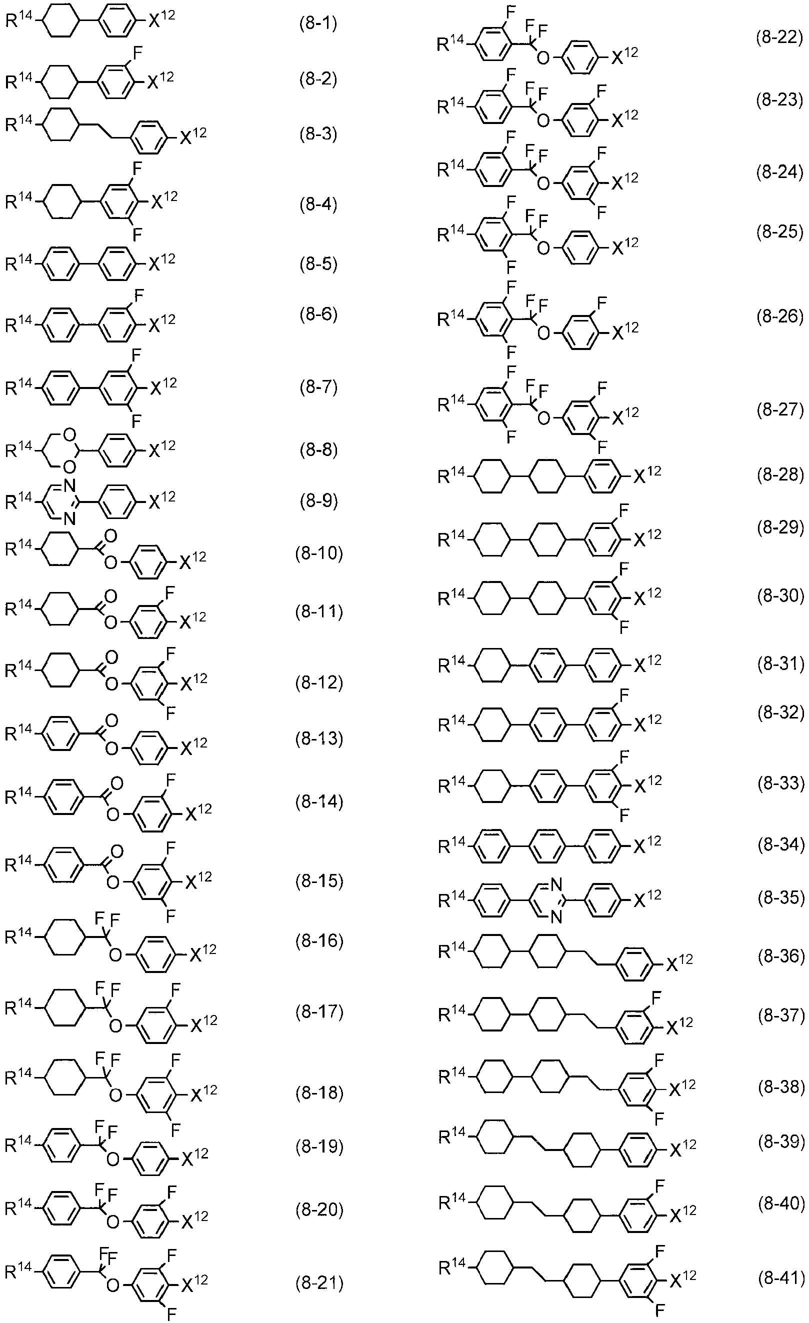

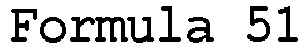

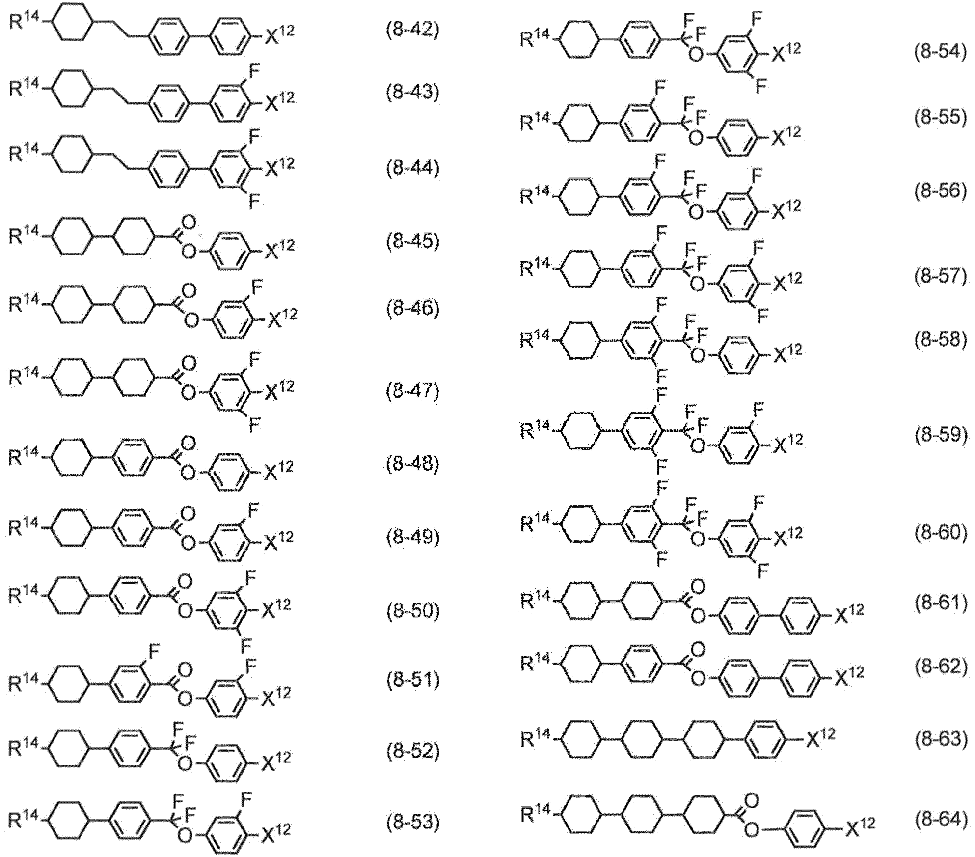

Component D has positive dielectric anisotropy and a value thereof is large, and therefore is mainly used when a composition for the TN mode or the like is prepared. Addition of component D can increase the dielectric anisotropy of the composition. Component D is effective in extending the temperature range of the liquid crystal phase, adjusting the viscosity or adjusting the optical anisotropy. Component D is also useful for adjustment of the voltage-transmittance curve of the device.

When the composition for the TN mode or the like is prepared, a content of component D is suitably in the range of 1% by weight to 99% by weight, preferably in the range of 10% by weight to 97% by weight, and further preferably in the range of 40% by weight to 95% by weight, based on the total weight of the liquid crystal composition. When component D is added to a composition having negative dielectric anisotropy, the content of component D may be, for example, 30% by weight or less based on the total weight of the liquid crystal composition. Addition of component D allows adjustment of the elastic constant of the composition and adjustment of the voltage-transmittance curve of the device.

Component E includes compounds (11) to (19). The compounds have phenylene in which hydrogen in lateral positions are replaced by two halogens, such as 2,3-difluoro-1,4-phenylene. Specific examples of preferred component E include compounds (11-1) to (11-9), compounds (12-1) to (12-19), compounds (13-1) and (13-2), compounds (14-1) to (14-3), compounds (15-1) to (15-3), compounds (16-1) to (16-11), compounds (17-1) to (17-3), compounds (18-1) to (18-3) and compound (19-1) . In the compounds, R¹⁵, R¹⁶ and R¹⁷ are independently alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine, and R¹⁷ may be hydrogen or fluorine.

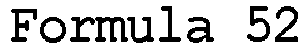

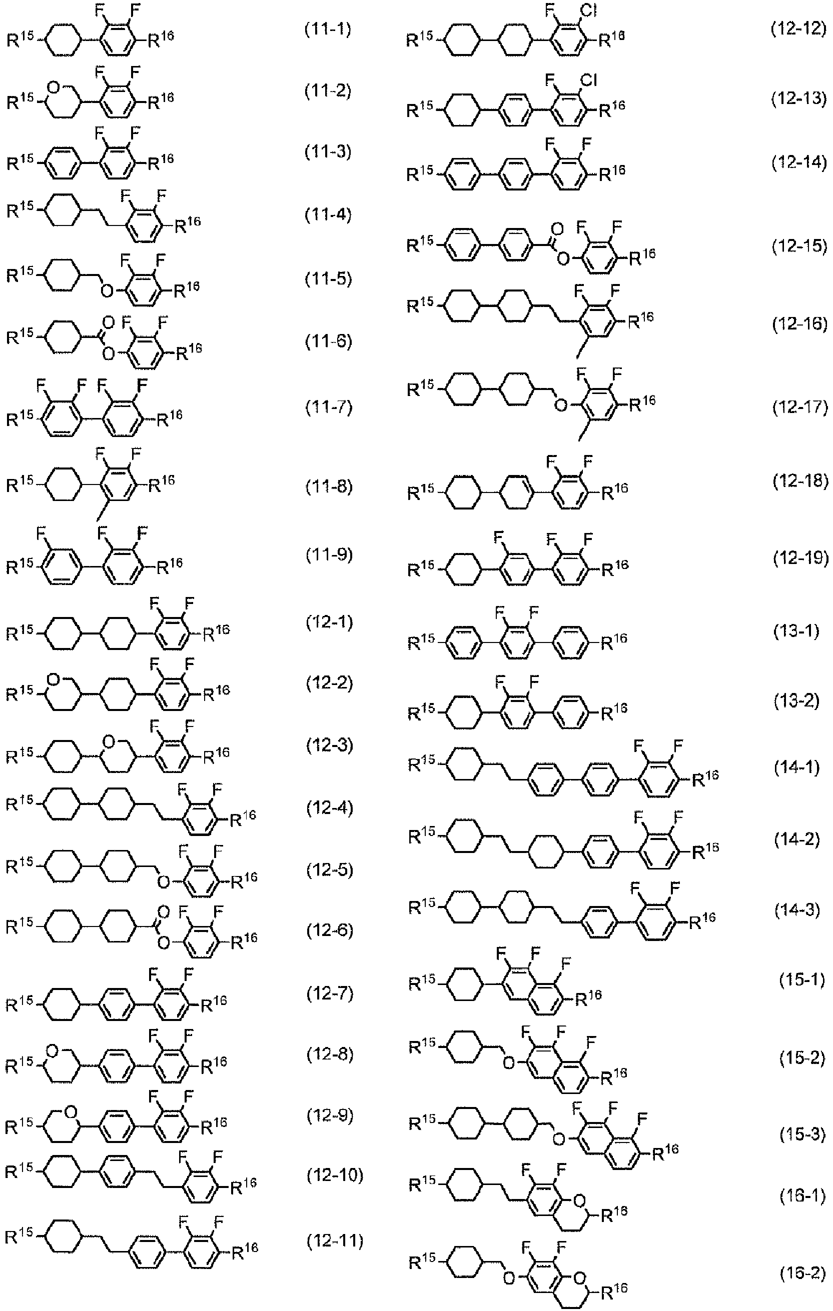

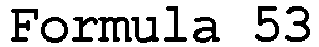

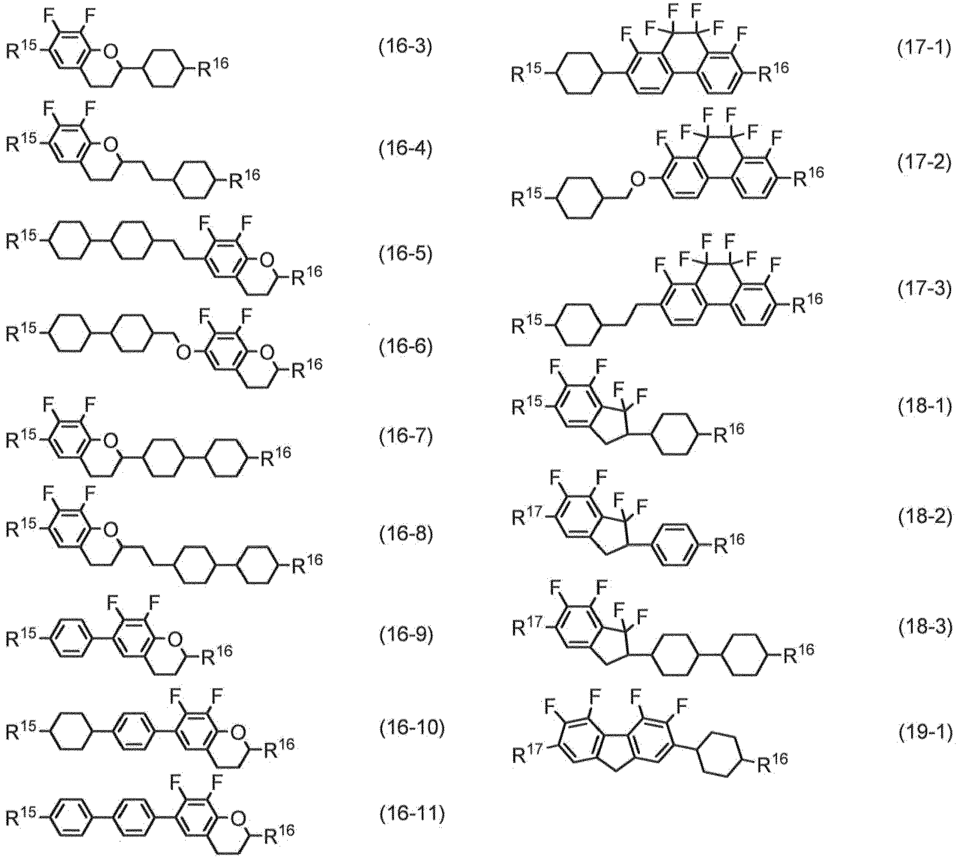

Component E has large negative dielectric anisotropy. Component E is used when a composition for the IPS mode, the VA mode, the PSA mode or the like is prepared. As a content of component E is increased, the dielectric anisotropy of the composition is negatively increased, but the viscosity is increased. Thus, as long as a desired value of threshold voltage of the device is met, the content may be, for example, as small as possible. When the dielectric anisotropy at a degree of -5 is taken into account, the content may be, for example, 40% by weight or more in order to allow a sufficient voltage driving based on the total weight of the liquid crystal composition.

Among types of component E, compound (11) is a bicyclic compound, and therefore is effective in decreasing the viscosity, adjusting the optical anisotropy or increasing the dielectric anisotropy. Compounds (12) and (13) are a tricyclic compound, and compound (14) is a tetracyclic compound, and therefore are effective in increasing the maximum temperature, the optical anisotropy or the dielectric anisotropy. Compounds (15) to (19) are effective in increasing the dielectric anisotropy.

When a composition for the IPS mode, the VA mode, the PSA mode or the like is prepared, the content of component E may be, for example, 40% by weight or more, and further preferably in the range of 50% by weight to 95% by weight, based on the total weight of the liquid crystal composition. When component E is added to a composition having positive dielectric anisotropy, the content of component E may be, for example, 30% by weight or less based on the weight of the liquid crystal composition. Addition of component E allows adjustment of the elastic constant of the composition and adjustment of the voltage-transmittance curve of the device.

A liquid crystal composition satisfying at least one of characteristics such as high maximum temperature, low minimum temperature, small viscosity, suitable optical anisotropy, large positive or negative dielectric anisotropy, large specific resistance, high stability to ultraviolet light, high stability to heat and a large elastic constant can be prepared by suitably combining components B, C, D and E described above. Any other liquid crystal compound different from components B, C, D and E may be added thereto when necessary.

### 3-2. Additive

The liquid crystal composition is prepared according to a publicly known method. For example, the component compounds are mixed and dissolved in each other by heating. According to an application, an additive may be added to the composition. Specific examples of the additives include the polymerizable compound, the polymerization initiator, the polymerization inhibitor, the optically active compound, the antioxidant, the ultraviolet light absorber, the light stabilizer, the heat stabilizer and the antifoaming agent. Such additives are well known to those skilled in the art, and described in literature.

The polymerizable compound is added for the purpose of forming the polymer in the liquid crystal composition. Compound (1) is polymerized by irradiation with ultraviolet light while voltage is applied between electrodes, and thus the polymer is formed in the liquid crystal composition. On the occasion, compound (1) is fixed in a state where the polar group noncovalently interacts with a substrate surface of glass (or of metal oxide). Thus, capability of controlling alignment of liquid crystal molecules is further improved, and suitable pretilt can be obtained, and therefore a response time is shortened.

Preferred examples of the polymerizable compound include acrylate, methacrylate, a vinyl compound, a vinyloxy compound, propenyl ether, an epoxy compound (oxirane, oxetane) and vinyl ketone. Further preferred examples include a compound having at least one acryloyloxy and a compound having at least one methacryloyloxy. Still further preferred examples also include a compound having both acryloyloxy and methacryloyloxy.

Compound (1) has a polar group. On the other hand, compound (20) has no polar group. A preferred example of compound (20) will be described.

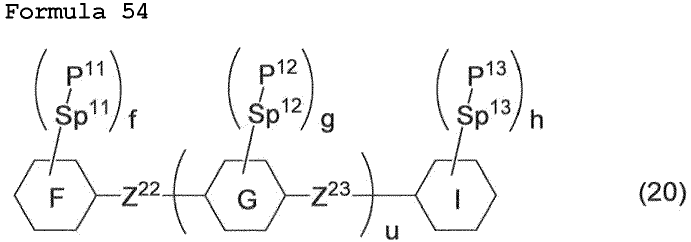

In formula (20), ring F and ring I are independently cyclohexyl, cyclohexenyl, phenyl, 1-naphthyl, 2-naphthyl, tetrahydropyran-2-yl, 1,3-dioxane-2-yl, pyrimidine-2-yl or pyridine-2-yl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen.

Preferred ring F or ring I is cyclohexyl, cyclohexenyl, phenyl, fluorophenyl, difluorophenyl, 1-naphthyl or 2-naphthyl. Further preferred ring F or ring I is cyclohexyl, cyclohexenyl or phenyl. Particularly preferred ring F or ring I is phenyl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen.

Ring G is 1,4-cyclohexylene, 1,4-cyclohexenylene, 1, 4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl, naphthalene-2,7-diyl, phenanthrene-2,7-diyl, tetrahydropyran-2,5-diyl, 1,3-dioxane-2,5-diyl, pyrimidine-2,5-diyl or pyridine-2,5-diyl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen.

Preferred ring G is 1,4-cyclohexylene, 1,4-cyclohexenylene, 1,4-phenylene, 2-fluoro-1,4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl or naphthalene-2,7-diyl. Further preferred ring G is 1,4-cyclohexylene, 1,4-cyclohexenylene, 1,4-phenylene or 2-fluoro-1,4-phenylene. Particularly preferred ring G is 1,4-phenylene or 2-fluoro-1,4-phenylene. Most preferred ring G is 1,4-phenylene.

In formula (20), Z²² and Z²³ are independently a single bond or alkylene having 1 to 10 carbons, and in the alkylene, at least one piece of -CH₂- may be replaced by -O-, -CO-, -COO- or -OCO-, and at least one piece of -CH₂CH₂- may be replaced by -CH=CH-, -C(CH₃)=CH-, -CH=C (CH₃)- or -C (CH₃)=C (CH₃)-, and in the groups, at least one hydrogen may be replaced by fluorine or chlorine. Preferred Z²² or Z²³ is independently a single bond, -CH₂CH₂-, -CH₂O-, -OCH₂-, -COO- or -OCO-. Further preferred Z²² or Z²³ is a single bond.

In compound (20), P¹¹, P¹² and P¹³ are independently a polymerizable group. Preferred P¹¹ to P¹³ are independently a group selected from the group of polymerizable groups represented by formula (P-1) to formula (P-5). Further preferred P¹¹ to P¹³ are independently a group represented by formula (P-1) or formula (P-5). Particularly preferred P¹¹ to P¹³ are independently a group represented by formula (P-1). A preferred group represented by formula (P-1) is acryloyloxy (-OCO-CH=CH₂) or methacryloyloxy (-OCO-C(CH₃)=CH₂). A wavy line in formula (P-1) to formula (P-5) represents a site to form a bonding with Sp¹¹, Sp¹² or Sp¹³.

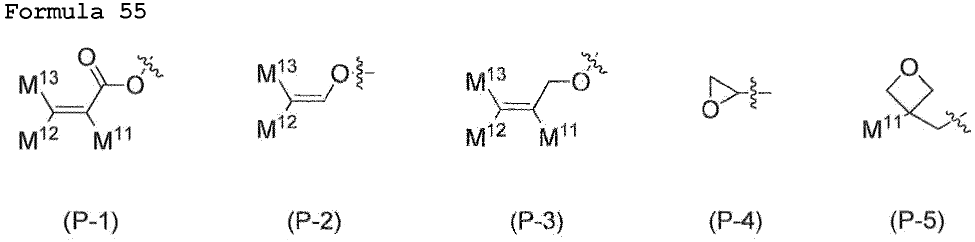

In formula (P-1) to formula (P-5), M¹¹, M¹² and M¹³ are independently hydrogen, fluorine, alkyl having 1 to 5 carbons, or alkyl having 1 to 5 carbons in which at least one hydrogen is replaced by halogen. Preferred M¹¹, M¹² or M¹³ is independently hydrogen or methyl for increasing reactivity. Further preferred M¹¹ is hydrogen or methyl, and further preferred M¹² or M¹³ is independently hydrogen.

In formula (20), Sp¹¹, Sp¹² and Sp¹³ are independently a single bond or alkylene having 1 to 10 carbons, and in the alkylene, at least one piece of -CH₂- may be replaced by -O-, -COO-, -OCO- or -OCOO-, and at least one piece of -CH₂CH₂- may be replaced by -CH=CH- or -C≡C-, and in the groups, at least one hydrogen may be replaced by fluorine or chlorine. Preferred Sp¹¹, Sp¹² or Sp¹³ is a single bond.

In formula (20), u is 0, 1 or 2. Preferred u is 0 or 1.

Then, f, g and h are independently 0, 1, 2, 3 or 4, and a sum of f, g and his 1 or more. Preferred f, g or his 1 or 2. A sum of preferred f, g and h is 2, 3 or 4. A sum of further preferred f, g and h is 2 or 3.

Further preferred examples include compounds (20-1-1) to (20-1-5), compounds (20-2-1) to (20-2-5), compound (20-4-1), compound (20-5-1), compound (20-6-1), compound (20-7-1) and compounds (20-8) to (20-11). In compounds (20-1-1) to (20-1-5), compounds (20-2-1) to (20-2-5), compound (20-4-1), compound (20-5-1), compound (20-6-1), compound (20-7-1) and compounds (20-8) to (20-11), R²⁵ to R³¹ are independently hydrogen or methyl, R³², R³³ and R³⁴ are independently hydrogen or alkyl having 1 to 5 carbons, and at least one of R³², R³³ and R³⁴ is alkyl having 1 to 5 carbons; v and x are independently 0 or 1; t and u are independently an integer from 1 to 10; and L³¹ to L³⁶ are independently hydrogen or fluorine, and L³⁷ and L³⁸ are independently hydrogen, fluorine or methyl.

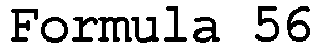

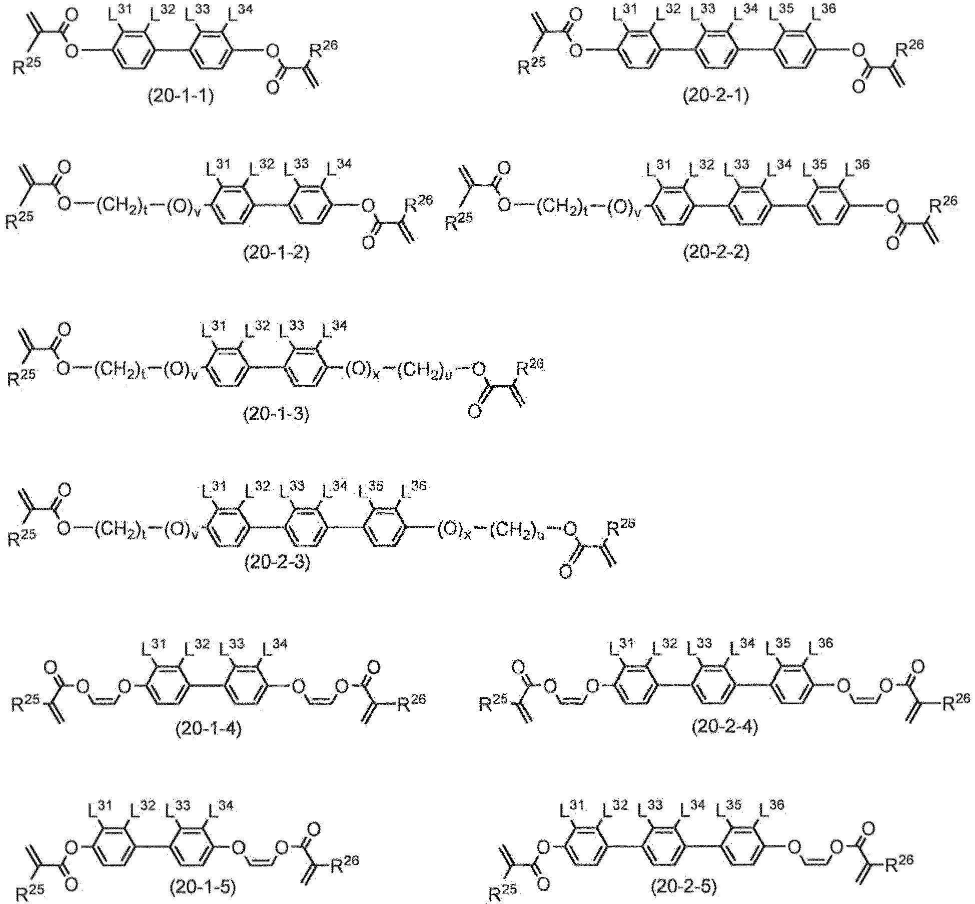

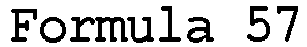

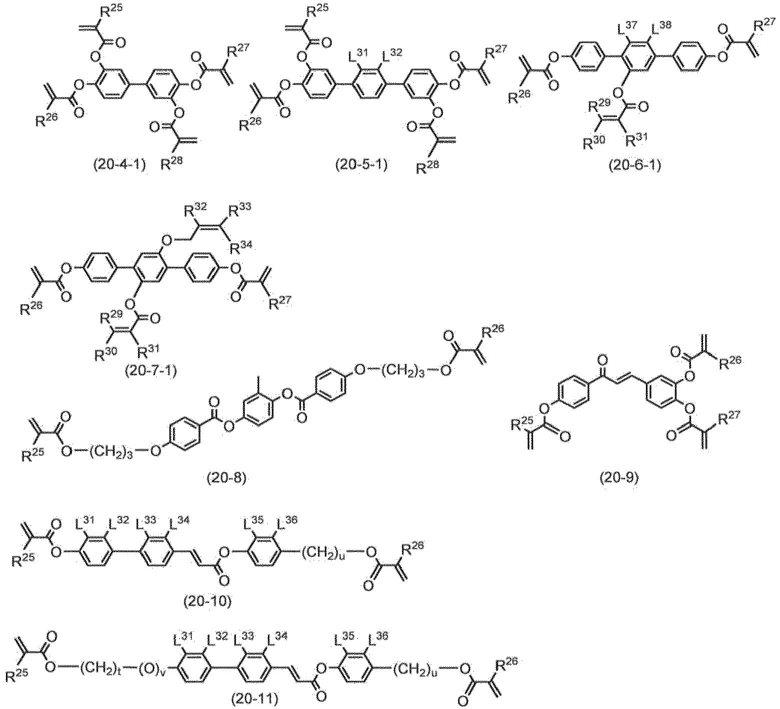

A polymerizable compound can be rapidly polymerized by adding the polymerization initiator such as photoradical polymerization initiator. An amount of a remaining polymerizable compound can be decreased by optimizing reaction conditions. Specific examples of the photoradical polymerization initiator include TPO, 1173 and 4265 from Darocur series of BASF SE, and 184, 369, 500, 651, 784, 819, 907, 1300, 1700, 1800, 1850 and 2959 from Irgacure series thereof.

Additional examples of the photoradical polymerization initiator include 4-methoxyphenyl-2,4-bis(trichloromethyl)triazine, 2-(4-butoxystyryl)-5-trichloromethyl-1,3,4-oxadiazole, 9-phenylacridine, 9,10-benzphenazine, a benzophenone-Michler's ketone mixture, a hexaarylbiimidazole-mercaptobenzimidazole mixture, 1-(4-isopropylphenyl)-2-hydroxy-2-methylpropane-1-one, benzyl dimethyl ketal, 2-methyl-1-[4-(methylthio)phenyl]-2-morpholinopropane-1-one, a mixture of 2,4-diethylxanthone and methyl p-dimethylaminobenzoate, and a mixture of benzophenone and methyltriethanolamine.

After the photoradical polymerization initiator is added to the liquid crystal composition, polymerization can be performed by irradiation with ultraviolet light while an electric field is applied. However, an unreacted polymerization initiator or a decomposition product of the polymerization initiator may cause a poor display such as image persistence in the device. In order to prevent such an event, photopolymerization may be performed with no addition of the polymerization initiator. A preferred wavelength of irradiation light is in the range of 150 nanometers to 500 nanometers . A further preferred wavelength is in the range of 250 nanometers to 450 nanometers, and a most preferred wavelength is in the range of 300 nanometers to 400 nanometers.

Upon storing the polymerizable compound, the polymerization inhibitor may be added thereto for preventing polymerization. The polymerizable compound is ordinarily added to the composition without removing the polymerization inhibitor. Specific examples of the polymerization inhibitor include hydroquinone, a hydroquinone derivative such as methylhydroquinone, 4-t-butylcatechol, 4-methoxyphenol and phenothiazine.

The optically active compound is effective in inducing a helical structure in liquid crystal molecules to give a required twist angle, and thereby preventing a reverse twist. A helical pitch can be adjusted by adding the optically active compound. Two or more optically active compounds may be added for the purpose of adjusting temperature dependence of the helical pitch. Specific examples of a preferred optically active compound include compounds (Op-1) to (Op-18) described below. In compound (Op-18), ring J is 1,4-cyclohexylene or 1,4-phenylene, and R²⁸ is alkyl having 1 to 10 carbons. Asterisk mark (*) represents asymmetrical carbon.

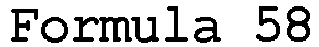

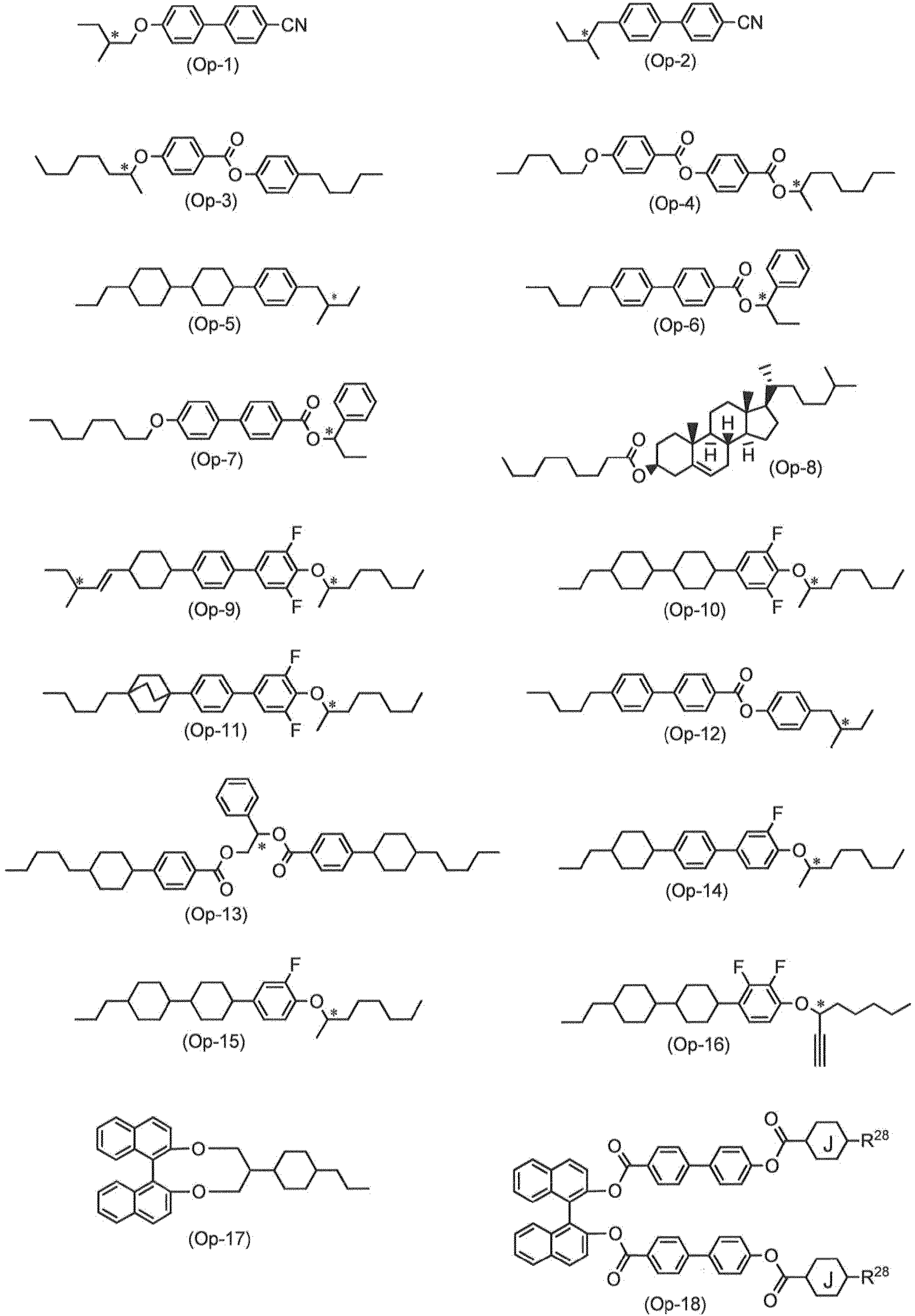

The antioxidant is effective for maintaining the large voltage holding ratio. Specific examples of a preferred antioxidant include compounds (AO-1) and (AO-2) described below; and Irganox 415, Irganox 565, Irganox 1010, Irganox 1035, Irganox 3114 and Irganox 1098 (trade names; BASF SE). The ultraviolet light absorber is effective for preventing a decrease of the maximum temperature. Preferred examples of the ultraviolet light absorbers include a benzophenone derivative, a benzoate derivative and a triazole derivative, and specific examples include compounds (AO-3) and (AO-4) described below; Tinuvin 329, Tinuvin P, Tinuvin 326, Tinuvin 234, Tinuvin 213, Tinuvin 400, Tinuvin 328 and Tinuvin 99-2 (trade names; BASF SE); and 1,4-diazabicyclo[2.2.2]octane (DABCO).

The light stabilizer such as an amine having steric hindrance is preferred for maintaining the large voltage holding ratio. Specific examples of a preferred light stabilizer include compounds (AO-5), (AO-6) and (AO-7) described below; Tinuvin 144, Tinuvin 765 and Tinuvin 770DF (trade names; BASF SE) ; and LA-77Y and LA-77G (trade names; ADEKA Corporation). The heat stabilizer is also effective for maintaining the large voltage holding ratio, and preferred examples include Irgafos 168 (trade name; BASF SE). A dichroic dye such as an azo dye or an anthraquinone dye is added to the composition to be adapted for a device having a guest host (GH) mode. The antifoaming agent is effective for preventing foam formation. Specific examples of a preferred antifoaming agent include dimethyl silicone oil and methylphenyl silicone oil.

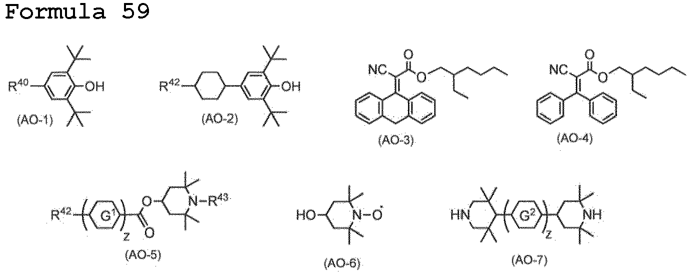

In compound (AO-1), R⁴⁰ is alkyl having 1 to 20 carbons, alkoxy having 1 to 20 carbons, -COOR⁴¹ or -CH₂CH₂COOR⁴¹, in which R⁴¹ is alkyl having 1 to 20 carbons. In compounds (AO-2) and (AO-5), R⁴² is alkyl having 1 to 20 carbons. In compound (AO-5), R⁴³ is hydrogen, methyl or O˙ (oxygen radical); and ring G¹ is 1,4-cyclohexylene or 1,4-phenylene; and in compound (AO-7), ring G² is 1,4-cyclohexylene, 1,4-phenylene or 1,4-phenylene in which at least one hydrogen is replaced by fluorine; and in compounds (AO-5) and (AO-7), z is 1, 2 or 3.

### 4. Liquid crystal display device

The liquid crystal composition can be used for a liquid crystal display device having an operating mode such as the PC mode, the TN mode, the STN mode, the OCB mode and the PSA mode, and driven by an active matrix mode. The composition can also be used in a liquid crystal display device having an operating mode such as the PC mode, the TN mode, the STN mode, the OCB mode, the VA mode and the IPS mode, and driven by a passive matrix mode. The devices can be applied to any of a reflective type, a transmissive type and a transflective type.

The composition is also suitable for a nematic curvilinear aligned phase (NCAP) device, and the composition is microencapsulated herein. The composition can also be used for a polymer dispersed liquid crystal display device (PDLCD) and a polymer network liquid crystal display device (PNLCD) . In the compositions, a lot of polymerizable compounds are added. On the other hand, when a proportion of the polymerizable compound is 10% by weight or less based on the total weight of the liquid crystal composition, the liquid crystal display device having the PSA mode can be prepared. A preferred proportion is in the range of 0.1% by weight to 2% by weight. A further preferred proportion is in the range of 0.2% by weight to 1.0% by weight. The device having the PSA mode can be driven by the driving mode such as the active matrix mode and the passive matrix mode. Such a device can also be applied to any of the reflective type, the transmissive type and the transflective type.

In the polymer sustained alignment mode device, the polymer contained in the composition aligns liquid crystal molecules. The polar compound helps alignment of liquid crystal molecules. More specifically, the polar compound can be used in place of an alignment film. One example of a method for producing such a device is as described below. The device having two substrates referred to as an array substrate and a color filter substrate is prepared. The substrates have no alignment film. At least one of the substrates has an electrode layer. The liquid crystal composition is prepared by mixing the liquid crystal compounds. The polymerizable compound and the polar compound may be added to the composition, and an additive may be further added thereto when necessary. The composition is injected into the device. The device is irradiated with light in a state in which voltage is applied thereto. As a kind of light beams with which the device is irradiated, ultraviolet light is preferred. The polymerizable compound is polymerized by irradiation with light. A composition comprising the polymer is formed by the polymerization, and a device having the PSA mode is prepared.

In the above procedure, the polar group interacts with the substrate surface, and therefore the polar compound is aligned on a substrate. The polar compound aligns liquid crystal molecules. When a plurality of polar groups exist, the interaction with the substrate surface is increased, and thus the alignment at low concentration can be achieved. When voltage is applied, alignment of the liquid crystal molecules is further promoted by action of an electric field. The polymerizable compound is also aligned according to the alignment. The polymerizable compound is polymerized by irradiation with light using ultraviolet light or the like in the above state, and therefore a polymer maintaining the alignment is formed. The alignment of the liquid crystal molecules is additionally stable by an effect of the polymer, and therefore the response time of the device is shortened. The image persistence is caused due to poor operation in the liquid crystal molecules, and therefore the persistence is also simultaneously improved by the effect of the polymer. Compound (1) is polymerizable, and therefore is consumed by polymerization. Moreover, compound (1) is also consumed by copolymerization with any other polymerizable compound. Accordingly, compound (1) has the polar group, but consumed, and therefore a liquid crystal display device having a large voltage holding ratio can be obtained. In addition, if a polar compound having polymerizability is used, both effects of the polar compound and the polymerizable compound can be achieved with one compound, and therefore polymerizable compound having no polar group may occasionally not be required.

### Examples

The invention will be described in greater detail by way of Examples (including Synthesis Examples and Use Examples) . The invention is not limited by the Examples. An embodiment of the invention also includes a mixture prepared by mixing at least two compositions in Use Example.

### 1. Example of compound (1)

Reaction was performed under a nitrogen atmosphere unless otherwise stated. Compound (1) was prepared according to procedures shown in Example 1 and so forth. The thus prepared compound was identified by a method such as an NMR analysis. Characteristics of compound (1), a liquid crystal compound, a composition and a device were measured by methods described below.

NMR analysis: For measurement, DRX-500 made by Bruker BioSpin Corporation was used. In ¹H-NMR measurement, a sample was dissolved in a deuterated solvent such as CDCl₃, and measurement was carried out under conditions of room temperature, 500 MHz and 16 times of accumulation. Tetramethylsilane was used as an internal standard. In ¹⁹F-NMR measurement, CFCl₃ was used as an internal standard, and measurement was carried out under conditions of 24 times of accumulation. In explaining nuclear magnetic resonance spectra obtained, s, d, t, q, quin, sext and m stand for a singlet, a doublet, a triplet, a quartet, a quintet, a sextet and a multiplet, and br being broad, respectively.

Gas chromatographic analysis: For measurement, GC-2010 Gas Chromatograph made by Shimadzu Corporation was used. As a column, a capillary column DB-1 (length 60 m, bore 0.25 mm, film thickness 0.25 µm) made by Agilent Technologies, Inc. was used. As a carrier gas, helium (1 mL/minute) was used. A temperature of a sample vaporizing chamber and a temperature of a detector (FID) part were set to 300°C and 300°C, respectively. A sample was dissolved in acetone and prepared to be a 1 weight % solution, and then 1 microliter of the solution obtained was injected into the sample vaporizing chamber. As a recorder, GC Solution System made by Shimadzu Corporation or the like was used.

HPLC Analysis: For measurement, Prominence (LC-20AD; SPD-20A) made by Shimadzu Corporation was used. As a column, YMC-Pack ODS-A (length 150 mm, bore 4.6 mm, particle diameter 5 µm) made by YMC Co., Ltd. was used. As an eluate, acetonitrile and water were appropriately mixed and used. As a detector, a UV detector, an RI detector, a CORONA detector or the like was appropriately used. When the UV detector was used, a detection wavelength was set at 254 nanometers. A sample was dissolved in acetonitrile and prepared to be a 0.1 weight % solution, and then 1 microliter of the solution was injected into a sample chamber. As a recorder, C-R7Aplus made by Shimadzu Corporation was used.

Ultraviolet-Visible Spectrophotometry: For measurement, PharmaSpec UV-1700 made by Shimadzu Corporation was used. A detection wavelength was adjusted in the range of 190 nanometers to 700 nanometers. A sample was dissolved in acetonitrile and prepared to be a 0.01 mmol/L solution, and measurement was carried out by putting the solution in a quartz cell (optical path length: 1 cm).

Sample for measurement: Upon measuring phase structure and a transition temperature (a clearing point, a melting point, a polymerization starting temperature or the like), a compound itself was used as a sample.

Measuring method: Characteristics were measured according to methods described below. Most of the measuring methods are applied as described in the Standard of Japan Electronics and Information Technology Industries Association (hereinafter abbreviated as JEITA) (JEITA ED-2521B) discussed and established by JEITA, or modified thereon. No thin film transistor (TFT) was attached to a TN device used for measurement.

### (1) Phase structure

A sample was placed on a hot plate in a melting point apparatus (FP-52 Hot Stage made by Mettler-Toledo International Inc.) equipped with a polarizing microscope. A state of phase and a change thereof were observed with the polarizing microscope while the sample was heated at a rate of 3°C per minute, and a kind of the phase was specified.

### (2) Transition temperature (°C)

For measurement, a high sensitivity differential scanning calorimeter, X-DSC7000, made by SSI NanoTechnology Inc. was used. A sample was heated and then cooled at a rate of 3°C per minute, and a starting point of an endothermic peak or an exothermic peak caused by a phase change of the sample was determined by extrapolation, and thus a transition temperature was determined. A melting point and a polymerization starting temperature of a compound were also measured using the apparatus. Temperature at which a compound undergoes transition from a solid to a liquid crystal phase such as the smectic phase and the nematic phase may be occasionally abbreviated as "minimum temperature of the liquid crystal phase." Temperature at which the compound undergoes transition from the liquid crystal phase to liquid may be occasionally abbreviated as "clearing point."

A crystal was expressed as C. When kinds of the crystals were distinguishable, each of the crystals was expressed as C₁ or C₂. The smectic phase or the nematic phase was expressed as S or N. When smectic A phase, smectic B phase, smectic C phase or smectic F phase was distinguishable among the smectic phases, the phases were expressed as S_{A}, S_{B}, S_{C} or S_{F}, respectively. A liquid (isotropic) was expressed as I. A transition temperature was expressed as "C 50.0 N 100.0 I," for example. The expression indicates that a transition temperature from the crystals to the nematic phase is 50.0°C, and a transition temperature from the nematic phase to the liquid is 100.0°C.

### (3) Maximum temperature of nematic phase (T_{NI} or NI; °C)

A sample was placed on a hot plate in a melting point apparatus equipped with a polarizing microscope, and heated at a rate of 1°C per minute. Temperature when part of the sample began to change from a nematic phase to an isotropic liquid was measured. A maximum temperature of the nematic phase may be occasionally abbreviated as "maximum temperature." When the sample was a mixture of compound (1) and the base liquid crystal, the maximum temperature was expressed in terms of a symbol T_{NI}. When the sample was a mixture of compound (1) and a compound such as components B, C and D, the maximum temperature was expressed as a symbol NI.

### (4) Minimum temperature of nematic phase (Tc; °C)

Samples each having a nematic phase were kept in freezers at temperatures of 0°C, -10°C, -20°C, -30°C and -40°C for 10 days, and then liquid crystal phases were observed. For example, when the sample maintained the nematic phase at -20°C and changed to crystals or the smectic phase at -30°C, T_{C} was expressed as T_{C} ≤ -20°C. A minimum temperature of the nematic phase may be occasionally abbreviated as "minimum temperature."

### (5) Viscosity (bulk viscosity; η; measured at 20°C; mPa·s)

For measurement, a cone-plate (E type) rotational viscometer made by Tokyo Keiki Inc. was used.

### (6) Optical anisotropy (refractive index anisotropy; measured at 25°C; Δn)

Measurement was carried out by an Abbe refractometer with a polarizing plate mounted on an ocular, using light at a wavelength of 589 nanometers. A surface of a main prism was rubbed in one direction, and then a sample was added dropwise onto the main prism. A refractive index (n∥) was measured when a direction of polarized light was parallel to a direction of rubbing. A refractive index (n⊥) was measured when the direction of polarized light was perpendicular to the direction of rubbing. A value of optical anisotropy (Δn) was calculated from an equation: Δn = n∥ - n⊥.

### (7) Specific resistance (ρ; measured at 25°C; Ω cm)

Into a vessel equipped with electrodes, 1.0 milliliter of sample was injected. DC voltage (10 V) was applied to the vessel, and DC current after 10 seconds was measured. Specific resistance was calculated from the following equation: (specific resistance) = {(voltage) × (electric capacity of a vessel)} / {(direct current) × (dielectric constant of vacuum)}.

Measurement methods of characteristics may be occasionally different between a sample having positive dielectric anisotropy and a sample having negative dielectric anisotropy. Measurement methods of the sample having positive dielectric anisotropy were described in sections (8a) to (12a). Measurement methods of the sample having negative dielectric anisotropy were described in sections (8b) to (12b) .

### (8a) Viscosity (rotational viscosity; γ1; measured at 25°C; mPa·s)

Positive dielectric anisotropy: Measurement was carried out according to a method described in M. Imai et al., Molecular Crystals and Liquid Crystals, Vol. 259, 37 (1995). A sample was put in a TN device in which a twist angle was 0 degrees and a distance (cell gap) between two glass substrates was 5 micrometers. Voltage was applied stepwise to the device in the range of 16 V to 19.5 V at an increment of 0.5 V. After a period of 0.2 second with no voltage application, voltage was repeatedly applied under conditions of only one rectangular wave (rectangular pulse; 0.2 second) and no voltage application (2 seconds) . Apeak current and a peak time of transient current generated by the applied rectangular waves were measured. A value of rotational viscosity was obtained from the measured values and calculation equation (8) described on page 40 of the paper presented by M. Imai et al. As a value of dielectric anisotropy required for the calculation, a value measured in a section of the dielectric anisotropy as described below using the device in measurement of the rotational viscosity was used.

### (8b) Viscosity (rotational viscosity; γ1; measured at 25°C; mPa·s)

Negative dielectric anisotropy: Measurement was carried out according to a method described in M. Imai et al., Molecular Crystals and Liquid Crystals, Vol. 259, 37 (1995). A sample was put in a VA device in which a distance (cell gap) between two glass substrates was 20 µm. Voltage was applied stepwise to the device in the range of 39 V 50 V at an increment of 1 V. After a period of 0.2 second with no voltage application, voltage was repeatedly applied under conditions of only one rectangular wave (rectangular pulse; 0.2 second) and no voltage application (2 seconds). A peak current and a peak time of transient current generated by the applied rectangular waves were measured. A value of rotational viscosity was obtained from the measured values and calculation equation (8) described on page 40 of the paper presented by M. Imai et al. In dielectric anisotropy required for the calculation, a value measured in a section of the dielectric anisotropy as described below was used.

### (9a) Dielectric anisotropy (Δε; measured at 25°C)

Positive dielectric anisotropy: A value of dielectric anisotropy was calculated from an equation: Δε = ε∥ - ε⊥. A dielectric constant (ε∥ and ε⊥) was measured as described below.

### (1) Measurement of dielectric constant (ε∥) :

A polyimide solution was applied to a well-cleaned glass substrate. After calcining the glass substrate, rubbing treatment was applied to the alignment film obtained. A sample was poured into a TN device in which the distance between the two glass substrates (cell gap) was 9 micrometers and a twist angle was 80 degrees. Sine waves (10 V, 1 kHz) were applied to the device, and after 2 seconds, a dielectric constant (ε∥) of liquid crystal molecules in a major axis direction was measured.

### (2) Measurement of dielectric constant (ε⊥):

An ethanol (20 mL) solution of octadecyltriethoxysilane (0.16 mL) was applied to a well-cleaned glass substrate. After rotating the glass substrate with a spinner, the glass substrate was heated at 150°C for 1 hour. A sample was put in a VA device in which a distance (cell gap) between two glass substrates was 4 micrometers, and the device was sealed with an ultraviolet-curable adhesive. Sine waves (0.5 V, 1 kHz) were applied to the device, and after 2 seconds, a dielectric constant (ε⊥) of liquid crystal molecules in a minor axis direction was measured.

### (9b) Dielectric anisotropy (Δε; measured at 25°C)

Negative dielectric anisotropy: A value of dielectric anisotropy was calculated from an equation: Δε = ε∥ - ε⊥. A dielectric constant (ε∥ and ε⊥) was measured as described below.

### (1) Measurement of dielectric constant (ε∥) :

An ethanol (20 mL) solution of octadecyltriethoxysilane (0.16 mL) was applied to a well-cleaned glass substrate. After rotating the glass substrate with a spinner, the glass substrate was heated at 150°C for 1 hour. A sample was put in a VA device in which a distance (cell gap) between two glass substrates was 4 micrometers, and the device was sealed with an ultraviolet-curable adhesive. Sine waves (0.5 V, 1 kHz) were applied to the device, and after 2 seconds, a dielectric constant (ε∥) of liquid crystal molecules in a major axis direction was measured.

### (2) Measurement of dielectric constant (ε⊥):

A polyimide solution was applied to a well-cleaned glass substrate. After calcining the glass substrate, rubbing treatment was applied to the alignment film obtained. A sample was poured into a TN device in which the distance between the two glass substrates (cell gap) was 9 micrometers and a twist angle was 80 degrees. Sine waves (0.5 V, 1 kHz) were applied to the device, and after 2 seconds, a dielectric constant (ε⊥) of liquid crystal molecules in a minor axis direction was measured.

### (10a) Elastic constant (K; measured at 25°C; pN)

Positive dielectric anisotropy: For measurement, HP4284A LCR Meter made by Yokogawa-Hewlett-Packard Co. was used. A sample was poured into a horizontal alignment device in which the distance between the two glass substrates (cell gap) was 20 micrometers. An electric charge of 0 V to 20 V was applied to the device, and electrostatic capacity and applied voltage were measured. The measured values of electrostatic capacity (C) and applied voltage (V) were fitted to equation (2.98) and equation (2.101) on page 75 of "Liquid Crystal Device Handbook" (Ekisho Debaisu Handobukku in Japanese; Nikkan Kogyo Shimbun, Ltd.), and values of K₁₁ and K₃₃ were obtained from equation (2.99). Next, K₂₂ was calculated using the previously determined values of K₁₁ and K₃₃ in equation (3.18) on page 171. Elastic constant K was expressed in terms of a mean value of the thus determined K₁₁, K₂₂ and K₃₃.

### (10b) Elastic constant (K₁₁ and K₃₃; measured at 25°C; pN)

Negative dielectric anisotropy: For measurement, Elastic Constant Measurement System Model EC-1 made by TOYO Corporation was used. A sample was put in a vertical alignment device in which a distance (cell gap) between two glass substrates was 20 micrometers. An electric charge of 20 V to 0 V was applied to the device, and electrostatic capacity and applied voltage were measured. The measured values of electrostatic capacity (C) and applied voltage (V) were fitted to equation (2.98) and equation (2.101) on page 75 of "Liquid Crystal Device Handbook" (Ekisho Debaisu Handobukku, in Japanese; Nikkan Kogyo Shimbun, Ltd.), and values of elastic constant were obtained from equation (2.100).

### (11a) Threshold voltage (Vth; measured at 25°C; V)

Positive dielectric anisotropy: For measurement, an LCD5100 luminance meter made by Otsuka Electronics Co., Ltd. was used. A light source was a halogen lamp. A sample was poured into a TN device with a normally white mode in which the distance between the two glass substrates (cell gap) was 0.45/Δn (µm) and a twist angle was 80 degrees. The voltage to be applied to this device (32 Hz, rectangular waves) was stepwise increased in 0.02 V increments from 0 V up to 10 V. On the occasion, the device was irradiated with light from a direction perpendicular to the device, and an amount of light transmitted through the device was measured. A voltage-transmittance curve was prepared, in which the maximum amount of light corresponds to 100% transmittance and the minimum amount of light corresponds to 0% transmittance. A threshold voltage is expressed in terms of a voltage at 90% transmittance.

### (11b) Threshold voltage (Vth; measured at 25°C; V)

Negative dielectric anisotropy: For measurement, an LCD5100 luminance meter made by Otsuka Electronics Co., Ltd. was used. A light source was a halogen lamp. A sample was put in a normally black mode VA device in which a distance (cell gap) between two glass substrates was 4 micrometers and a rubbing direction was anti-parallel, and the device was sealed with an ultraviolet-curable adhesive. A voltage (60 Hz, rectangular waves) to be applied to the device was stepwise increased from 0 V to 20 V at an increment of 0.02 V. On the occasion, the device was irradiated with light from a direction perpendicular to the device, and an amount of light transmitted through the device was measured. A voltage-transmittance curve was prepared, in which the maximum amount of light corresponds to 100% transmittance and the minimum amount of light corresponds to 0% transmittance. Threshold voltage was expressed in terms of voltage at 10% transmittance.

### (12a) Response time (τ; measured at 25°C; ms)

Positive dielectric anisotropy: For measurement, an LCD5100 luminance meter made by Otsuka Electronics Co., Ltd. was used. A light source was a halogen lamp. A low-pass filter was set to 5 kHz. A sample was poured into a TN device with a normally white mode in which the distance between the two glass substrates (cell gap) was 5.0 micrometers and a twist angle was 80 degrees. A voltage (rectangular waves; 60 Hz, 5 V, 0.5 second) was applied to the device. On the occasion, the device was irradiated with light from a direction perpendicular to the device, and an amount of light transmitted through the device was measured. The maximum amount of light corresponds to 100% transmittance, and the minimum amount of light corresponds to 0% transmittance. A rise time (τr; millisecond) was expressed in terms of time required for a change from 90% transmittance to 10% transmittance. A fall time (τf; millisecond) was expressed in terms of time required for a change from 10% transmittance to 90% transmittance. A response time was expressed by a sum of the rise time and the fall time thus obtained.

### (12b) Response time (τ; measured at 25°C; ms)

Negative dielectric anisotropy: For measurement, an LCD5100 luminance meter made by Otsuka Electronics Co., Ltd. was used. A light source was a halogen lamp. A low-pass filter was set to 5 kHz. A sample was put in a normally black mode PVA device in which a distance (cell gap) between two glass substrates was 3.2 micrometers and a rubbing direction was anti-parallel. The device was sealed with an ultraviolet-curable adhesive. Voltage having a degree of slightly exceeding threshold voltage was applied to the device for 1 minute, and then the device was irradiated with ultraviolet light of 23.5 mW/cm2 for 8 minutes while voltage of 5.6 V was applied to the device. Rectangular waves (60 Hz, 10 V, 0.5 second) were applied to the device. On the occasion, the device was irradiated with light from a direction perpendicular to the device, and an amount of light transmitted through the device was measured. The maximum amount of light corresponds to 100% transmittance, and the minimum amount of light corresponds to 0% transmittance. A response time was expressed in terms of time required for a change from 90% transmittance to 10% transmittance (fall time; millisecond).

### (13) Voltage holding ratio

The polymerizable compound was polymerized by irradiating the device with ultraviolet light using a black light, F40T10/BL (peak wavelength of 369 nm) made by EYE GRAPHICS CO., LTD. The device was charged by applying a pulse voltage (60 microseconds at 1 V) at 60°C. A decaying voltage was measured for 1.67 seconds with a high-speed voltmeter, and area A between a voltage curve and a horizontal axis in a unit cycle was determined. Area B is an area without decay. A voltage holding ratio is expressed in terms of a percentage of area A to area B.

### Raw material

Solmix (registered trademark) A-11 is a mixture of ethanol (85.5%), methanol (13.4%) and isopropanol (1.1%), and was purchased from Japan Alcohol Trading Co., Ltd.

### Synthesis Example 1

### Synthesis of compound (1-2-30)

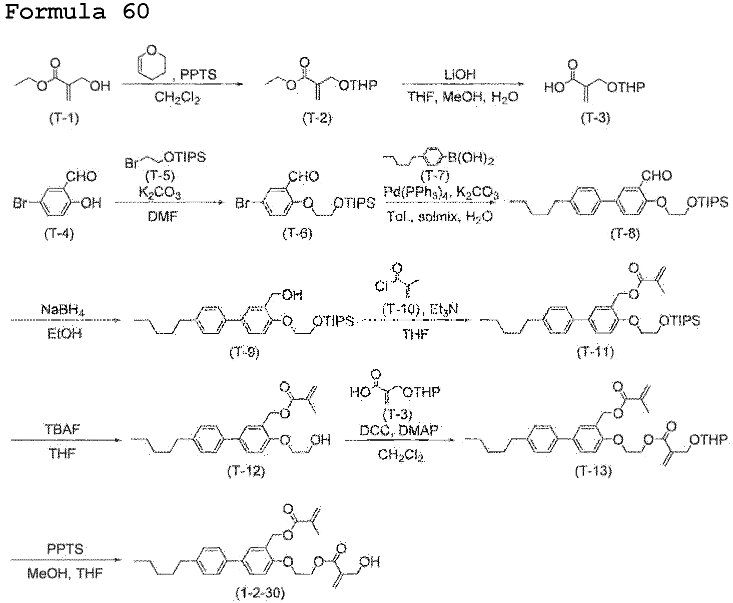

### First step

Compound (T-1) (30.0 g), 3,4-dihydro-2H-pyran (23.3 g) and pyridinium p-toluenesulfonate (PPTS) (5.80 g) were put in a reaction vessel, and the resulting mixture was stirred at 50°C for 10 hours. An insoluble matter was filtered off, and then the resulting reaction mixture was poured into water, and an aqueous layer was subjected to extraction with dichloromethane. Combined organic layers were washed with water, and dried over anhydrous magnesium sulfate. The solution was concentrated under reduced pressure, and the residue was purified by silica gel chromatography (heptane : ethyl acetate = 2 : 1 in a volume ratio of a solvent) to obtain compound (T-2) (39.5 g; 80%).

### Second step

Compound (T-2) (39.5 g), THF (400 mL) and water (400 mL) were put in a reaction vessel, and the resulting mixture was cooled down to 0°C. Thereto, lithium hydroxide monohydrate (15.4 g) was added, and the resulting mixture was stirred for 12 hours while returning to room temperature. The resulting reaction mixture was poured into water, and 6 N hydrochloric acid (60 mL) was slowly added to acidify the resulting mixture, and then an aqueous layer was subjected to extraction with ethyl acetate. Combined organic layers were washed with water, and dried over anhydrous magnesium sulfate. The solution was concentrated under reduced pressure to obtain compound (T-3) (32.6 g; 95%) .

### Third step

Compound (T-4) (40.0 g), compound (T-5) (61.6 g), potassium carbonate (55.0 g) and DMF (400 mL) were put in a reaction vessel, and the resulting mixture was stirred at 100°C for 2 hours. The resulting reaction mixture was poured into water, and an aqueous layer was subjected to extraction with toluene. Combined organic layers were washed with brine, and dried over anhydrous magnesium sulfate. The solution was concentrated under reduced pressure, and the residue was purified by silica gel chromatography (heptane : toluene = 1 : 1 in a volume ratio of a solvent) to obtain compound (T-6) (75.0 g; 94%) .

### Fourth step

Compound (T-6) (20.0 g), compound (T-7) (11.5 g), potassium carbonate (13.8 g), tetrakis(triphenylphosphine)palladium (1.73 g), toluene (250 mL), Solmix (registered trademark) A-11 (250 mL) and water (50.0 mL) were put in a reaction vessel, and the resulting mixture was refluxed under heating at 75°C for 3 hours. The resulting reaction mixture was poured into water, and an aqueous layer was subjected to extraction with toluene. Combined organic layers were washed with water and brine in the order, and dried over anhydrous magnesium sulfate. The solution was concentrated under reduced pressure, and the residue was purified by silica gel chromatography (heptane : toluene = 1 : 1 in a volume ratio of a solvent) to obtain compound (T-8) (20.5 g; 88%) .

### Fifth step

Sodium borohydride (1.02 g) and ethanol (100 mL) were put in a reaction vessel, and the resulting mixture was cooled down to 0°C. Thereto, an ethanol (200 mL) solution of compound (T-8) (20.5 g) was slowly added dropwise, and the resulting mixture was stirred for 8 hours while returning to room temperature. The resulting reaction mixture was poured into water, and an aqueous layer was subjected to extraction with ethyl acetate. Combined organic layers were washed with water and brine in the order, and dried over anhydrous magnesium sulfate. The solution was concentrated under reduced pressure, and the residue was purified by silica gel chromatography (toluene : ethyl acetate = 4 : 1 in a volume ratio of a solvent) to obtain compound (T-9) (19.0 g; 90%).

### Sixth step

Compound (T-9) (19.0 g), triethylamine (6.8 mL) and THF (300 mL) were put in a reaction vessel, and the resulting mixture was cooled down to 0°C. Thereto, compound (T-10) (4.6 mL) was slowly added dropwise, and the resulting mixture was stirred for 4 hours while returning to room temperature. The resulting reaction mixture was poured into water, and an aqueous layer was subjected to extraction with toluene. Combined organic layers were washed with water and brine in the order, and dried over anhydrous magnesium sulfate. The solution was concentrated under reduced pressure, and the residue was purified by silica gel chromatography (heptane : toluene = 4 : 1 in a volume ratio of a solvent) to obtain compound (T-11) (17.5 g; 81%).

### Seventh step

Compound (T-11) (17.5 g) and THF (300 mL) were put in a reaction vessel, and the resulting mixture was cooled down to 0°C. Thereto, tetrabutylammonium fluoride (TBAF) (1.00 M; THF solution; 39.0 mL) was slowly added dropwise, and the resulting mixture was stirred for 3 hours while returning to room temperature. The resulting reaction mixture was poured into water, and an aqueous layer was subjected to extraction with ethyl acetate. Combined organic layers were washed with brine, and dried over anhydrous magnesium sulfate. The solution was concentrated under reduced pressure, and the residue was purified by silica gel chromatography (toluene : ethyl acetate = 4 : 1 in a volume ratio of a solvent) to obtain compound (T-12) (11.0 g; 89%).

### Eighth step

Compound (T-12) (7.00 g), compound (T-3) (4.09 g), DMAP (1.12 g) and dichloromethane (100 mL) were put in a reaction vessel, and the resulting mixture was cooled down to 0°C. Thereto, a dichloromethane (25.0 mL) solution of DCC (4.53 g) was slowly added dropwise, and the resulting mixture was stirred for 12 hours while returning to room temperature. An insoluble matter was filtered off, and then the resulting reaction mixture was poured into water, and an aqueous layer was subjected to extraction with dichloromethane. Combined organic layers were washed with water, and dried over anhydrous magnesium sulfate. The solution was concentrated under reduced pressure, and the residue was purified by silica gel chromatography (toluene : ethyl acetate = 9 : 1 in a volume ratio of a solvent) to obtain compound (T-13) (8.20 g; 81%).

### Ninth step

Compound (T-13) (8.00 g), PPTS (1.83 g), THF (100 mL) and methanol (100 mL) were put in a reaction vessel, and the resulting mixture was stirred at 50°C for 5 hours. The resulting reaction mixture was poured into water, and an aqueous layer was subjected to extraction with ethyl acetate. Combined organic layers were washed with water, and dried over anhydrous magnesium sulfate. The solution was concentrated under reduced pressure, and the residue was purified by silica gel chromatography (toluene : ethyl acetate = 4 : 1 in a volume ratio of a solvent) to obtain compound (1-2-30) (4.00 g; 59%).

An NMR analysis value of the resulting compound (1-2-30) was as described below.
¹H-NMR: chemical shift δ (ppm; CDCl₃): 7.58 (d, J = 2.3 Hz, 1H), 7.50 (dd, J = 8.6 Hz, J = 2.4 Hz, 1H), 7.46 (d, J = 8.1 Hz, 2H), 7.23 (d, J = 8.1 Hz, 2H), 6.94 (d, J = 8.6 Hz, 1H), 6.29 (s, 1H), 6.16 (s, 1H), 5.87 (d, J = 1.2 Hz, 1H), 5.58 (t, J = 1.7 Hz, 1H), 5.32 (s, 2H), 4.56 (t, J = 4.4 Hz, 2H), 4.35 (d, J = 6.4 Hz, 2H), 4.29 (t, J = 4.5 Hz, 2H), 2.63 (t, J = 7.9 Hz, 2H), 2.54 (t, J = 5.9 Hz, 1H), 1.97 (s, 3H), 1.68 - 1.60 (m, 2H), 1.40 - 1.31 (m, 4H), 0.90 (t, J = 6.9 Hz, 3H).

### Synthesis Example 2

### Synthesis of compound (1-3-31)

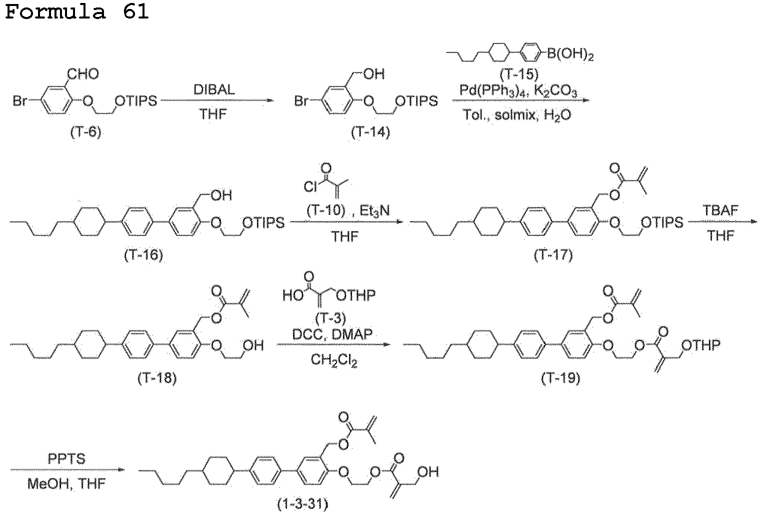

### First step

Compound (T-6) (55.0 g) and THF (550 mL) were put in a reaction vessel, and the resulting mixture was cooled down to 0°C. Thereto, didiisobutylaluminum hydride (1.02 M; n-hexane solution; 164 mL) was slowly added dropwise, and the resulting mixture was stirred for 5 hours while returning to room temperature. The resulting reaction mixture was poured into water, and an insoluble matter was filtered off, and then an aqueous layer was subjected to extraction with ethyl acetate. Combined organic layers were washed with brine, and dried over anhydrous magnesium sulfate. The solution was concentrated under reduced pressure, and the residue was purified by silica gel chromatography (toluene : ethyl acetate = 5 : 1 in a volume ratio of a solvent) to obtain compound (T-14) (52.0 g; 94%).

### Second step

Compound (T-14) (6.50 g), compound (T-15) (5.30 g), potassium carbonate (4.45 g), tetrakis(triphenylphosphine)palladium (0.559 g), toluene (30.0 mL), Solmix (registered trademark) A-11 (30.0 mL) and water (6.0 mL) were put in a reaction vessel, and the resulting mixture was refluxed under heating at 75°C for 5 hours. The resulting reaction mixture was poured into water, and an aqueous layer was subjected to extraction with toluene. Combined organic layers were washed with water and brine in the order, and dried over anhydrous magnesium sulfate. The solution was concentrated under reduced pressure, and the residue was purified by silica gel chromatography (toluene : ethyl acetate = 5 : 1 in a volume ratio of a solvent) to obtain compound (T-16) (7.80 g; 88%).

### Third step

Compound (T-17) (7.20 g; 82%) was obtained by using compound (T-16) (7.80 g) as a raw material in the same technique as in the sixth step in Synthesis Example 1.

### Fourth step

Compound (T-18) (4.80 g; 89%) was obtained by using compound (T-17) (7.20 g) as a raw material in the same technique as in the seventh step in Synthesis Example 1.

### Fifth step

Compound (T-19) (5.20 g; 80%) was obtained by using compound (T-18) (4.80 g) as a raw material in the same technique as in the eighth step in Synthesis Example 1.

### Sixth step

Compound (1-3-31) (3.20 g; 71%) was obtained by using compound (T-19) (5.20 g) as a raw material in the same technique as in the ninth step in Synthesis Example 1.

An NMR analysis value of the resulting compound (1-3-31) was as described below.
¹H-NMR: chemical shift δ (ppm; CDCl₃): 7.58 (d, J = 2.4 Hz, 1H), 7.50 (dd, J = 8.5 Hz, J = 2.2 Hz, 1H), 7.46 (d, J = 8.2 Hz, 2H), 7.27 (d, J = 8.3 Hz, 2H), 6.95 (d, J = 8.6 Hz, 1H), 6.30 (s, 1H), 6.16 (s, 1H), 5.87 (d, J = 1.1 Hz, 1H), 5.58 (t, J = 1.8 Hz, 1H), 5.29 (s, 2H), 4.57 (t, J = 4.9 Hz, 2H), 4.35 (d, J = 6.7 Hz, 2H), 4.30 (t, J = 4.5 Hz, 2H), 2.49 (tt, J = 6.2 Hz, J = 3.0 Hz, 2H), 2.37 (t, J = 6.5 Hz, 1H), 1.97 (s, 3H), 1.96 - 1.85 (m, 4H), 1.53 - 1.42 (m, 2H), 1.38 - 1.19 (m, 8H), 1.12 - 1.01 (m, 2H), 0.90 (t, J = 6.9 Hz, 3H).

Physical properties of compound (1-3-31) were as described below. Transition temperature: C 52.5 I.

### Synthesis Example 3

### Synthesis of compound (1-3-44)

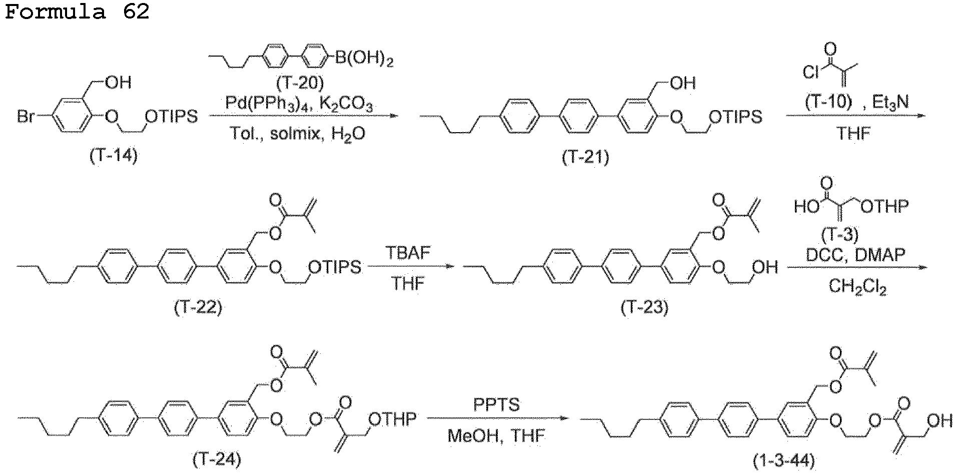

### First step

Compound (T-21) (10.5 g; 81%) was obtained by using compound (T-14) (9.60 g) and compound (T-20) (10.0 g) as raw material in the same technique as in the second step in Synthesis Example 2.

### Second step

Compound (T-22) (9.00 g; 76%) was obtained by using compound (T-21) (10.5 g) as a raw material in the same technique as in the sixth step in Synthesis Example 1.

### Third step

Compound (T-23) (5.95 g; 89%) was obtained by using compound (T-22) (9.00 g) as a raw material in the same technique as in the seventh step in Synthesis Example 1.

### Fourth step

Compound (T-24) (6.10 g; 76%) was obtained by using compound (T-23) (5.95 g) as a raw material in the same technique as in the eighth step in Synthesis Example 1.

### Fifth step

Compound (1-3-44) (5.00 g; 95%) was obtained by using compound (T-24) (6.10 g) as a raw material in the same technique as in the ninth step in Synthesis Example 1.

An NMR analysis value of the resulting compound (1-3-44) was as described below.
¹H-NMR: chemical shift δ (ppm; CDCl₃) : 7.68 - 7.52 (m, 8H), 7.29 - 7.24 (m, 2H), 6.98 (d, J = 8.5 Hz, 1H), 6.31 (s, 1H), 6.17 (s, 1H), 5.88 (s, 1H), 5.59 (t, J = 1.2 Hz, 1H), 5.31 (s, 2H), 4.58 (t, J = 4.7 Hz, 2H), 4.36 (d, J = 6.7 Hz, 2H), 4.31 (t, J = 4.6 Hz, 2H), 2.65 (t, J = 7.7 Hz, 2H), 2.41 (t, J = 6.7 Hz, 1H), 1.99 (s, 3H), 1.70 - 1.63 (m, 2H), 1.40 - 1.32 (m, 4H), 0.91 (t, J = 6.6 Hz, 3H) .

Physical properties of compound (1-3-44) were as described below. Transition temperature: C 84.6 I.

### Synthesis Example 4

### Synthesis of compound (1-3-51)

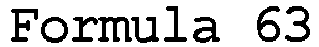

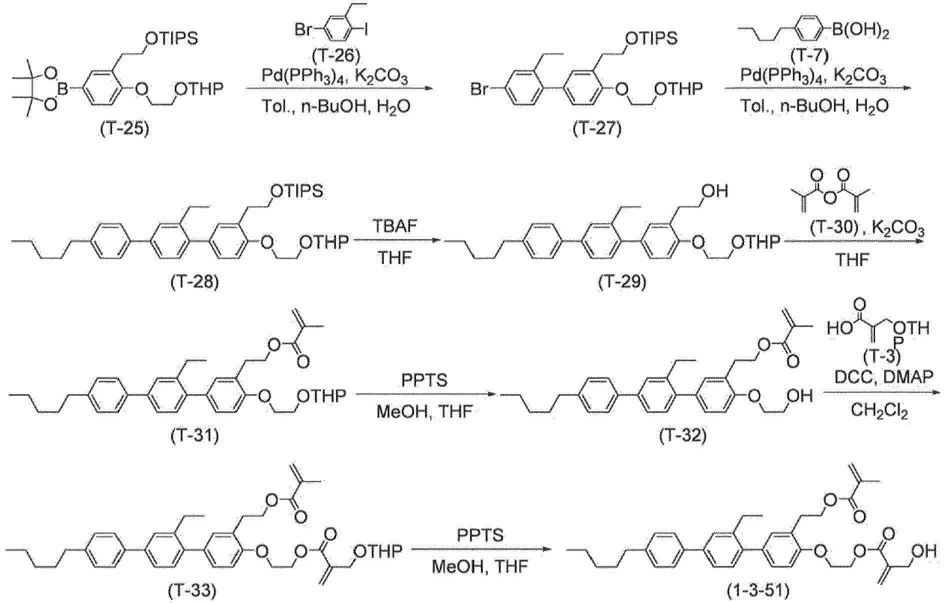

### First step

Compound (T-25) (57.3 g) prepared according to the technique described in WO 2016/114093 A, compound (T-26) (32.5 g), potassium carbonate (28.9 g), tetrakis(triphenylphosphine)palladium (1.21 g), toluene (160 mL), n-butanol (160 mL) and water (160 mL) were put in a reaction vessel, and the resulting mixture was refluxed under heating at 89°C for 10 hours. The resulting reaction mixture was poured into water, and an aqueous layer was subjected to extraction with toluene. Combined organic layers were washed with water and brine in the order, and dried over anhydrous magnesium sulfate. The solution was concentrated under reduced pressure, and the residue was purified by silica gel chromatography (heptane : toluene = 1 : 2 in a volume ratio of a solvent) to obtain compound (T-27) (46.8 g; 74%).

### Second step

Compound (T-27) (46.8 g), compound (T-7) (16.3 g), potassium carbonate (21.4 g), tetrakis(triphenylphosphine)palladium (1.34 g), toluene (210 mL), n-butanol (210 mL) and water (210 mL) were put in a reaction vessel, and the resulting mixture was refluxed under heating at 89°C for 3 hours. The resulting reaction mixture was poured into water, and an aqueous layer was subjected to extraction with toluene. Combined organic layers were washed with water and brine in the order, and dried over anhydrous magnesium sulfate. The solution was concentrated under reduced pressure, and the residue was purified by silica gel chromatography (heptane : ethyl acetate = 9 : 1 in a volume ratio of a solvent) to obtain compound (T-28) (49.2 g; 95%).

### Third step

Compound (T-29) (33.8 g; 89%) was obtained by using compound (T-28) (49.2 g) as a raw material in the same technique as in the seventh step in Synthesis Example 1.

### Fourth step

Compound (T-29) (18.8 g), potassium carbonate (13.1 g) and THF (280 mL) were put in a reaction vessel, and the resulting mixture was heated at 30°C. Thereto, compound (T-30) (14.4 mL) was slowly added dropwise, and the resulting mixture was stirred at 50°C for 20 hours. The resulting reaction mixture was poured into water, and an aqueous layer was subjected to extraction with ethyl acetate. Combined organic layers were washed with water and brine in the order, and dried over anhydrous magnesium sulfate. The solution was concentrated under reduced pressure, and the residue was purified by silica gel chromatography (heptane : ethyl acetate = 6 : 1 in a volume ratio of a solvent) to obtain compound (T-31) (17.6 g; 83%).

### Fifth step

Compound (T-32) (12.1 g; 80%) was obtained by using compound (T-31) (17.6 g) as a raw material in the same technique as in the ninth step in Synthesis Example 1.

### Sixth step

Compound (T-33) (1.63 g; 78%) was obtained by using compound (T-32) (1.56 g) as a raw material in the same technique as in the eighth step in Synthesis Example 1.

### Seventh step

Compound (1-3-51) (1.04 g; 73%) was obtained by using compound (T-33) (1.63 g) as a raw material in the same technique as in the ninth step in Synthesis Example 1.

An NMR analysis value of the resulting compound (1-3-51) was as described below.
¹H-NMR: chemical shift δ (ppm; CDCl₃) : 7.58 - 7.53 (m, 2H), 7.50 (d, J = 1.8 Hz, 1H), 7.43 (dd, J = 7.8 Hz, J = 1.9 Hz, 1H), 7.29 - 7.24 (m, 2H), 7.23 - 7.15 (m, 3H), 6.89 (d, J = 8.3 Hz, 1H), 6.33 (d, J = 0.7 Hz, 1H), 6.06 (s, 1H), 5.90 (d, J = 1.1 Hz, 1H), 5.52 (t, J = 1.4 Hz, 1H), 4.62 (t, J = 4.9 Hz, 2H), 4.38 - 4.34 (m, 4H), 4.30 (t, J = 4.5 Hz, 2H), 3.04 (t, J = 6.8 Hz, 2H), 2.69 - 2.62 (m, 4H), 2.53 (t, J = 6.5 Hz, 1H), 1.90 (s, 3H), 1.72 - 1.61 (m, 2H), 1.42 - 1.33 (m, 4H), 1.13 (t, J = 7.6 Hz, 3H), 0.91 (t, J = 6.8 Hz, 3H) .

Physical properties of compound (1-3-51) were as described below. Transition temperature: C 64.6 I.

### Synthesis Example 5

### Synthesis of compound (1-3-107)

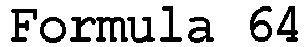

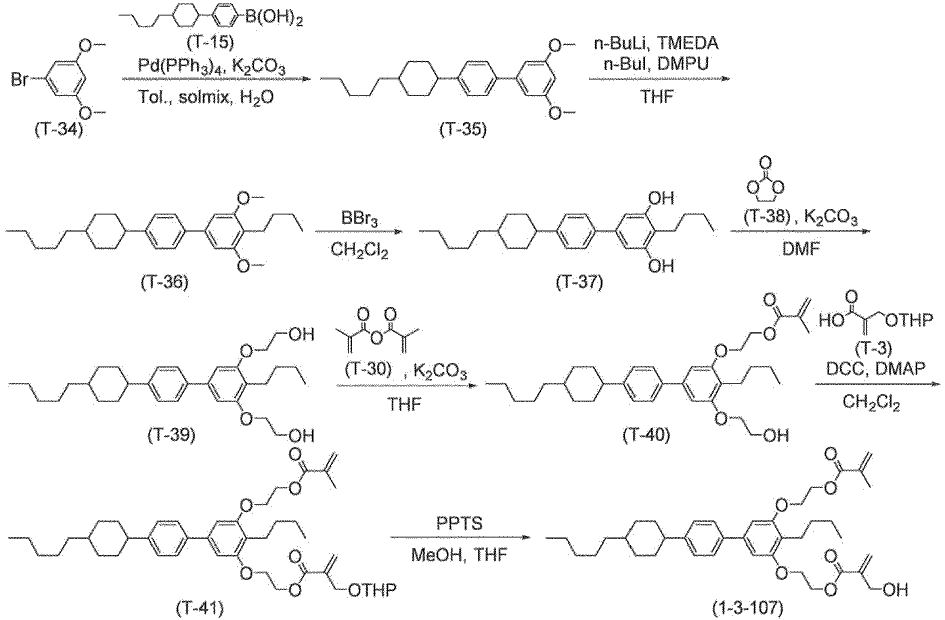

### First step

Compound (T-35) (33.4 g; 87%) was obtained by using compound (T-34) (37.3 g) as a raw material in the same technique as in the second step in Synthesis Example 2.

### Second step

Compound (T-35) (22.0 g), N,N,N',N'-tetramethylethylenediamine (TMEDA) (10.4 mL) and THF (440 mL) were put in a reaction vessel, and the resulting mixture was cooled down to 0°C. Thereto, n-butyllithium (1.55 M; n-hexane solution; 46.5 mL) was slowly added dropwise, and the resulting mixture was stirred at 0°C for 30 minutes. Thereto, 1-iodobutane (8.2 mL) and N,N'-dimethylpropylene urea (DMPU) (8.7 mL) were added dropwise in the order, and the resulting mixture was stirred for 1 hour while returning to room temperature. The resulting reaction mixture was poured into water, and an aqueous layer was subjected to extraction with toluene. Combined organic layers were washed with water, and dried over anhydrous magnesium sulfate. The solution was concentrated under reduced pressure, and the residue was purified by silica gel chromatography (heptane : toluene = 5 : 1 in a volume ratio of a solvent) to obtain compound (T-36) (18.7 g; 74%).

### Third step

Compound (T-36) (18.7 g) and dichloromethane (375 mL) were put in a reaction vessel, and the resulting mixture was cooled down to -50°C. Thereto, boron tribromide (1.00M; dichloromethane solution; 97.3 mL) was slowly added dropwise, and the resulting mixture was stirred for 12 hours while returning to room temperature. The resulting reaction mixture was poured into water, and an organic layer was washed with water, and dried over anhydrous magnesium sulfate. The solution was concentrated under reduced pressure, and the residue was purified by recrystallization from (heptane : toluene = 1 : 1 in a volume ratio of a solvent) to obtain compound (T-37) (16.4 g; 94%).

### Fourth step

Compound (T-37) (16.4 g), potassium carbonate (23.0 g) and DMF (330 mL) were put in a reaction vessel, and the resulting mixture was stirred at 110°C for 30 minutes. Thereto, compound (T-38) (9.15 g) was added, and the resulting mixture was stirred at 135°C for 3 hours. The resulting reaction mixture was poured into water, and an aqueous layer was subjected to extraction with ethyl acetate. Combined organic layers were washed with water, and dried over anhydrous magnesium sulfate. The solution was concentrated under reduced pressure, and the residue was purified by recrystallization from (heptane : toluene = 1 : 1 in a volume ratio of a solvent) to obtain compound (T-39) (19.2 g; 96%).

### Fifth step

Compound (T-40) (5.13 g; 45%) was obtained by using compound (T-39) (10.0 g) as a raw material in the same technique as in the fourth step in Synthesis Example 4.

### Sixth step

Compound (T-41) (5.98 g; 89%) was obtained by using compound (T-40) (5.13 g) as a raw material in the same technique as in the eighth step in Synthesis Example 1.

### Seventh step

Compound (1-3-107) (4.25 g; 80%) was obtained by using compound (T-41) (5.98 g) as a raw material in the same technique as in the ninth step in Synthesis Example 1.

An NMR analysis value of the resulting compound (1-3-107) was as described below.
¹H-NMR: chemical shift δ (ppm; CDCl₃): 7.46 (d, J = 8.1 Hz, 2H), 7.27 (d, J = 7.5 Hz, 2H), 6.71 (d, J = 3.3 Hz, 2H), 6.30 (s, 1H), 6.14 (s, 1H), 5.86 (d, J = 1.0 Hz, 1H), 5.58 (t, J = 1.4 Hz, 1H), 4.59 - 4.49 (m, 4H), 4.34 (d, J = 6.6 Hz, 2H), 4.30 - 4, 23 (m, 4H), 2.66 (t, J = 7.6 Hz, 2H), 2.51 (tt, J = 12.1 Hz, J = 3.1 Hz, 1H), 2.19 (t, J = 6.6 Hz, 1H), 1.98 - 1.84 (m, 7H), 1.53 - 1.42 (m, 4H), 1.38 - 1.19 (m, 11H), 1.12 - 1.00 (m, 2H), 0.93 - 0.84 (m, 3H).

Physical properties of compound (1-3-107) were as described below. Transition temperature: C 58.0 I.

### Synthesis Example 6

### Synthesis of compound (1-3-131)

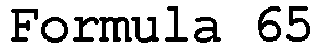

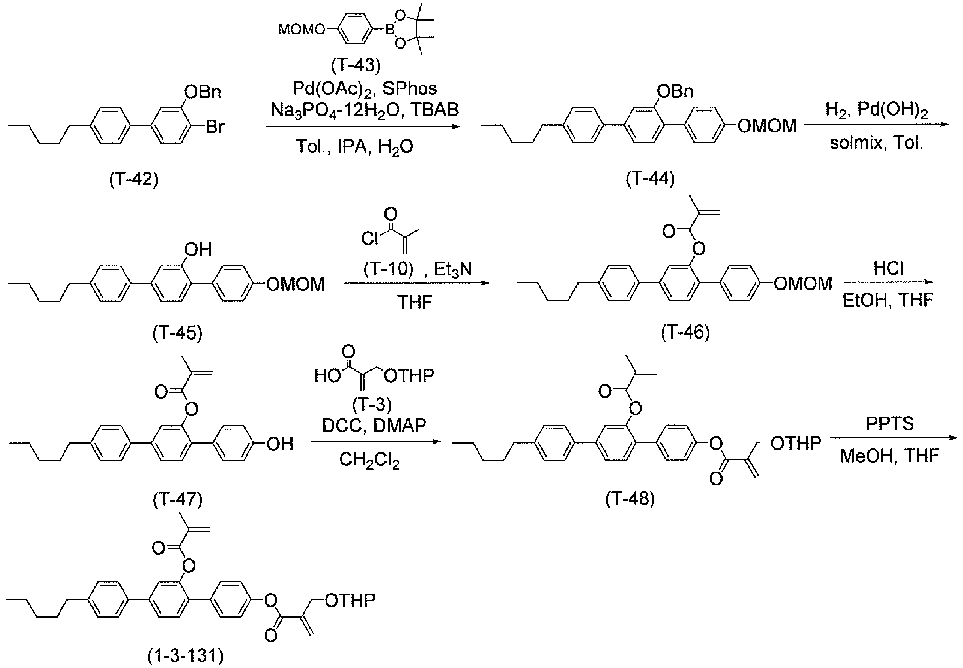

### First step

Compound (T-42) (11.0 g), compound (T-43) (7.81 g), palladium acetate (0.0302 g), 2-dicyclohexylphosphino-2',6'-dimethoxybiphenyl (SPhos) (0.110 g), trisodium phosphate dodecahydrate (20.4 g), tetrabutylammonium bromide (TBAB) (2.60 g), toluene (55.0 mL), IPA (55.0 mL) and water (55.0 mL) were put in a reaction vessel, and the resulting mixture was refluxed under heating at 80°C for 8 hours. The resulting reaction mixture was poured into water, and an aqueous layer was subjected to extraction with toluene. Combined organic layers were washed with water and brine in the order, and dried over anhydrous magnesium sulfate. The solution was concentrated under reduced pressure, and the residue was purified by silica gel chromatography (solvent: toluene) to obtain compound (T-44) (7.00 g; 56%).

### Second step

Compound (T-44) (7.00 g), palladium hydroxide (0.126 g) and Solmix (registered trademark) A-11 (56.0 mL) and toluene (14.0 mL) were put in a reaction vessel, and the resulting mixture was stirred at room temperature for 98 hours under a hydrogen atmosphere. A catalyst was removed therefrom by filtration, and then the resulting solution was concentrated under reduced pressure, and the residue was purified by silica gel chromatography (heptane : ethyl acetate = 4 : 1 in a volume ratio of a solvent) to obtain compound (T-45) (4.26 g; 75%).

### Third step

Compound (T-46) (3.23 g; 64%) was obtained by using compound (T-45) (4.26 g) as a raw material in the same technique as in the sixth step in Synthesis Example 1.

### Fourth step

Compound (T-46) (5.73 g), 6 N hydrochloric acid (6.40 mL), ethanol (55.0 mL) and THF (55.0 mL) were put in a reaction vessel, and the resulting mixture was stirred at 60°C for 4 hours. The resulting reaction mixture was poured into water, and an aqueous layer was subjected to extraction with ethyl acetate. Combined organic layers were washed with water, and dried over anhydrous magnesium sulfate. The solution was concentrated under reduced pressure, and the residue was purified by silica gel chromatography (heptane : ethyl acetate = 9 : 1 in a volume ratio of a solvent) to obtain compound (T-47) (5.00 g; 96%).

### Fifth step

Compound (T-48) (5.40 g; 76%) was obtained by using compound (T-47) (5.00 g) as a raw material in the same technique as in the eighth step in Synthesis Example 1.

### Sixth step

Compound (1-3-131) (2.70 g; 59%) was obtained by using compound (T-48) (5.40 g) as a raw material in the same technique as in the ninth step in Synthesis Example 1.

An NMR analysis value of the resulting compound (1-3-131) was as described below.
¹H-NMR: chemical shift δ (ppm; CDCl₃) 7.58 - 7.56 (m, 2H), 7.53 - 7.51 (m, 2H), 7.44 - 7.43 (m, 1H), 7.29 - 7.28 (m, 4H), 7.20 (d, J = 8.7 Hz, 2H), 6.53 (s, 1H), 6.20 (s, 1H), 6.10 (s, 1H), 5.52 (s, 1H), 4.50 - 4.49 (m, 2H)), 2.69 - 2.65 (m, 2H), 2.23 - 2.20 (m, 1H), 1.72 - 1.65 (m, 2H), 1.40 - 1.35 (m, 4H), 0.94 (t, J = 6.1 Hz, 3H).

Physical properties of compound (1-3-131) were as described below. Transition temperature: C 78.7 C 94.4 I.

### Synthesis Example 7

### Synthesis of compound (1-3-25)

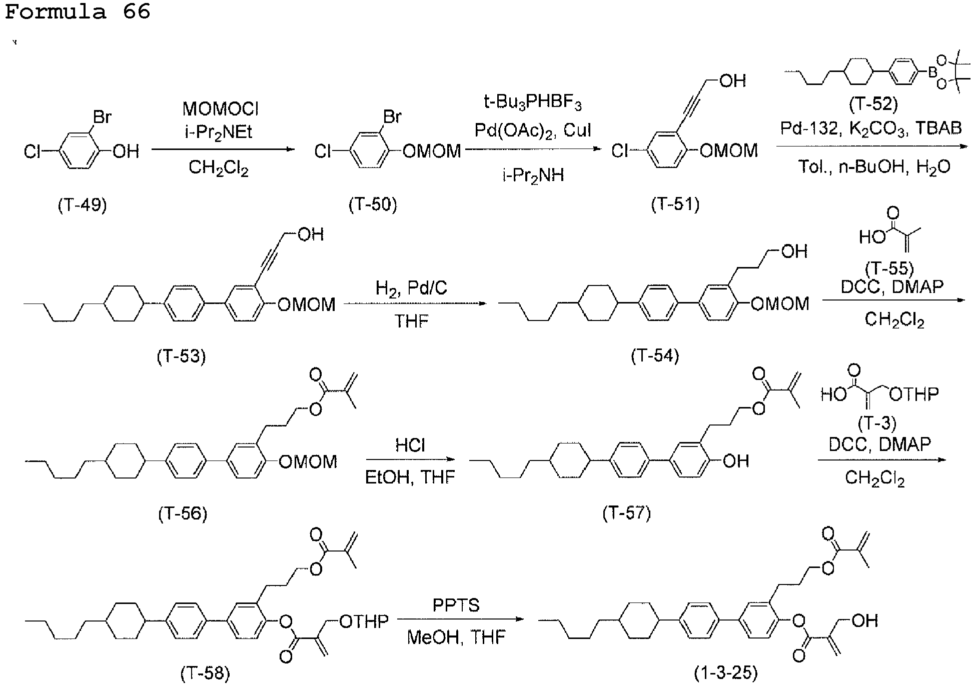

### First step

Compound (T-49) (25.0 g), diisopropylethylamine (26.6 mL) and dichloromethane (200 mL) were put in a reaction vessel, and the resulting mixture was cooled down to 0°C. Thereto, a dichloromethane (50.0 mL) solution of chloromethyl methyl ether (MOMOCl) (11.6 mL) was slowly added dropwise, and the resulting mixture was stirred at 0°C for 3 hours. The resulting reaction mixture was poured into water, and an aqueous layer was subjected to extraction with dichloromethane. Combined organic layers were washed with water, and dried over anhydrous magnesium sulfate. The solution was concentrated under reduced pressure, and the residue was purified by silica gel chromatography (heptane : ethyl acetate = 9 : 1 in a volume ratio of a solvent) to obtain compound (T-50) (28.7 g; 95%).

### Second step

Compound (T-50) (19.4 g), 2-propyn-1-ol (6.84 mL), palladium acetate (0.521 g), copper iodide (0.147 g), tri-t-butylphosphonium tetrafluoroborate (1.35 g) and diisopropylamine (230 ml) were put in a reaction vessel, and the resulting mixture was stirred at 60°C for 4 hours. The resulting reaction mixture was poured into water, and an aqueous layer was subjected to extraction with ethyl acetate. Combined organic layers were washed with water, and dried over anhydrous magnesium sulfate. The solution was concentrated under reduced pressure, and the residue was purified by silica gel chromatography (heptane : ethyl acetate = 1 : 2 in a volume ratio of a solvent) to obtain compound (T-51) (16.7 g; 95%).

### Third step

Compound (T-51) (20.2 g), compound (T-52) (31.8 g), dichlorobis[di-t-butyl(p-dimethylaminophenyl)phosphino]palladium(I I)(Pd-132) (0.631 g), potassium carbonate (24.6 g), TBAB (8.62 g), toluene (100 mL), n-butanol (100 mL) and water (100 mL) were put in a reaction vessel, and the resulting mixture was refluxed under heating at 100°C for 8 hours. The resulting reaction mixture was poured into water, and an aqueous layer was subjected to extraction with toluene. Combined organic layers were washed with water and brine in the order, and dried over anhydrous magnesium sulfate. The solution was concentrated under reduced pressure, and the residue was purified by silica gel chromatography (heptane : ethyl acetate = 1 : 1 in a volume ratio of a solvent) to obtain compound (T-53) (8.28 g; 22%).

### Fourth step

Compound (T-53) (8.28 g), a palladium on carbon catalyst (NX type of 5% Pd/C (50% wet); 0.0397 g; made by N.E. Chemcat Corporation) and THF (150 mL) were put in a reaction vessel, and the resulting mixture was stirred at room temperature for 24 hours under a hydrogen atmosphere. A catalyst was removed therefrom by filtration, and then the resulting solution was concentrated under reduced pressure, and the residue was purified by silica gel chromatography (heptane : ethyl acetate = 1 : 1 in a volume ratio of a solvent) to obtain compound (T-54) (8.57 g; 98%) .

### Fifth step

Compound (T-54) (7.00 g), methacrylic acid (T-55) (2.09 g), DMAP (1.23 g) and dichloromethane (140 mL) were put in a reaction vessel, and the resulting mixture was cooled down to 0°C. Thereto, a dichloromethane (30.0 mL) solution of DCC (6.25 g) was slowly added dropwise, and the resulting mixture was stirred for 12 hours while returning to room temperature. An insoluble matter was filtered off, and then the resulting reaction mixture was poured into water, and an aqueous layer was subjected to extraction with dichloromethane. Combined organic layers were washed with water, and dried over anhydrous magnesium sulfate. The solution was concentrated under reduced pressure, and the residue was purified by silica gel chromatography (heptane : ethyl acetate = 15 : 1 in a volume ratio of a solvent) to obtain compound (T-56) (7.45 g; 75%).

### Sixth step

Compound (T-57) (6.34 g; 96%) was obtained by using compound (T-56) (7.45 g) as a raw material in the same technique as in the fourth step in Synthesis Example 6.

### Seventh step

Compound (T-58) (7.12 g; 82%) was obtained by using compound (T-57) (6.34 g) as a raw material in the same technique as in the eighth step in Synthesis Example 1.

### Eighth step

Compound (1-3-25) (5.05 g; 82%) was obtained by using compound (T-58) (7.12 g) as a raw material in the same technique as in the ninth step in Synthesis Example 1.

An NMR analysis value of the resulting compound (1-3-25) was as described below.
¹H-NMR: chemical shift δ (ppm; CDCl₃) : 7.49 - 7.41 (m, 4H), 7.30 - 7.27 (m, 2H), 7.13 - 7.12 (m, 1H), 6.54 (s, 1H), 6.07 (s, 2H), 5.55 - 5.54 (m, 1H), 4.47 (d, J = 6.8 Hz, 2H), 4.18 (t, J = 6.5 Hz, 2H), 2.70 - 2.67 (m, 2H), 2.50 (tt, J = 9.1 Hz, J = 5.8 Hz, 1H), 2.30 (t, J = 6.6 Hz, 1H), 2.03 - 1.88 (m, 9H), 1.52 - 1.45 (m, 2H), 1.36 - 1.21 (m, 9H), 1.10 - 1.03 (m, 2H), 0.90 (t, J = 7.2 Hz, 3H) .

### Synthesis Example 8

### Synthesis of compound (1-3-60)

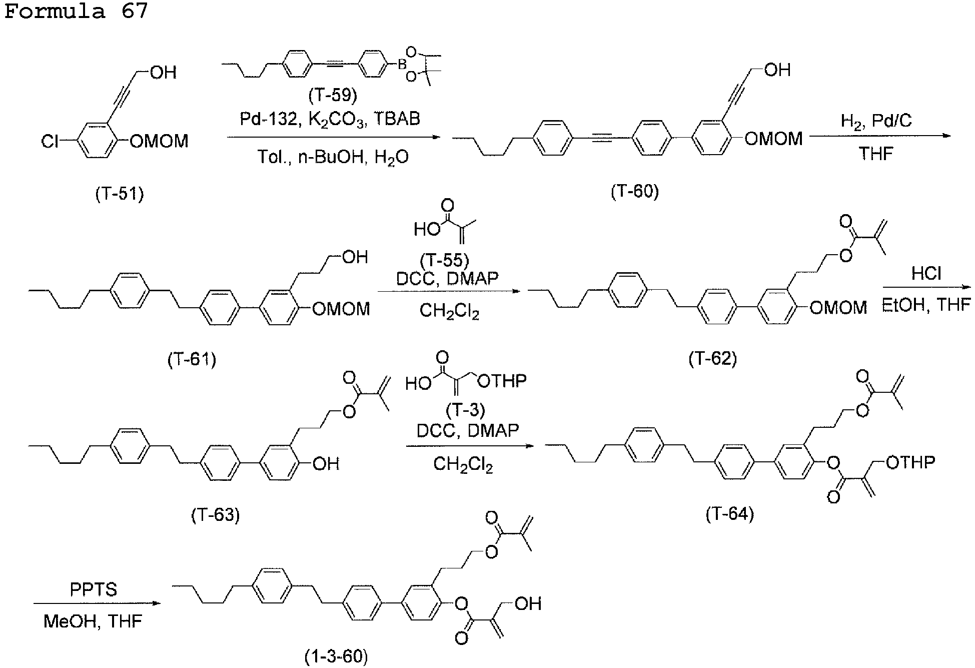

### First step

Compound (T-60) (20.4 g; 97%) was obtained by using compound (T-51) (10.9 g) and compound (T-59) (19.8 g) as raw materials in the same technique as in the third step in Synthesis Example 7.

### Second step

Compound (T-61) (20.3 g; 98%) was obtained by using compound (T-60) (20.4 g) as a raw material in the same technique as in the fourth step in Synthesis Example 7.

### Third step

Compound (T-62) (17.8 g; 66%) was obtained by using compound (T-61) (23.6 g) as a raw material in the same technique as in the fifth step in Synthesis Example 7.

### Fourth step

Compound (T-63) (12.0 g; 78%) was obtained by using compound (T-62) (16.8 g) as a raw material in the same technique as in the sixth step in Synthesis Example 7.

### Fifth step

Compound (T-64) (13.0 g; 76%) was obtained by using compound (T-63) (12.7 g) as a raw material in the same technique as in the eighth step in Synthesis Example 1.

### Sixth step

Compound (1-3-60) (8.74 g; 77%) was obtained by using compound (T-64) (13.0 g) as a raw material in the same technique as in the ninth step in Synthesis Example 1.

An NMR analysis value of the resulting compound (1-3-60) was as described below.
¹H-NMR: chemical shift δ (ppm; CDCl₃) : 7.49 - 7.44 (m, 4H), 7.26 (d, J = 7.8 Hz, 2H), 7.15 - 7.10 (m, 5H), 6.54 (s, 1H), 6.07 (s, 2H), 5.55 - 5.54 (m, 1H), 4.47 (d, J = 6.6 Hz, 2H), 4.19 (t, J = 6.4 Hz, 2H), 2.96 - 2.90 (m, 4H), 2.69 (t, J = 7.5 Hz, 2H), 2.58 (t, J = 7.7 Hz, 2H), 2.34 (t, J = 6.4 Hz, 1H), 2.04 - 1.98 (m, 2H), 1.92 (s, 3H), 1.64 - 1.56 (m, 3H), 1.36 - 1.29 (m, 4H), 0.89 (t, J = 6.7 Hz, 3H).

### Comparative Example 1

Compound (S-1) was prepared as a comparative compound, and characteristics were measured. The reason for selecting the compound is that the compound is described in WO 2017/047177 A, and similar to the compound of the invention.

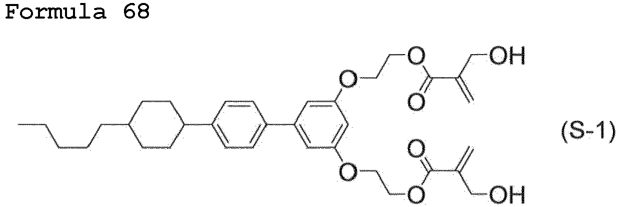

An NMR analysis value of comparative compound (S-1) was as described below.
¹H-NMR: chemical shift δ (ppm; CDCl₃) : 7.48 - 7.46 (m, 2H), 7.27 - 7.26 (m, 2H), 6.75 (d, J = 2.3 Hz, 2H), 6.47 - 6.46 (m, 1H), 6.30 (s, 2H), 5.86 (d, J = 1.1 Hz, 2H), 4.54 (t, 4.4 Hz, 4H), 4.33 (s, 4H), 4.27 - 4.25 (m, 4H), 2.52 - 2.47 (m, 1H), 2.34 (s, 2H), 1.98 - 1.83 (m, 4H), 1.51 - 1.44 (m, 2H), 1.35 - 1.20 (m, 9H), 1.09 - 1.02 (m, 2H), 0.90 (t, J = 6.9 Hz, 3H).

Physical properties of comparative compound (S-1) were as described below.

Transition temperature: C 58.8 I.

Comparison was made on compatibility with a liquid crystal composition between compound (No. 1-3-31) and comparative compound (S-1). Composition (i) comprising compounds (i-1) to (i-9) described below was used for evaluation.

A proportion of a component of composition (i) was expressed in terms of % by weight.

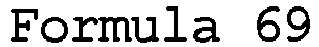

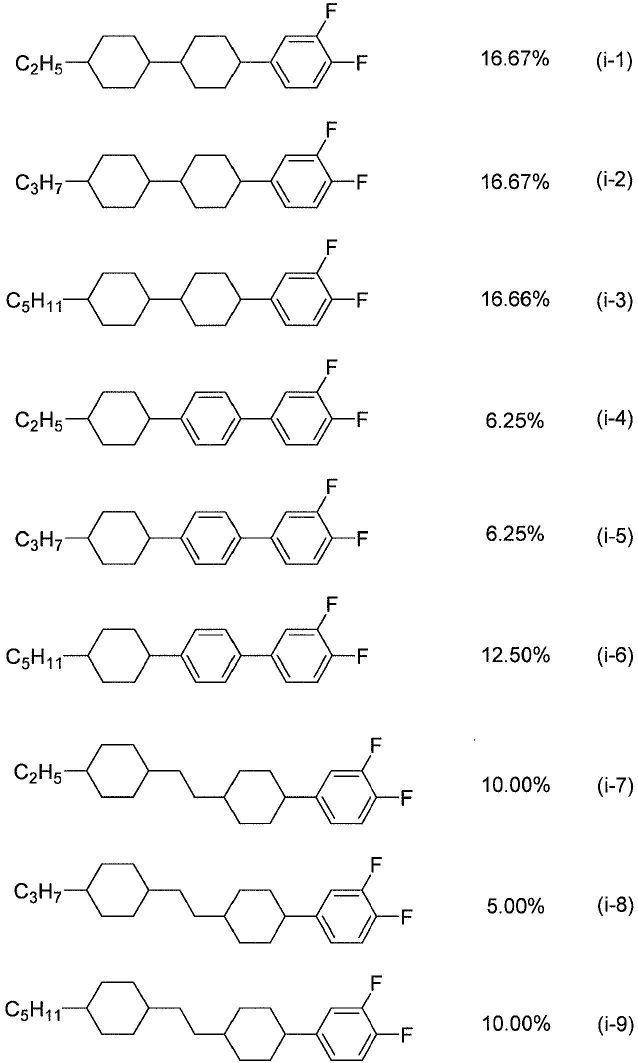

A sample in which compound (1-3-31) or comparative compound (S-1) was added to base liquid crystal (i) at a proportion of 0.1% by weight to 3.0% by weight was prepared. When the sample was left to stand at 25°C for 7 days and then visually observed, a case of maintaining the nematic phase was represented as "Good," and a case of precipitating crystals or the smectic phase was represented as "Poor."

### Table 2

**Table 2. Comparison of compatibility between compound (No. 1-3-31) and comparative compound (S-1)**

| Amount of addition (% by weight) | Compound (No. 1-3-31) | Comparative compound (S-1) |
|---|---|---|
| | 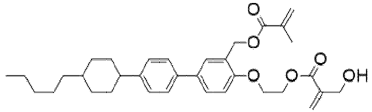 | 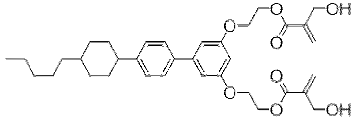 |
| 3.0 | Good | Poor |
| 1.0 | Good | Poor |
| 0.5 | Good | Poor |
| 0.2 | Good | Poor |
| 0.1 | Good | Good |

As a result of comparison of compatibility, while the nematic phase was maintained even if 3% by weight of compound (1-3-31) was added to the base liquid crystal, crystals were precipitated even if 0.2% by weight of comparative compound (S-1) was added thereto. The compounds are similar to each other in that the compounds have the same ring structure to which a plurality of polymerizable groups are bonded, but compatibility between both is significantly different. The reason can be considered that, while comparative compound (S-1) has a plurality of polar groups, compound (1-3-31) has only one polar group, and therefore affinity to the liquid crystal composition is improved. Accordingly, the compound of the present application can be considered to be an excellent compound having a plurality of polymerizable groups and maintaining practical compatibility.

Compounds described below can be prepared with reference to the methods described in Synthesis Examples and a section in "2. Synthesis of compound (1)."

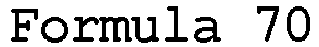

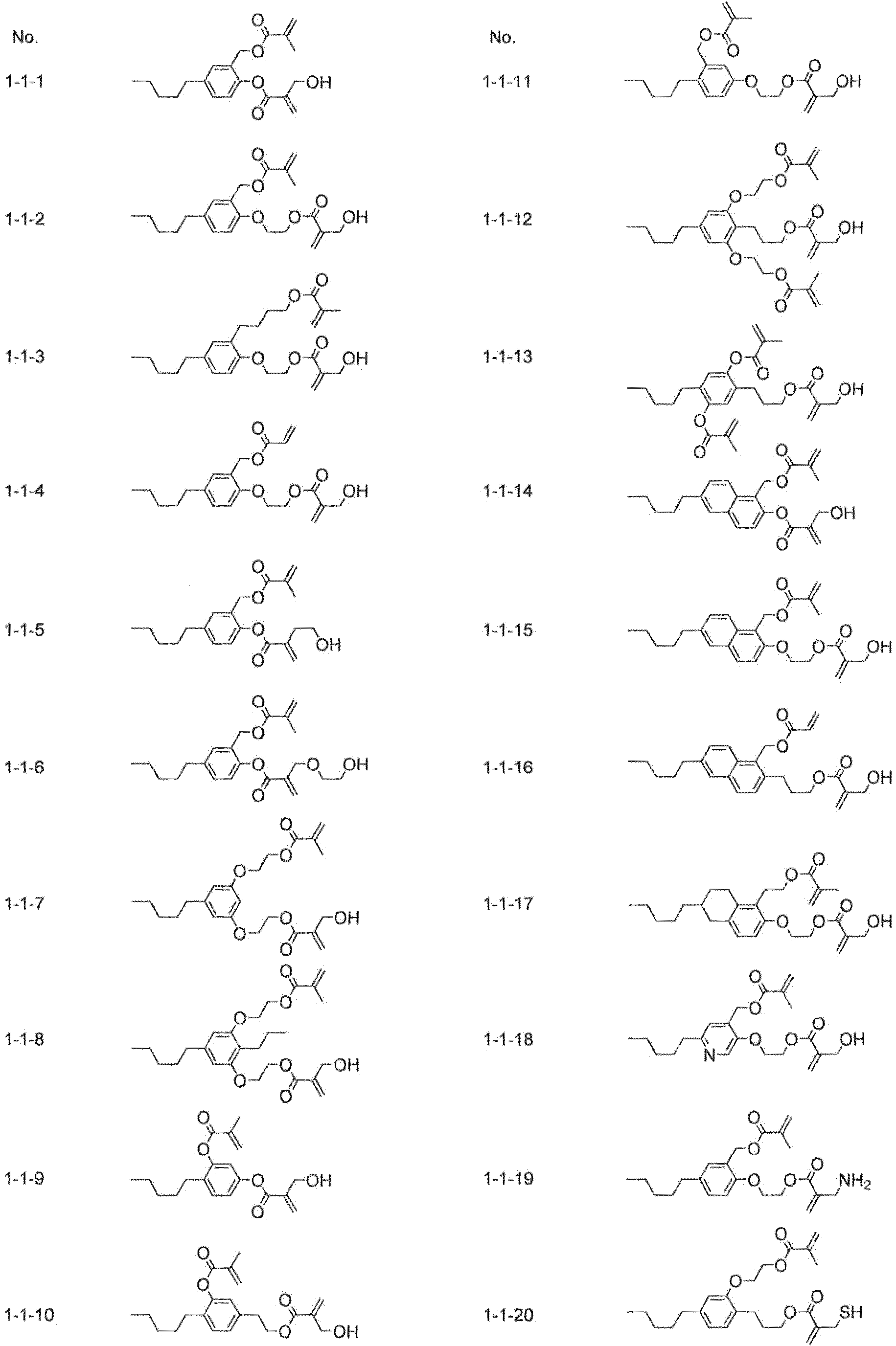

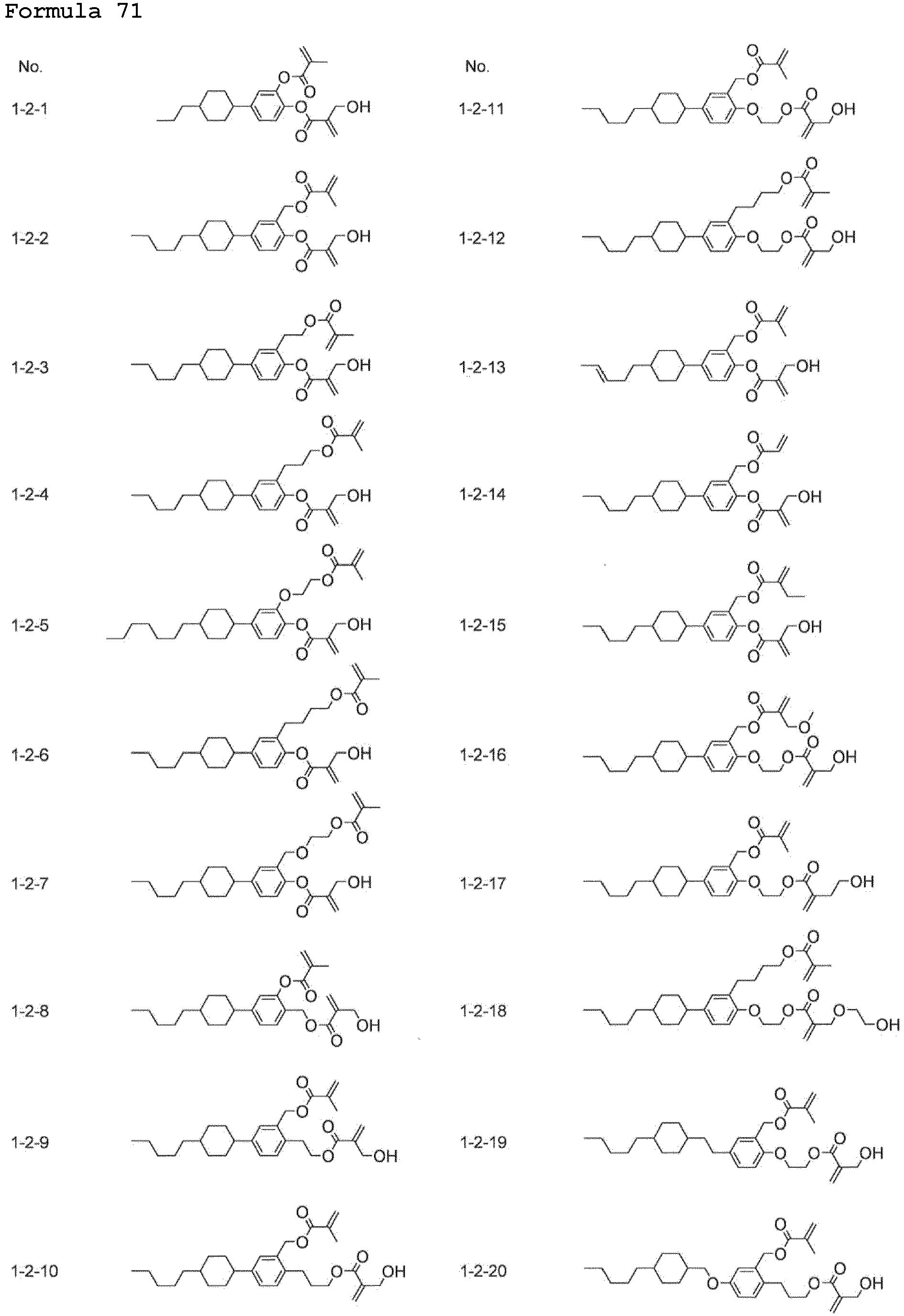

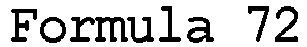

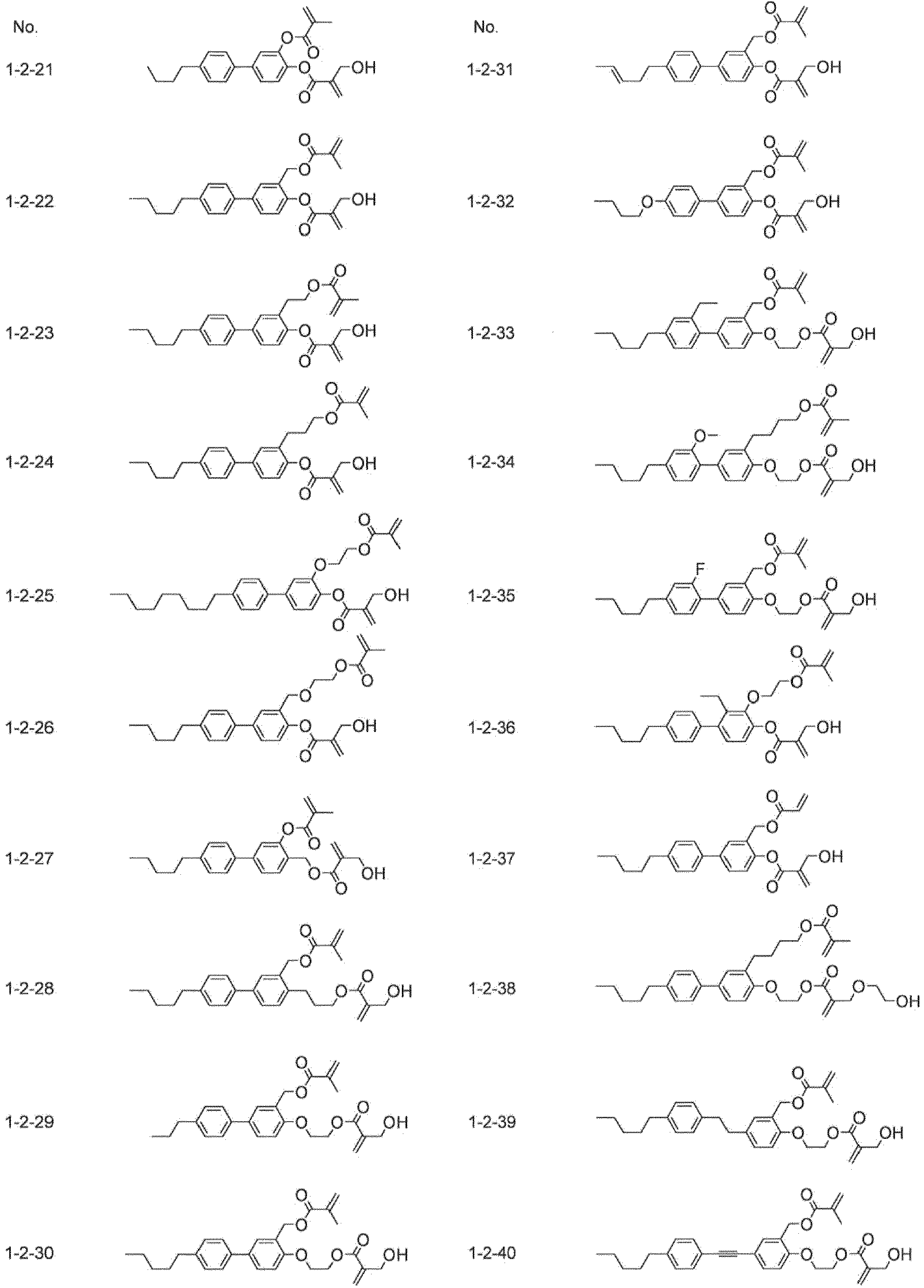

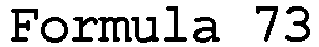

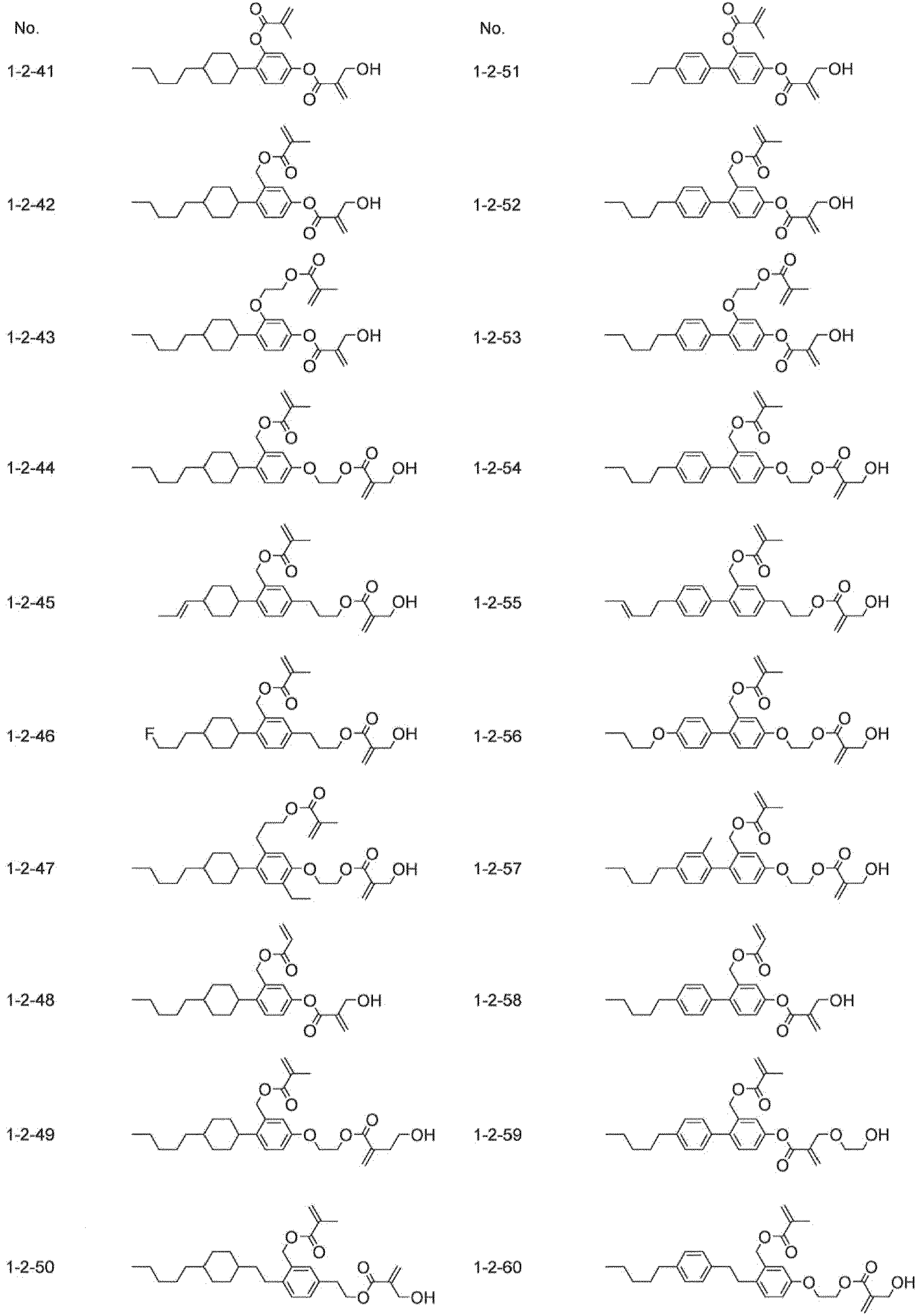

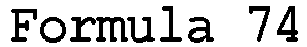

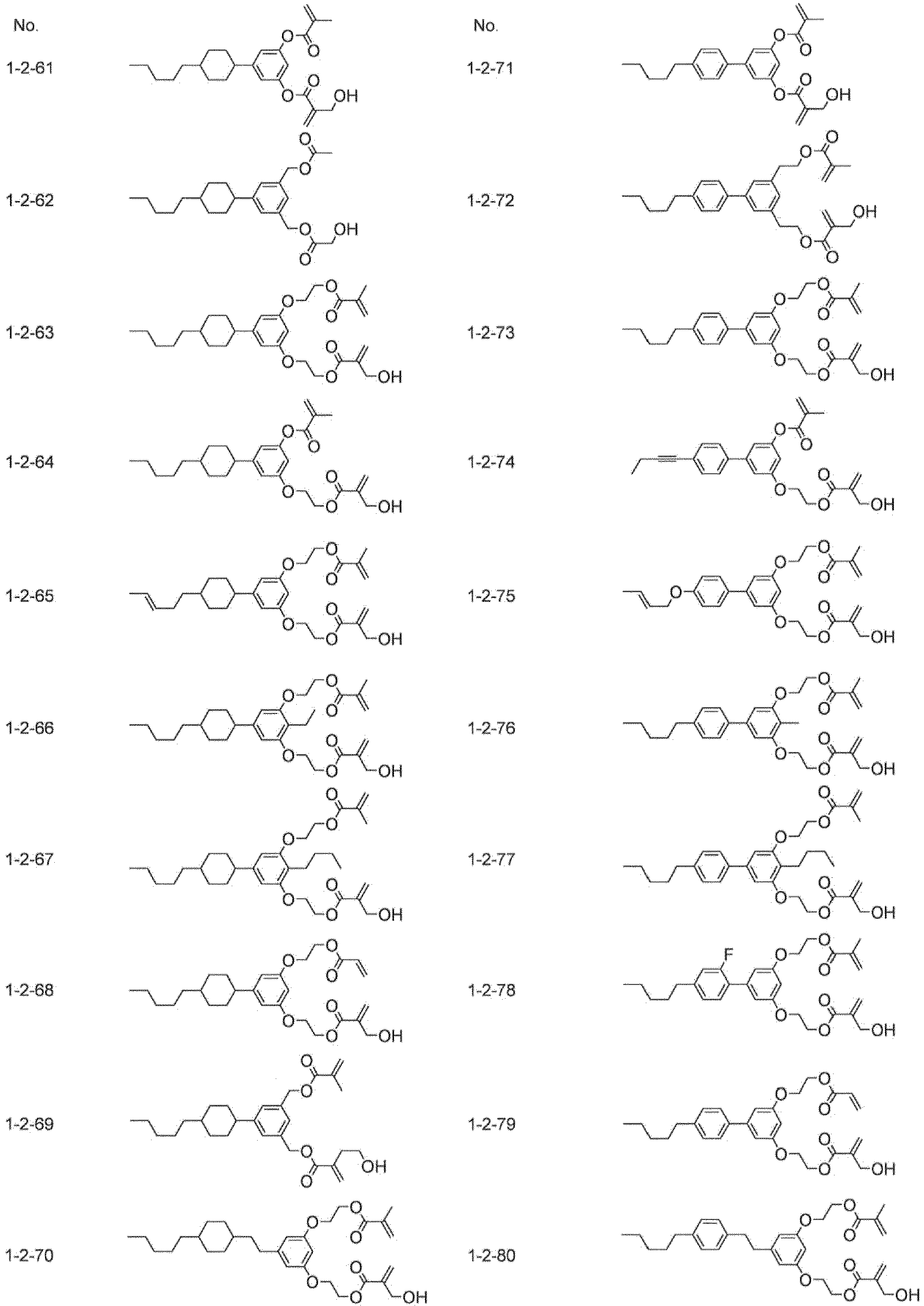

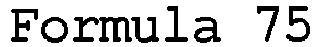

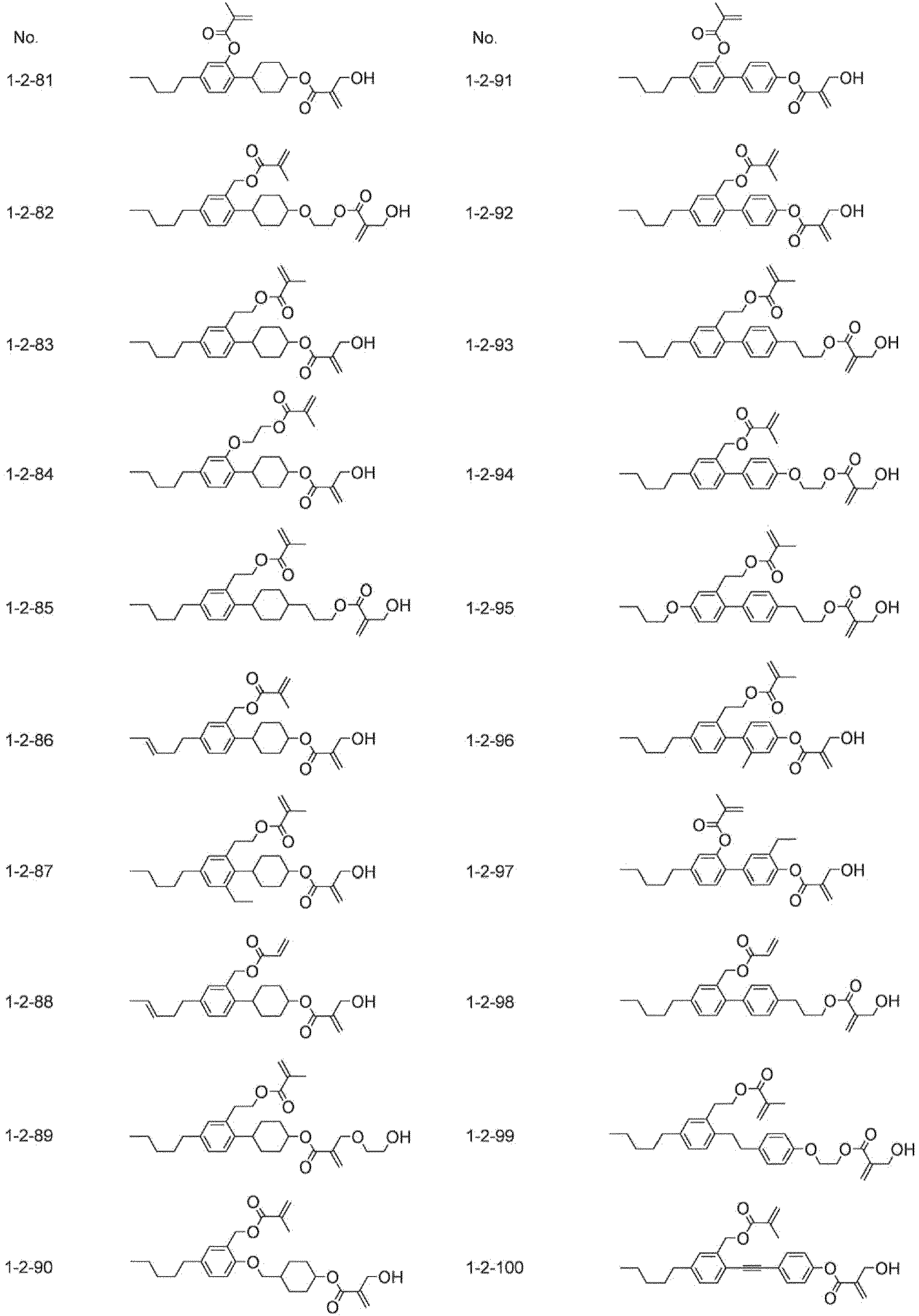

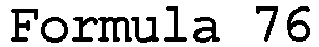

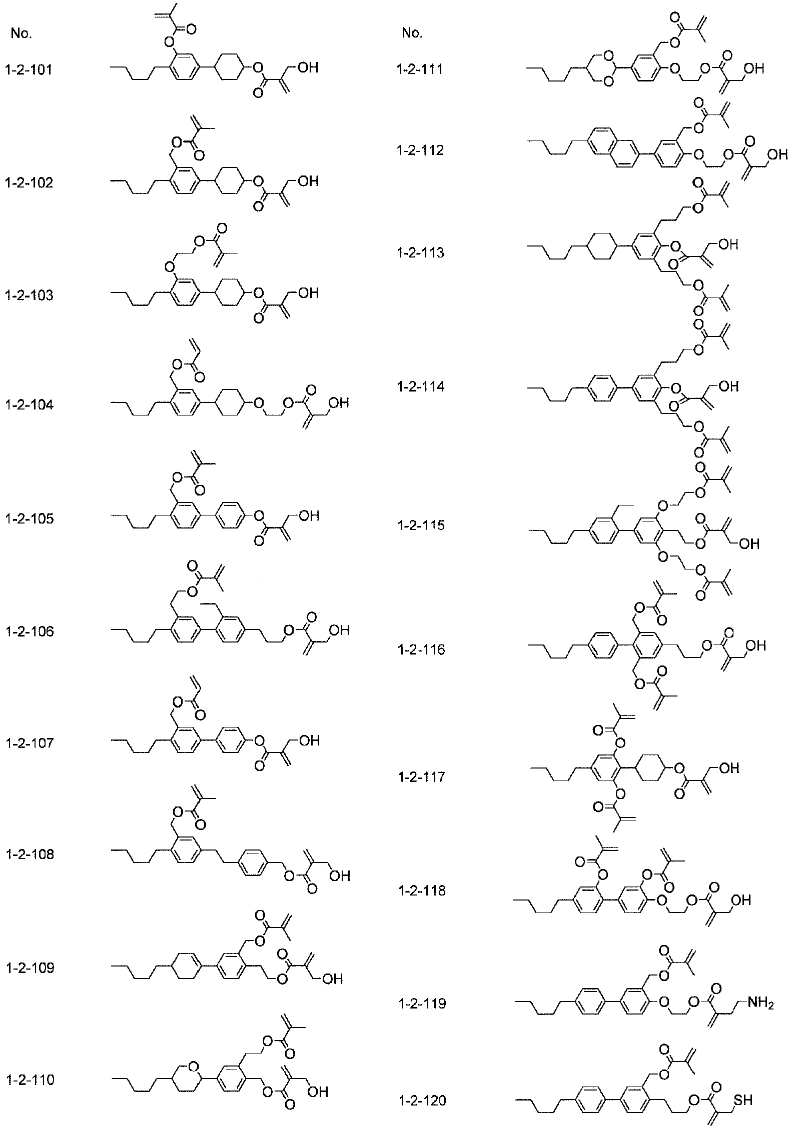

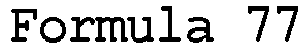

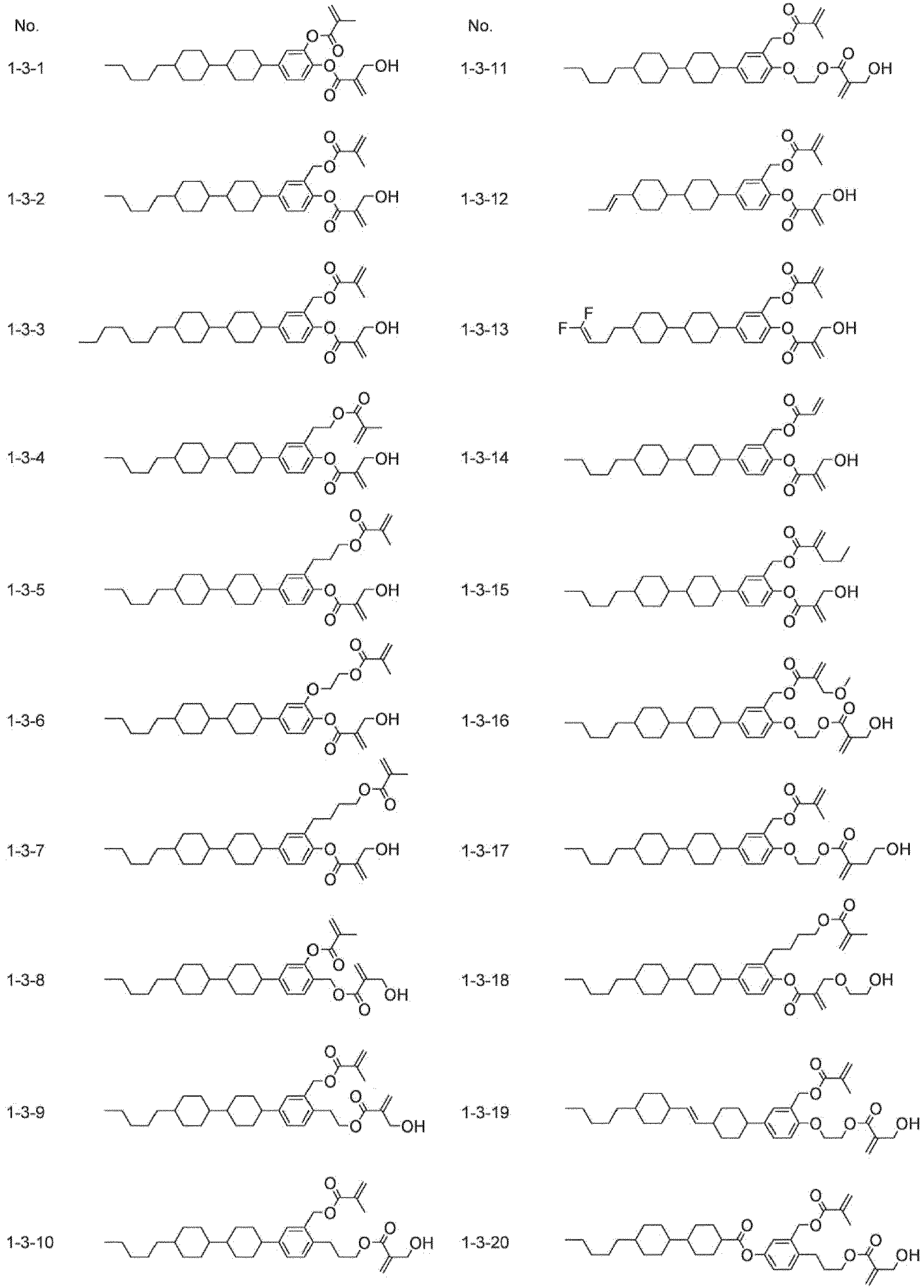

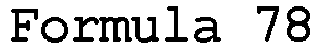

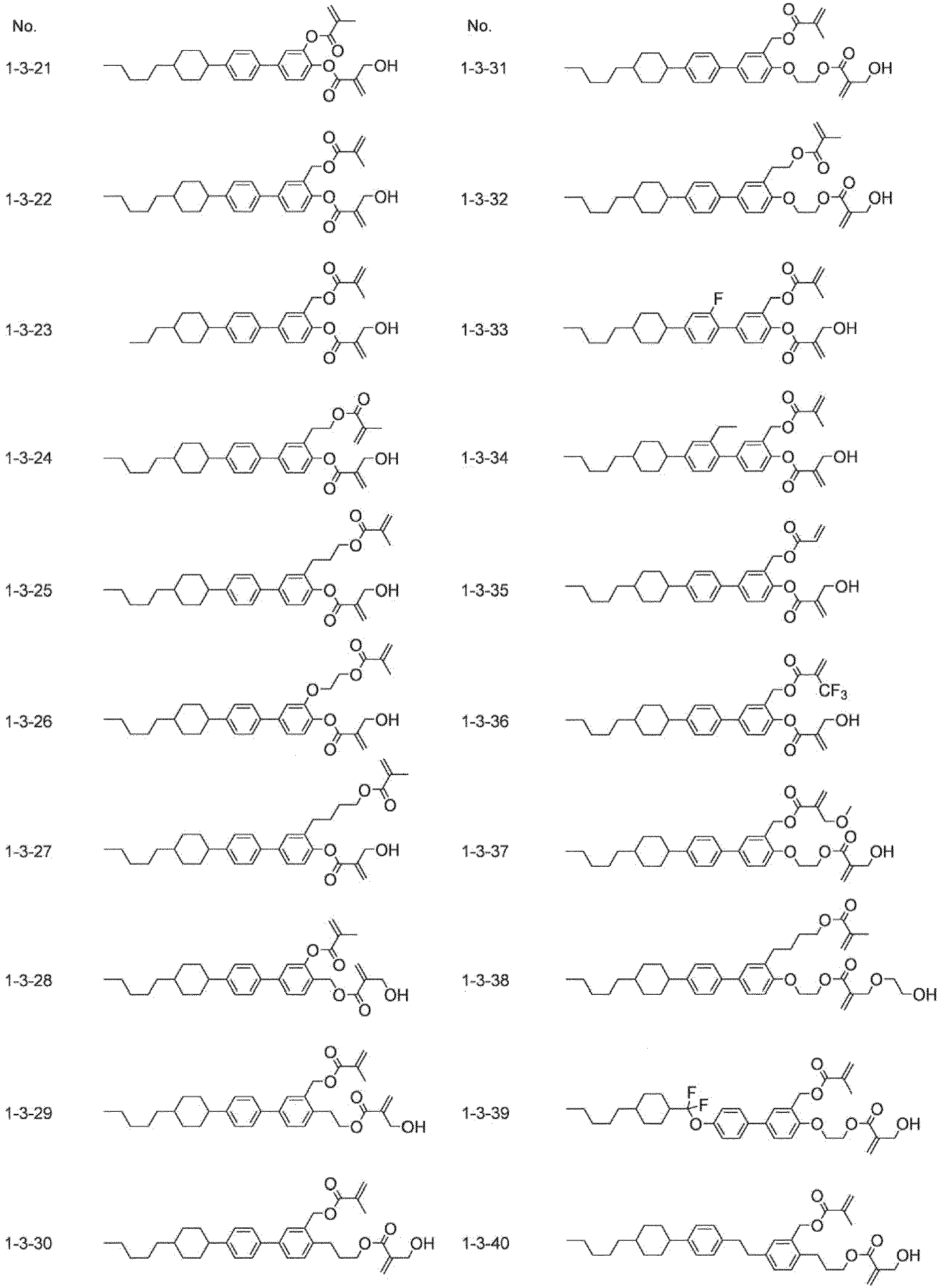

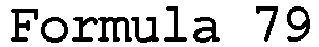

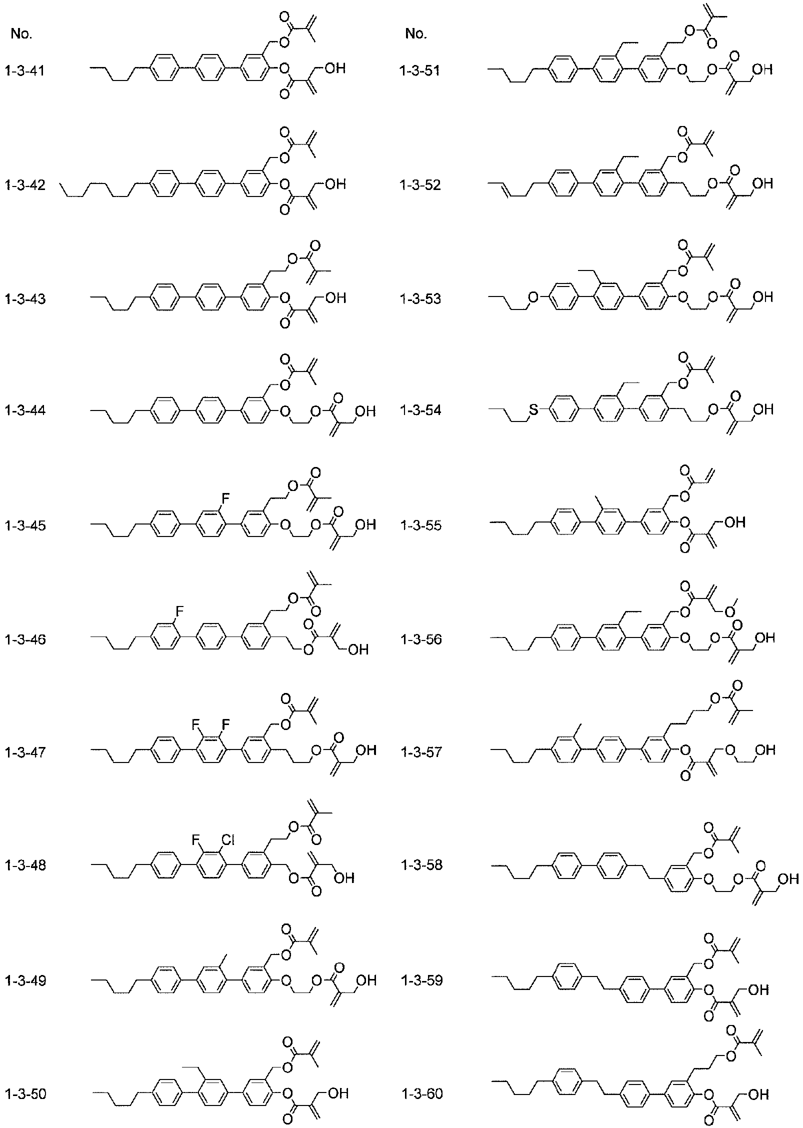

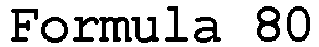

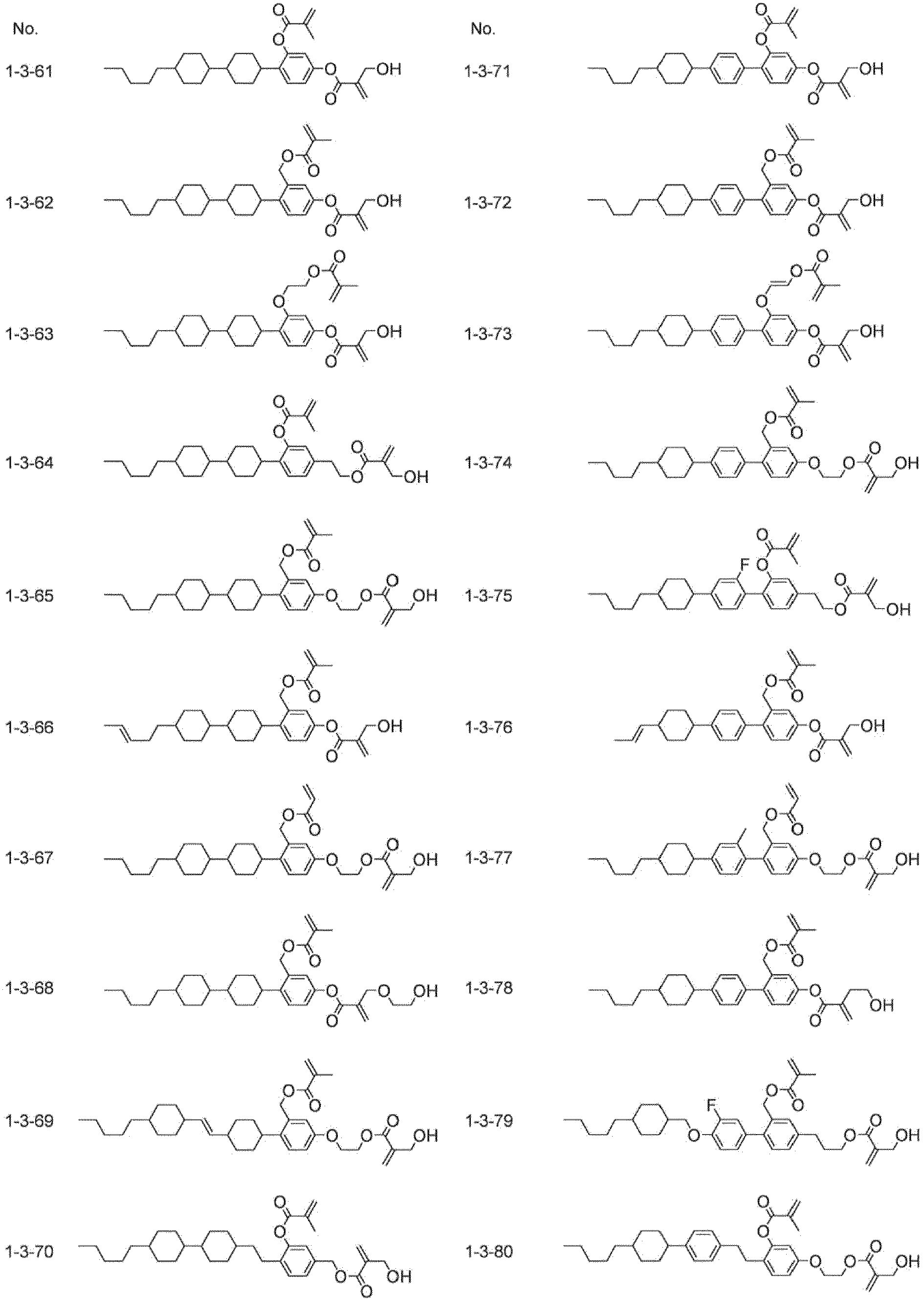

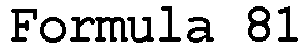

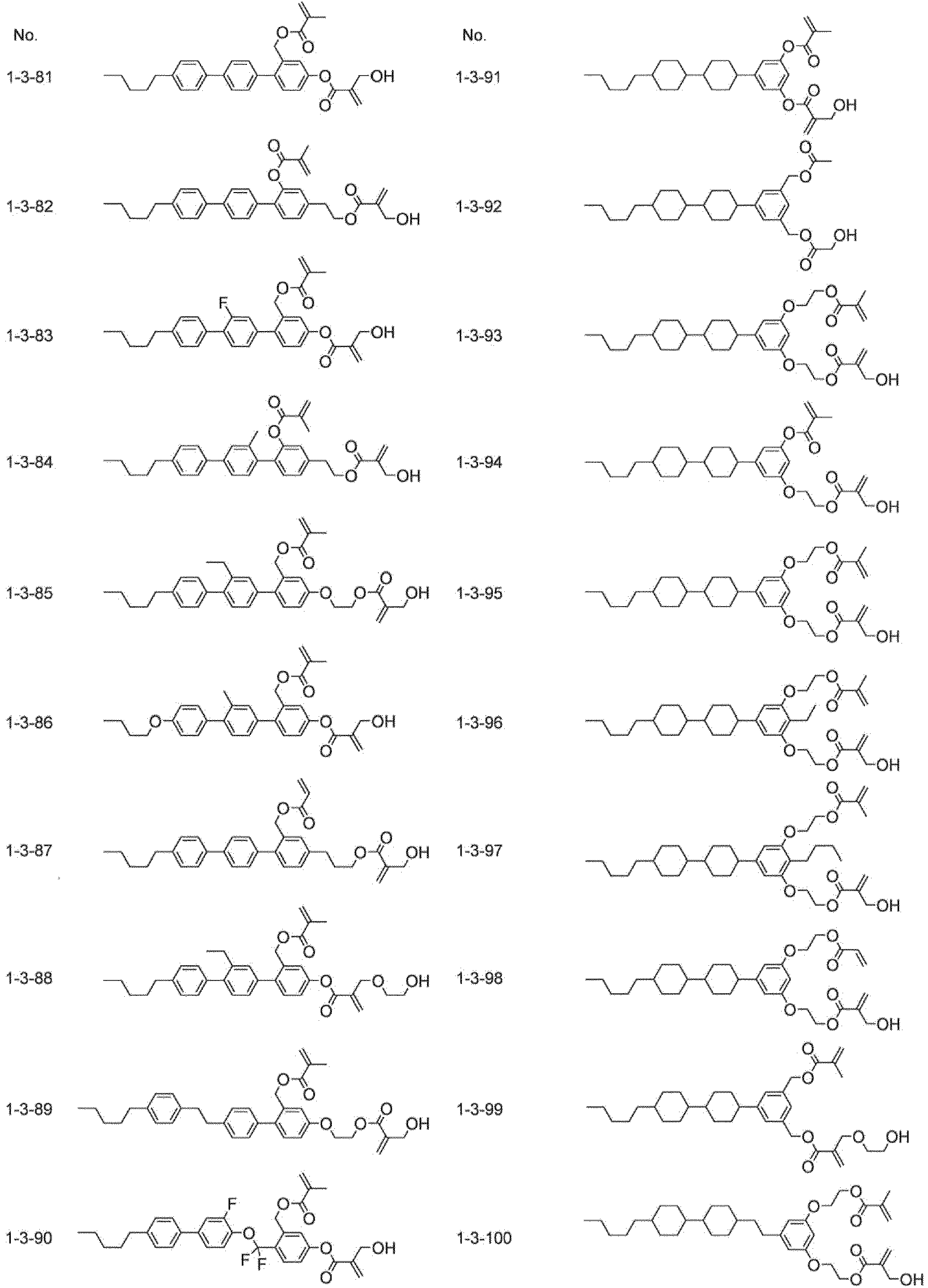

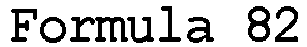

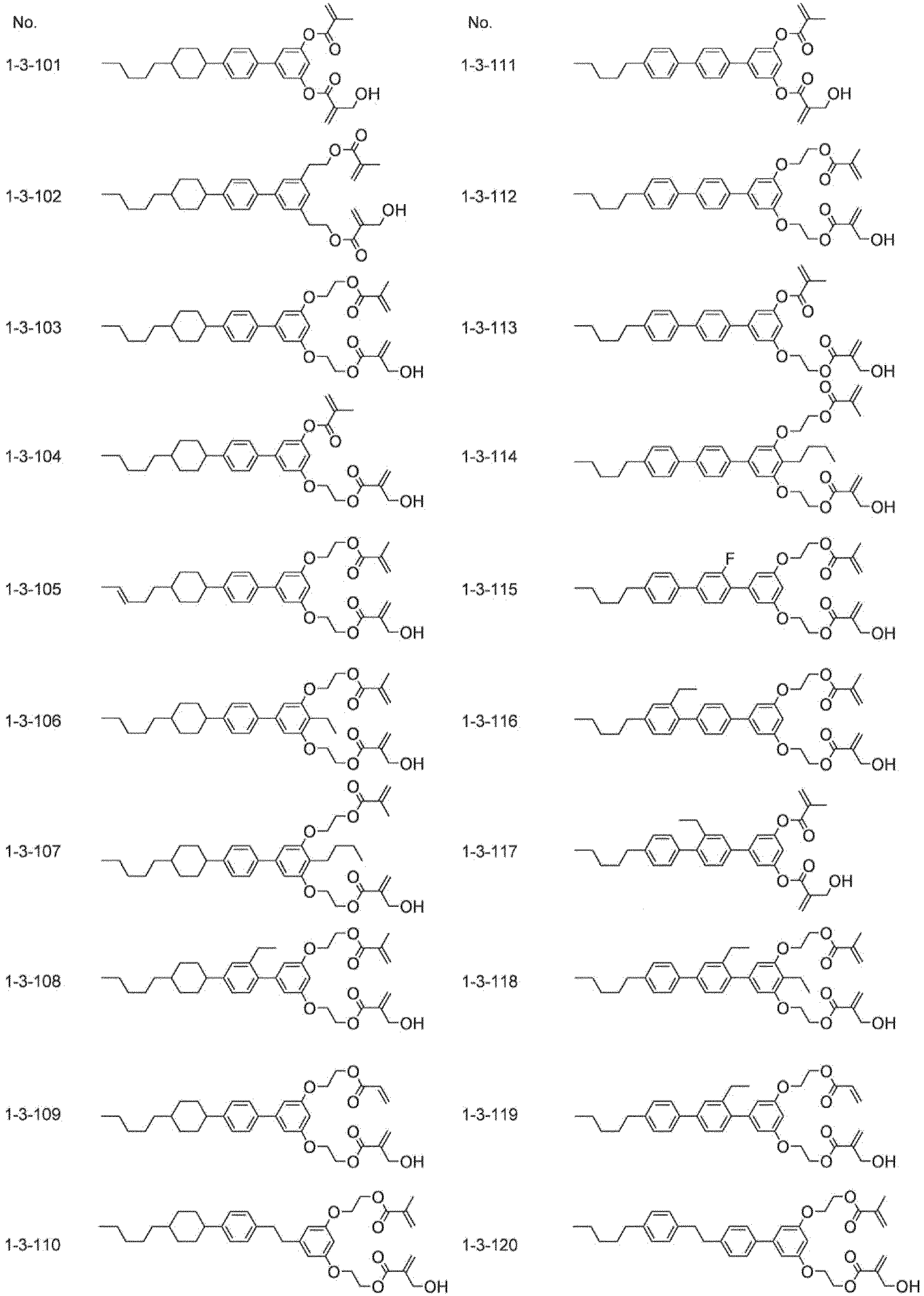

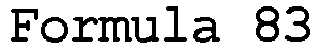

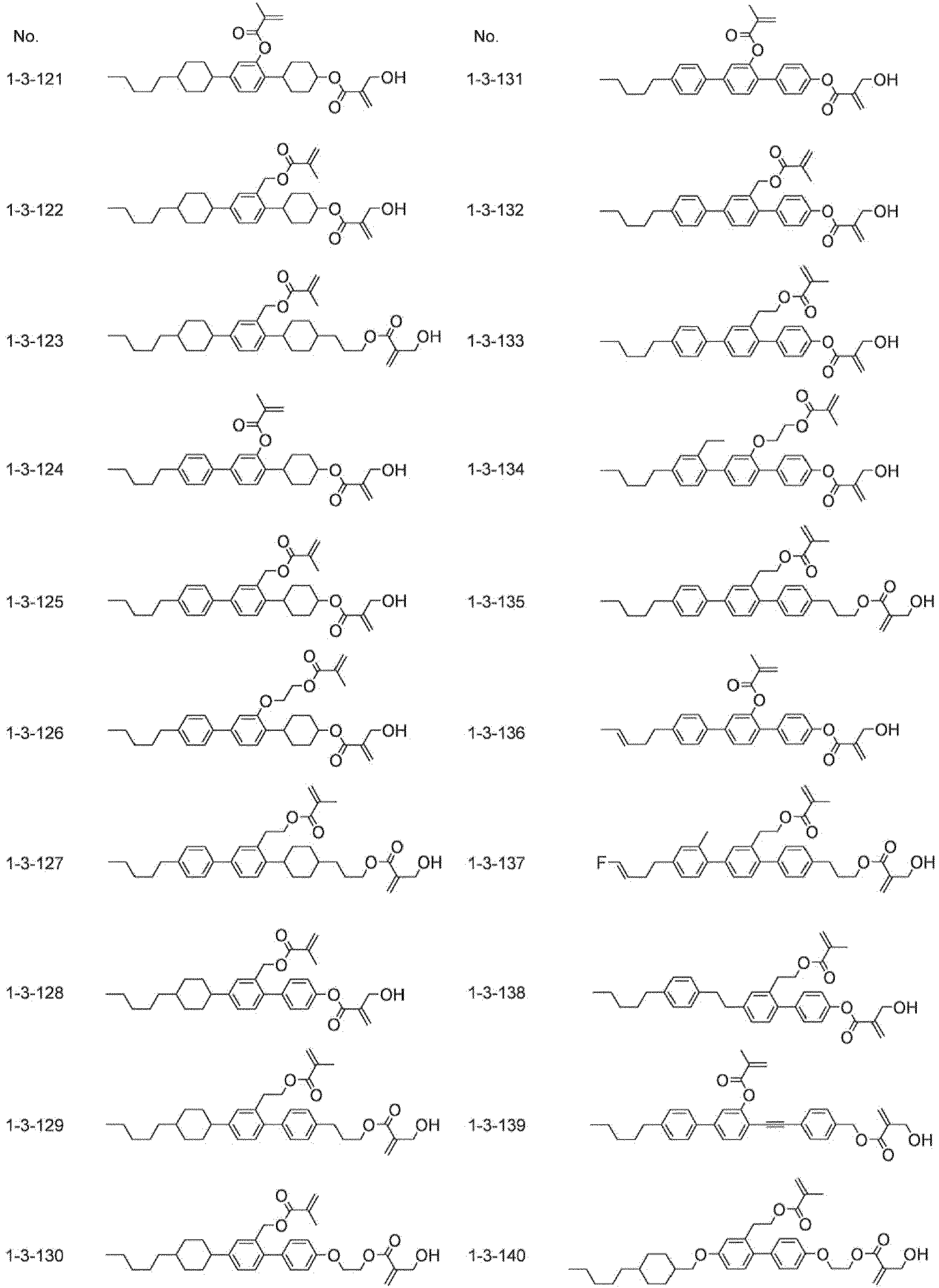

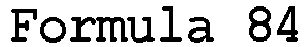

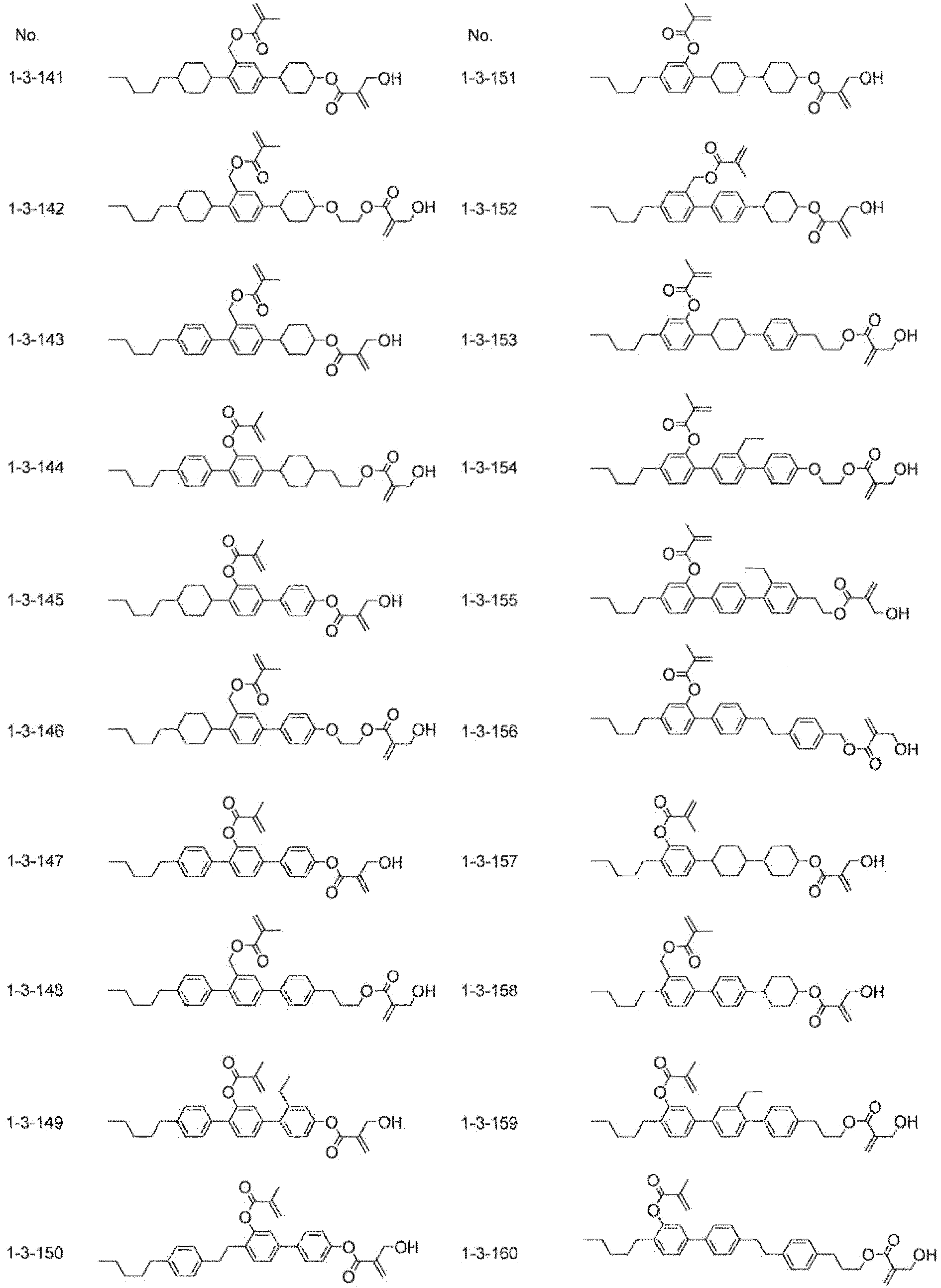

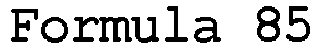

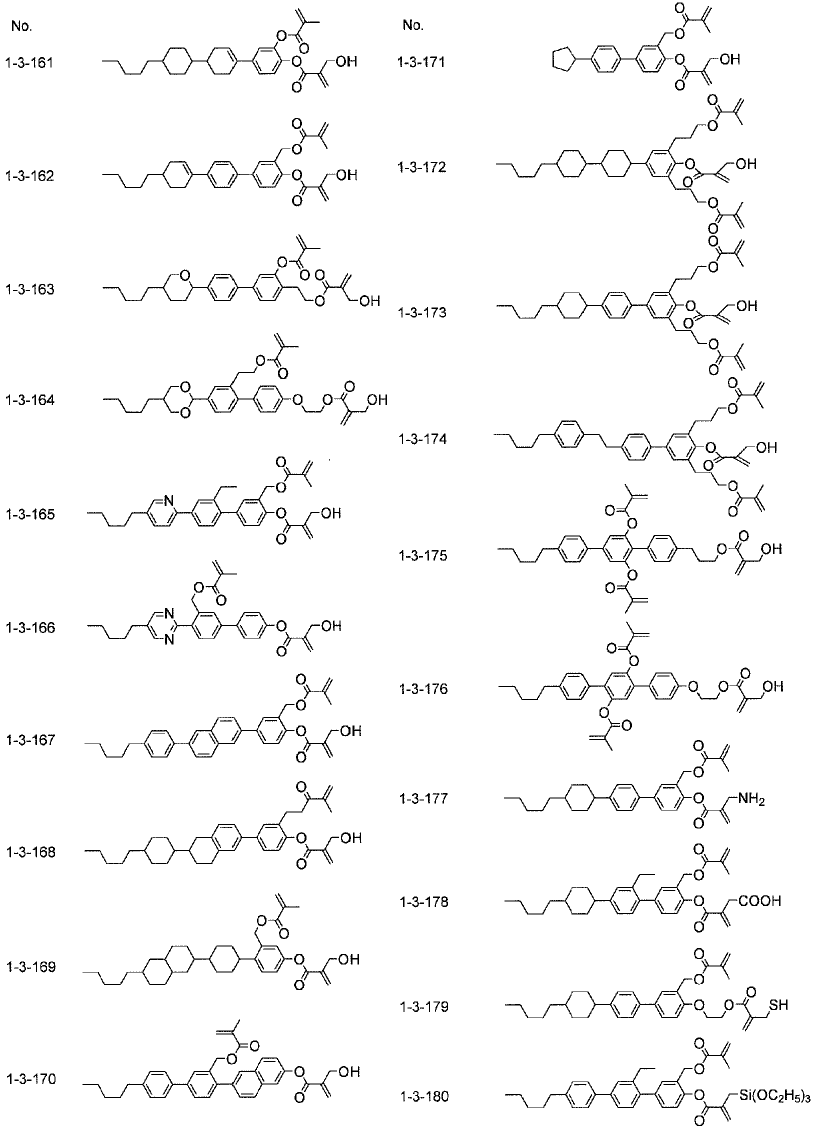

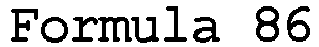

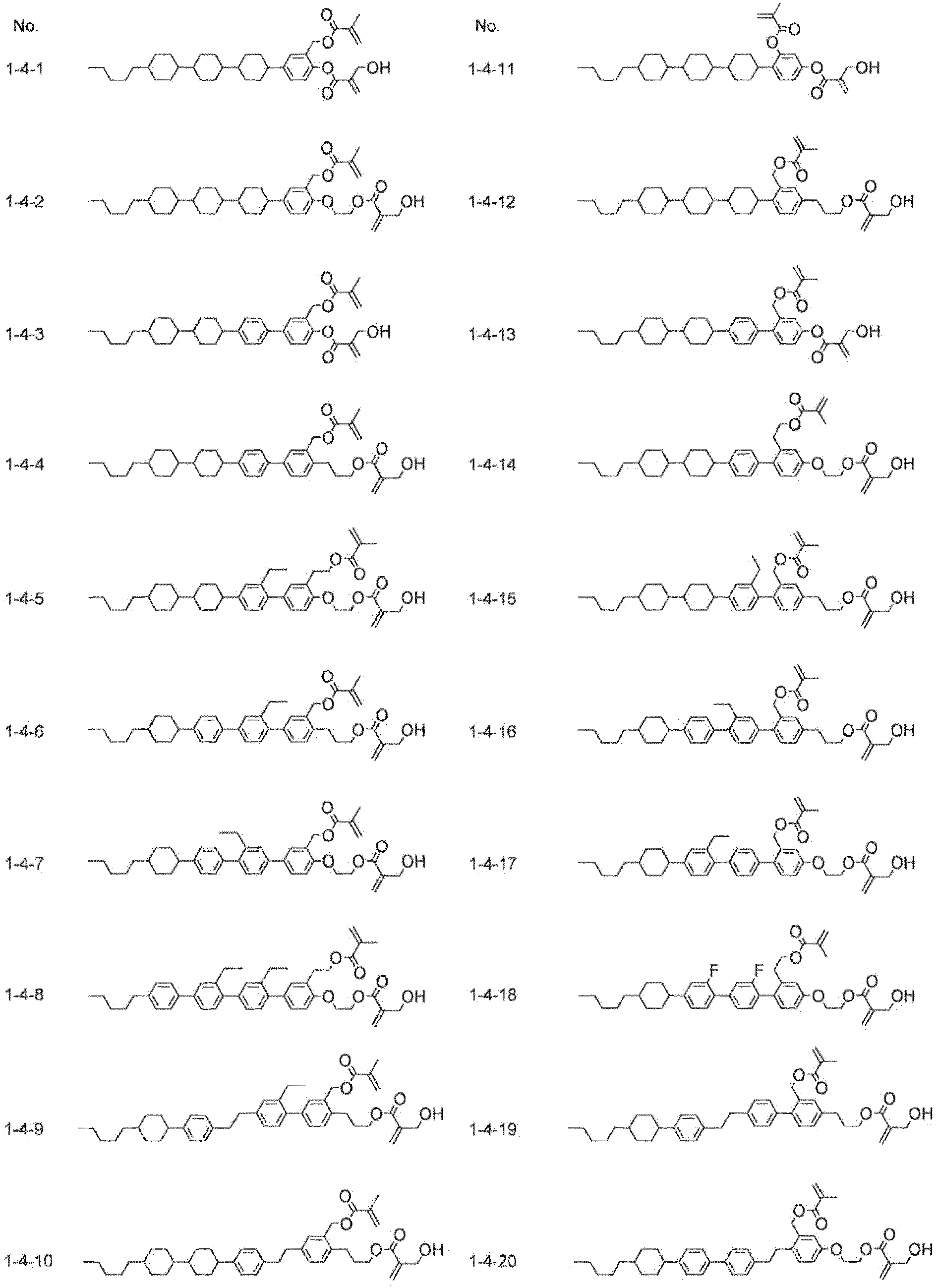

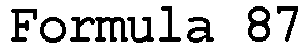

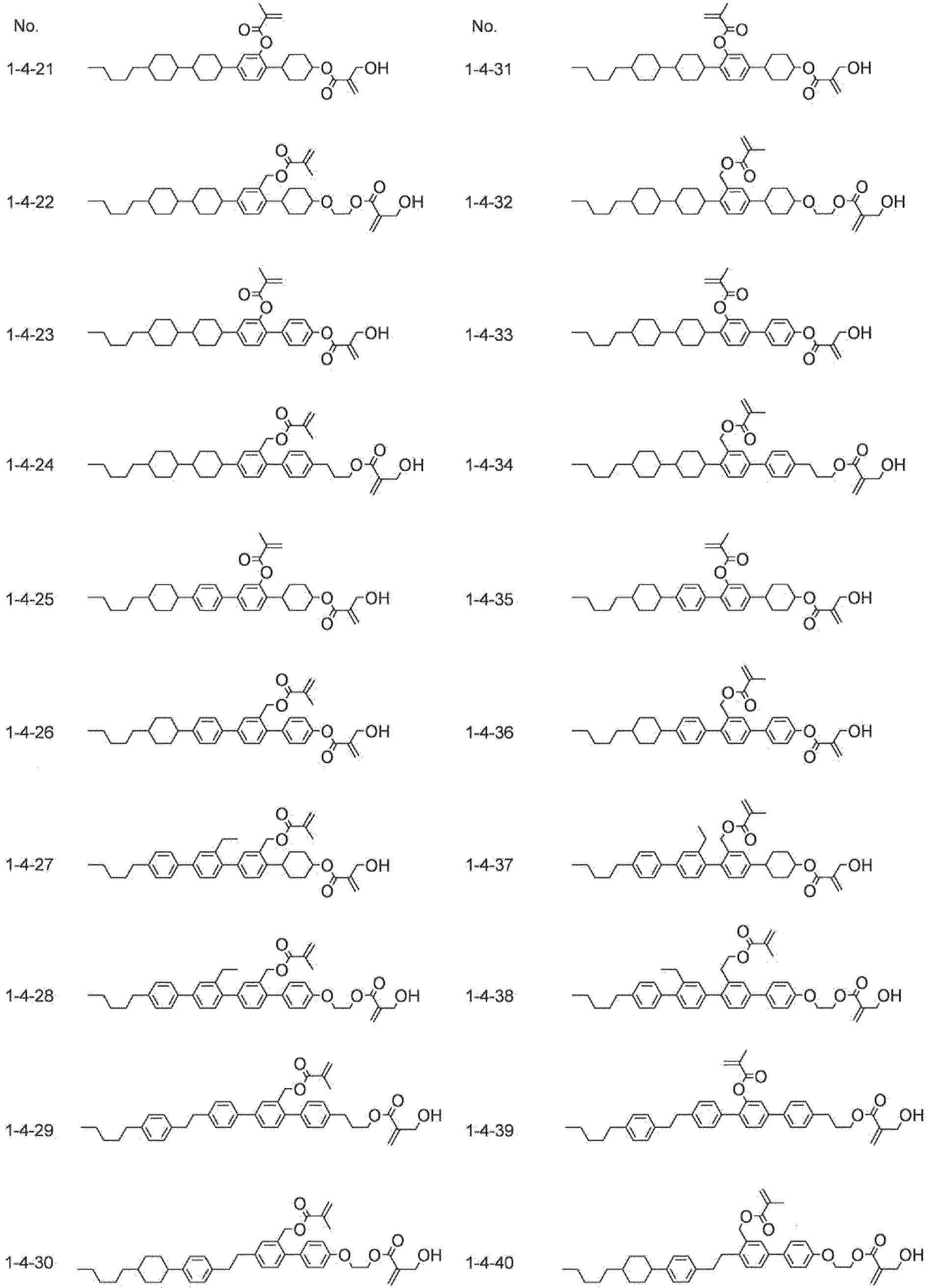

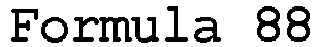

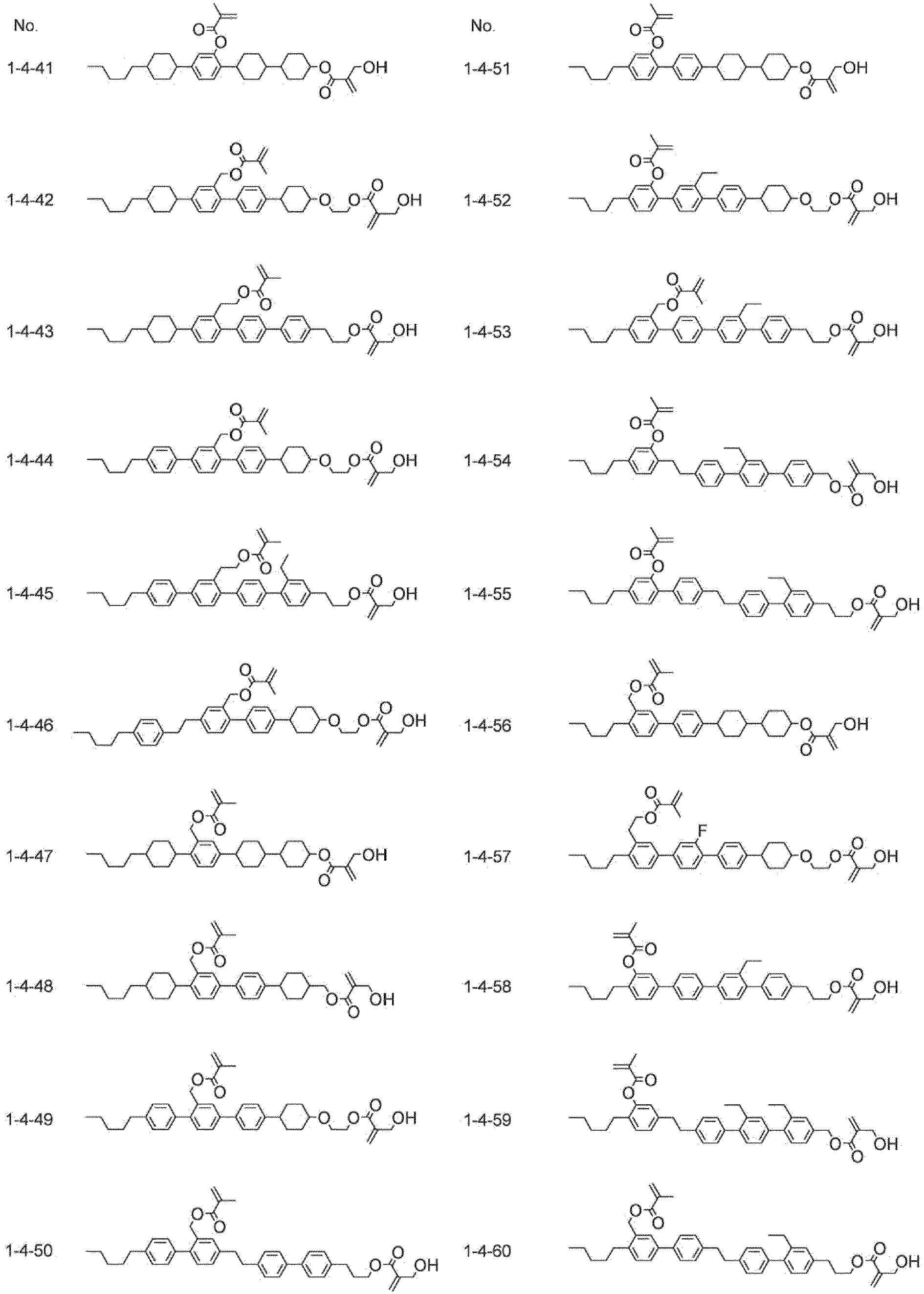

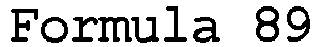

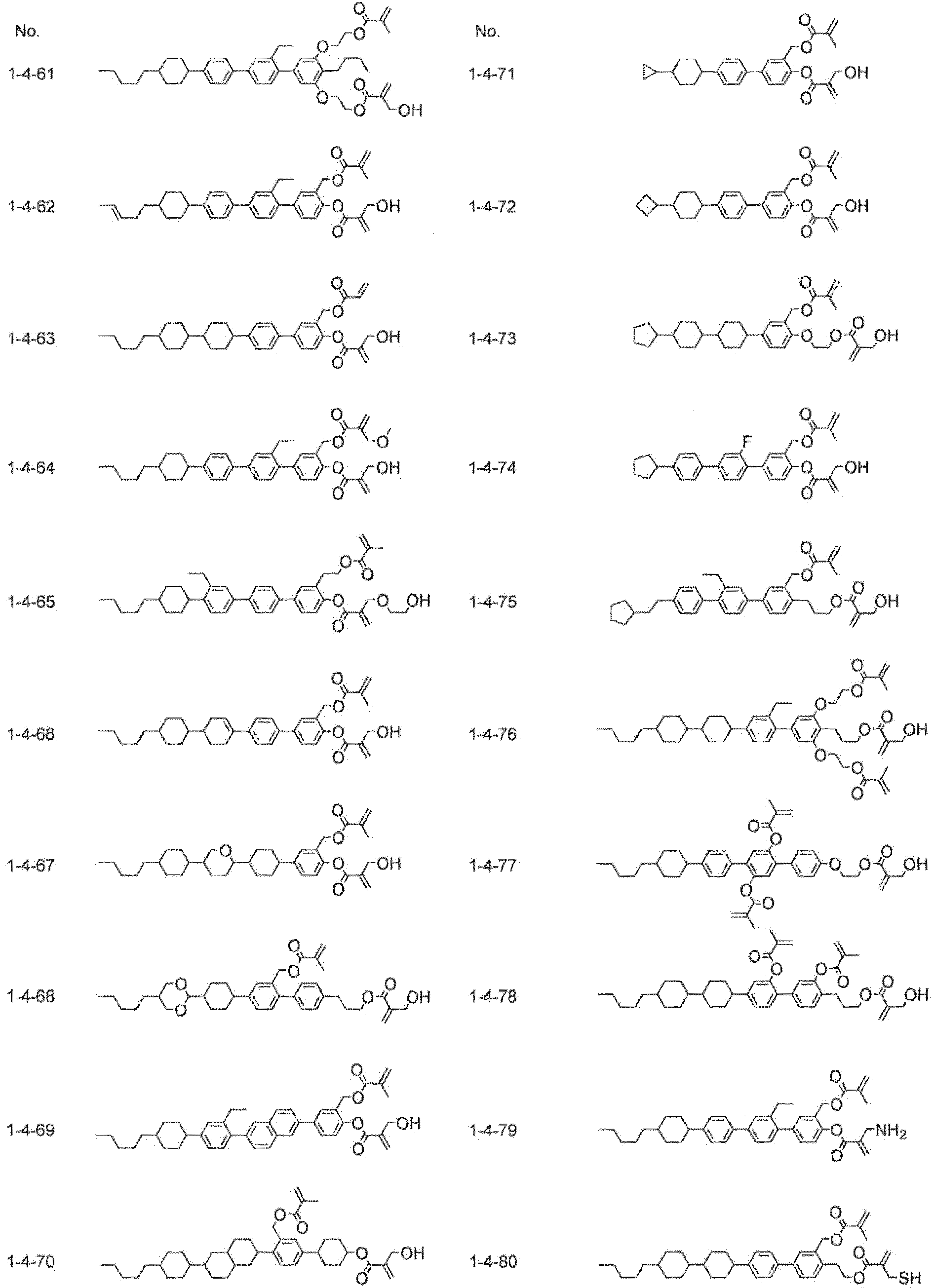

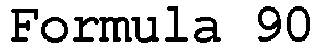

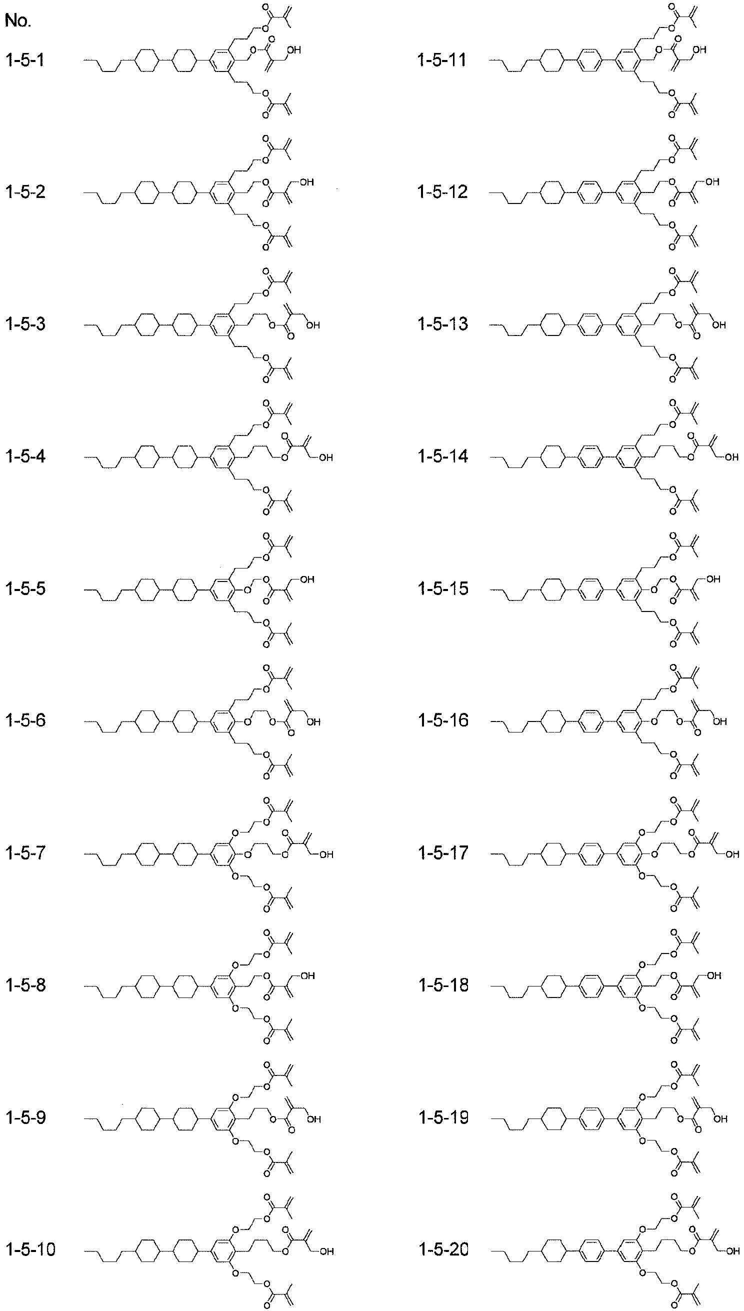

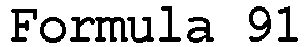

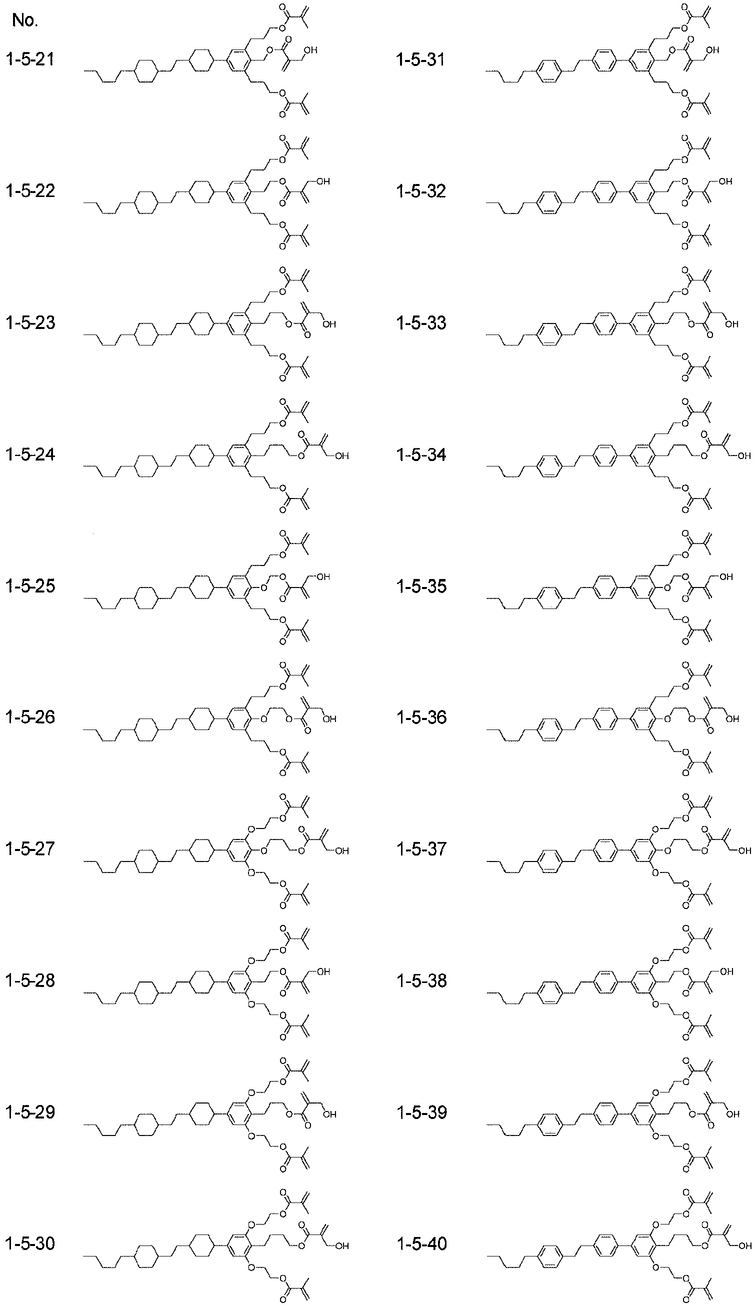

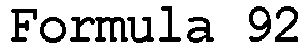

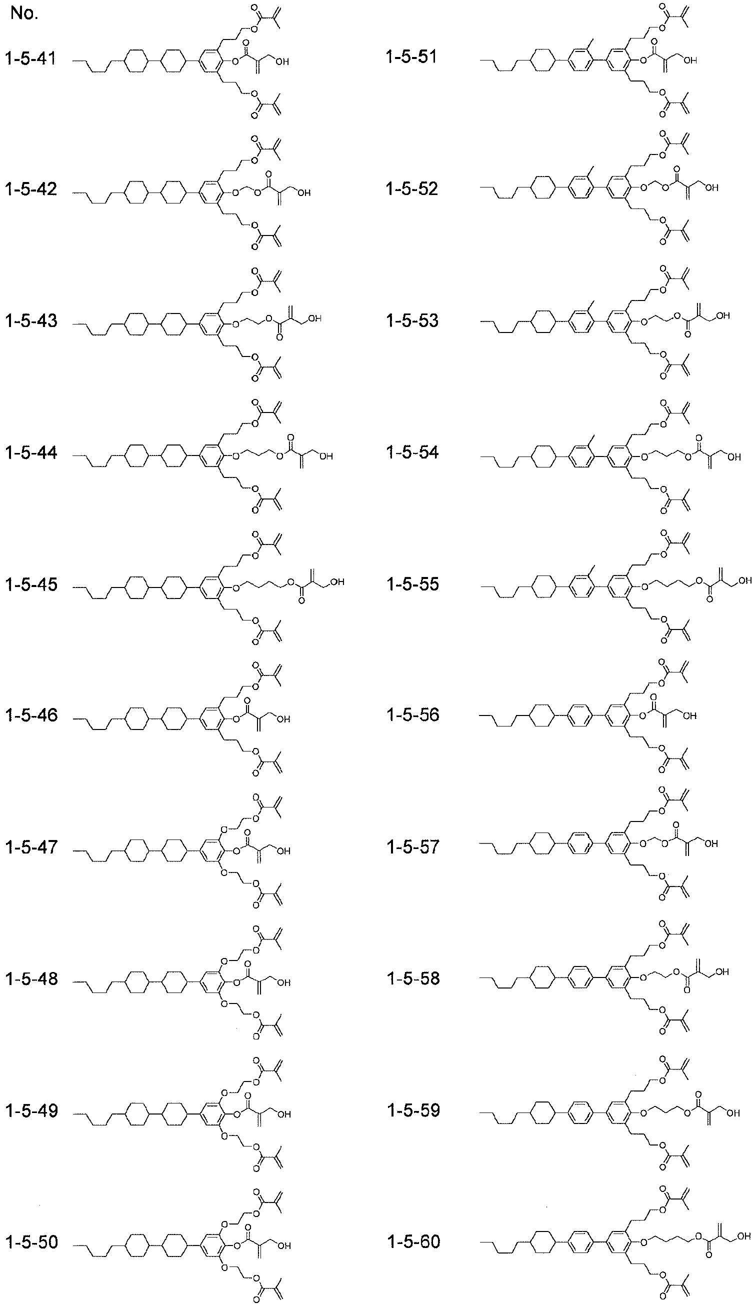

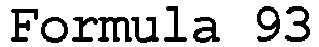

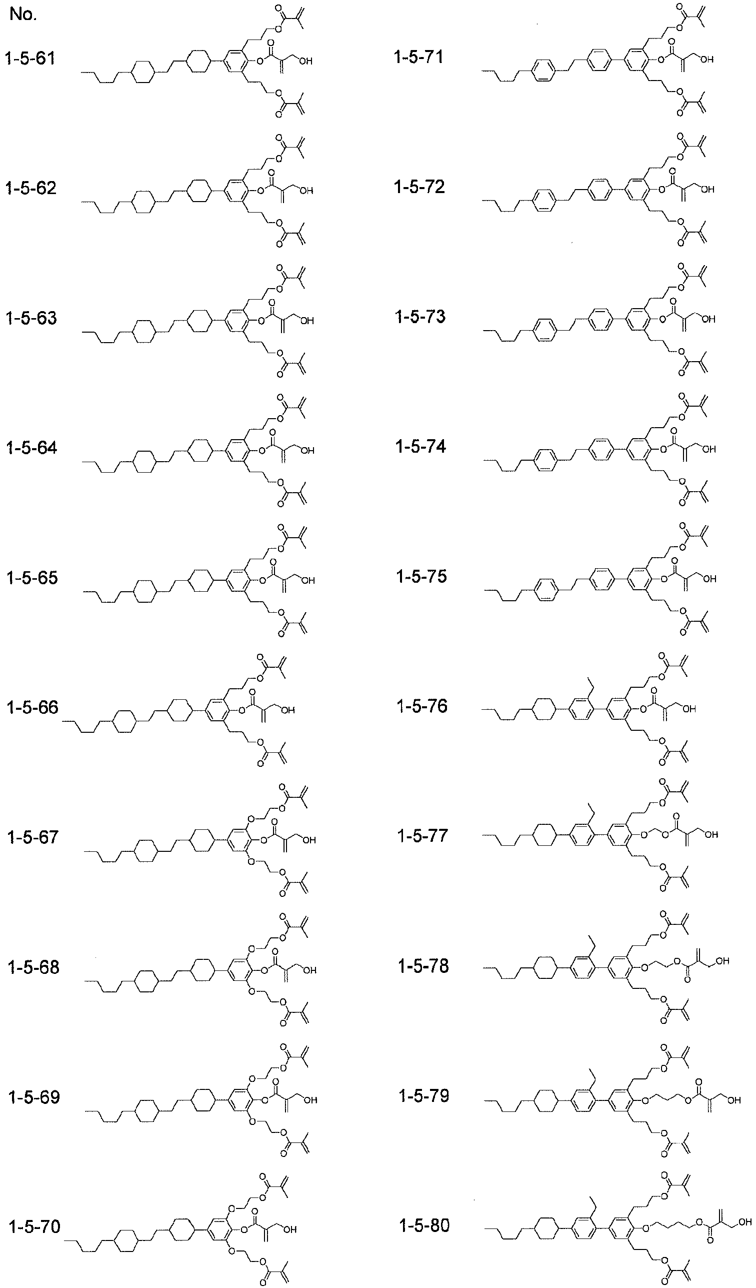

### 2. Examples of composition

The compounds in Examples were represented by using symbols according to definitions in Table 3 described below. In Table 3, the configuration of 1,4-cyclohexylene is trans. A parenthesized number next to a symbolized compound corresponds to the number of the compound. A symbol (-) means any other liquid crystal compound. A proportion (percentage) of the liquid crystal compound is expressed in terms of weight percent (% by weight) based on the weight of the liquid crystal composition. Values of the characteristics of the liquid crystal composition were summarized in the last part. The characteristics were measured according to the methods described above, and measured values are directly described (without extrapolation).

### Table 3

**Table 3. Method for description of compounds using symbols R-(A₁)-Z₁-·····-Zₙ-(Aₙ)-R'**

| 1) Left-terminal group R- | Symbol | 4) Ring structure -Aₙ- | Symbol |
|---|---|---|---|
| CₙH₂ₙ₊₁- | n- | 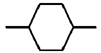 | |
| CₙH₂ₙ₊₁O- | nO- | | H |
| CₘH₂ₘ₊₁OCₙH₂ₙ- | mOn- | 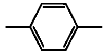 | |
| CH₂=CH- | V- | | B |
| CₙH₂ₙ₊₁-CH=CH- | nV- | | |
| CH₂=CH-CₙH₂ₙ- | Vn- | 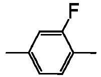 | |
| CₘH₂ₘ₊₁-CH=CH-CₙH₂ₙ- | mVn- | | B(F) |
| CF₂=CH- | VFF- | 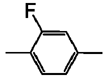 | |
| CF₂=CH-CₙH₂ₙ- | VFFn- | | |
| 2) Right-terminal group -R' | Symbol | | B(2F) |
| -CₙH₂ₙ₊₁ | -n | 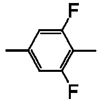 | |
| -OCₙH₂ₙ₊₁ | -On | | |
| -COOCH₃ | -EMe | | B(F,F) |
| -CH=CH₂ | -V | | |
| -CH=CH-CₙH₂ₙ₊₁ | -Vn | 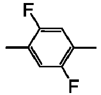 | |
| -CₙH₂ₙ-CH=CH₂ | -nV | | B(2F,5F) |
| -CₘH₂ₘ-CH=CH-CₙH₂ₙ₊₁ | -mVn | | |
| -CH=CF₂ | -VFF | 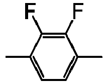 | |
| -F | -F | | B(2F,3F) |
| -Cl | -CL | | |
| -OCF₃ | -OCF3 | 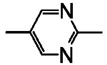 | |
| -OCF₂H | -OCF2H | | Py |
| -CF₃ | -CF3 | | |
| -OCH=CH-CF₃ | -OVCF3 | 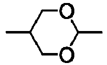 | |
| -C≡N | -C | | G |
| 3) Bonding group -Zₙ- | Symbol | | |
| -CₙH₂ₙ- | n | 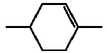 | ch |
| -COO- | E | 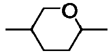 | |
| -CH=CH- | V | | dh |
| -CH₂O- | 1O | | |
| -OCH₂- | O1 | 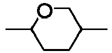 | Dh |
| -CF₂O- | X | | |
| -C≡C- | T | | |
| 5) Examples of description | | | |
| Example 1 3-HH-V | | Example 2 3-BB(F,F)XB(F,F)-F | |
| 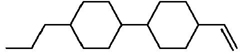 | | 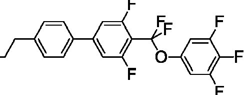 | |
| Example 3 3-HB-O2 | | Example 4 4-HHB(F)-F | |
| 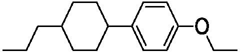 | | 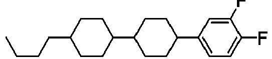 | |

### Use Example 1

| | | |
|---|---|---|
| 1-BB-3 | (2-8) | 7% |
| 1-BB-5 | (2-8) | 8% |
| 2-BTB-1 | (2-10) | 4% |
| 3-HHB-1 | (3-1) | 8% |
| 3-HHB-O1 | (3-1) | 6% |
| 3-HHB-3 | (3-1) | 14% |
| 3-HHB-F | (6-1) | 4% |
| 2-HHB(F)-F | (6-2) | 7% |
| 3-HHB(F)-F | (6-2) | 7% |
| 5-HHB(F)-F | (6-2) | 7% |
| 3-HHB(F,F)-F | (6-3) | 5% |
| 3-HHEB-F | (6-10) | 4% |
| 5-HHEB-F | (6-10) | 3% |
| 2-HB-C | (8-1) | 4% |
| 3-HB-C | (8-1) | 12% |

Compound (1-2-30) was added to the composition described above at a proportion of 1% by weight.

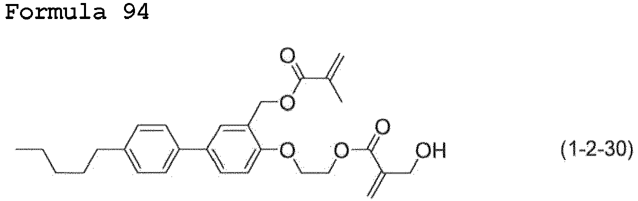

NI = 95.4°C; η = 15.9 mPa·s; Δn = 0.109; Δε = 4.8.

### Use Example 2

| | | |
|---|---|---|
| 3-HH-4 | (2-1) | 14% |
| 7-HB-1 | (2-5) | 5% |
| 5-HB-02 | (2-5) | 5% |
| 5-HBB(F)B-2 | (4-5) | 7% |
| 5-HBB(F)B-3 | (4-5) | 5% |
| 3-HB-CL | (5-2) | 13% |
| 3-HHB(F,F)-F | (6-3) | 3% |
| 3-HBB(F,F)-F | (6-24) | 28% |
| 5-HBB(F,F)-F | (6-24) | 20% |

Compound (1-3-31) was added to the composition described above at a proportion of 0.5% by weight.

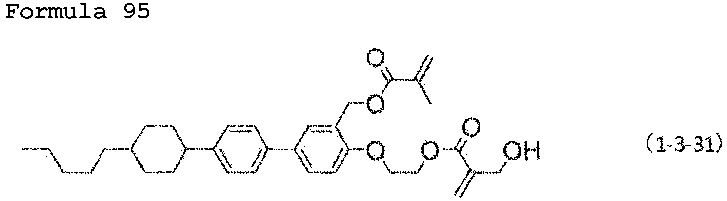

NI = 72.7°C; η = 17.7 mPa·s; Δn = 0.113; Δε = 5.2.

### Use Example 3

| | | |
|---|---|---|
| 1V2-HH-1 | (2-1) | 5% |
| 1V2-HH-3 | (2-1) | 6% |
| 7-HB(F,F)-F | (5-4) | 3% |
| 2-HHB(F)-F | (6-2) | 10% |
| 3-HHB(F)-F | (6-2) | 10% |
| 5-HHB(F)-F | (6-2) | 10% |
| 2-HBB-F | (6-22) | 4% |
| 3-HBB-F | (6-22) | 4% |
| 5-HBB-F | (6-22) | 3% |
| 2-HBB(F)-F | (6-23) | 9% |
| 3-HBB(F)-F | (6-23) | 9% |
| 5-HBB(F)-F | (6-23) | 16% |
| 3-HBB(F,F)-F | (6-24) | 3% |
| 5-HBB(F,F)-F | (6-24) | 8% |

Compound (1-3-44) was added to the composition described above at a proportion of 3% by weight.

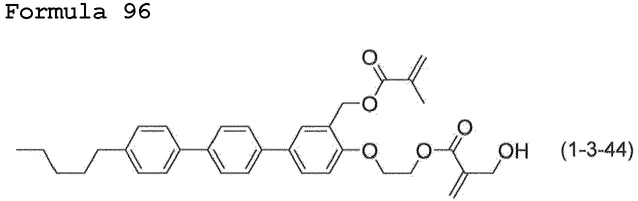

NI = 85.3°C; η = 23.1 mPa·s; Δn = 0.109; Δε = 5.2.

### Use Example 4

| | | |
|---|---|---|
| 2-HH-3 | (2-1) | 4% |
| 3-HH-4 | (2-1) | 10% |
| 101-HBBH-5 | (4-1) | 2% |
| 5-HB-CL | (5-2) | 16% |
| 3-HHB-F | (6-1) | 4% |
| 3-HHB-CL | (6-1) | 5% |
| 4-HHB-CL | (6-1) | 4% |
| 3-HHB(F)-F | (6-2) | 10% |
| 4-HHB(F)-F | (6-2) | 9% |
| 5-HHB(F)-F | (6-2) | 9% |
| 7-HHB(F)-F | (6-2) | 8% |
| 5-HBB(F)-F | (6-23) | 4% |
| 3-HHBB(F,F)-F | (7-6) | 3% |
| 4-HHBB(F,F)-F | (7-6) | 3% |
| 5-HHBB(F,F)-F | (7-6) | 3% |
| 3-HH2BB(F,F)-F | (7-15) | 3% |
| 4-HH2BB(F,F)-F | (7-15) | 3% |

Compound (1-3-51) was added to the composition described above at a proportion of 5% by weight.

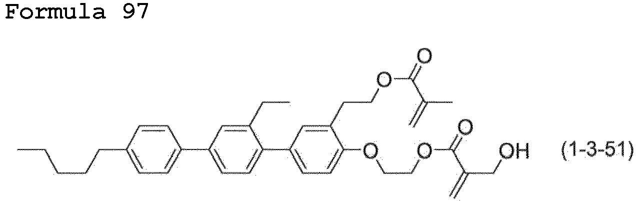

NI = 114.5°C; η = 19.6 mPa·s; Δn = 0.092; Δε = 3.9.

### Use Example 5

| | | |
|---|---|---|
| V-HBB-2 | (3-4) | 8% |
| 101-HBBH-4 | (4-1) | 3% |
| 101-HBBH-5 | (4-1) | 3% |
| 3-HHB(F,F)-F | (6-3) | 9% |
| 3-H2HB(F,F)-F | (6-15) | 10% |
| 4-H2HB(F,F)-F | (6-15) | 10% |
| 5-H2HB(F,F)-F | (6-15) | 8% |
| 3-HBB(F,F)-F | (6-24) | 11% |
| 5-HBB(F,F)-F | (6-24) | 20% |
| 3-H2BB(F,F)-F | (6-27) | 10% |
| 5-HHBB(F,F)-F | (7-6) | 3% |
| 3-HH2BB(F,F)-F | (7-15) | 3% |
| 5-HHEBB-F | (7-17) | 2% |

Compound (1-3-131) was added to the composition described above at a proportion of 1.5% by weight.

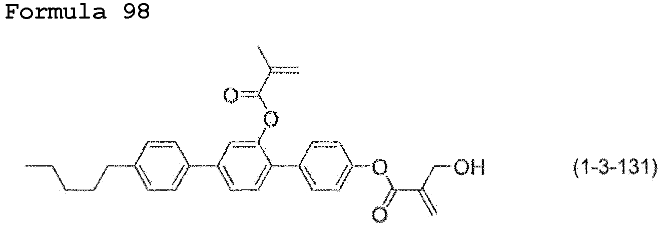

NI = 101.4°C; η = 32.4 mPa·s; Δn = 0.118; Δε = 8.4.

### Use Example 6

| | | |
|---|---|---|
| 5-HBBH-3 | (4-1) | 5% |
| 3-HB(F)BH-3 | (4-2) | 3% |
| 5-HB-F | (5-2) | 12% |
| 6-HB-F | (5-2) | 9% |
| 7-HB-F | (5-2) | 7% |
| 2-HHB-OCF3 | (6-1) | 5% |
| 3-HHB-OCF3 | (6-1) | 7% |
| 4-HHB-OCF3 | (6-1) | 7% |
| 5-HHB-OCF3 | (6-1) | 4% |
| 3-HHB(F,F)-OCF2H | (6-3) | 5% |
| 3-HHB(F,F)-OCF3 | (6-3) | 5% |
| 3-HH2B-OCF3 | (6-4) | 4% |
| 5-HH2B-OCF3 | (6-4) | 4% |
| 3-HH2B(F)-F | (6-5) | 3% |
| 3-HBB(F)-F | (6-23) | 10% |
| 5-HBB(F)-F | (6-23) | 10% |

Compound (1-3-25) was added to the composition described above at a proportion of 4% by weight.

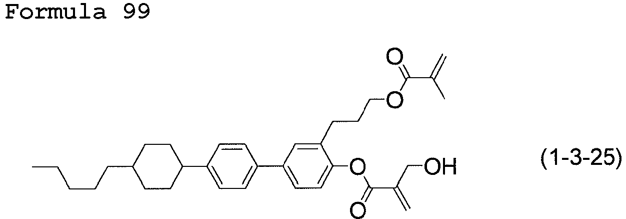

NI = 88.1°C; η = 15.9 mPa·s; Δn = 0.093; Δε = 4.46.

### Use Example 7

| | | |
|---|---|---|
| 3-HH-4 | (2-1) | 5% |
| 2-HH-5 | (2-1) | 5% |
| 5-B(F)BB-2 | (3-8) | 5% |
| 5-HB-CL | (5-2) | 11% |
| 3-HHB(F,F)-F | (6-3) | 7% |
| 3-HHEB(F,F)-F | (6-12) | 10% |
| 4-HHEB(F,F)-F | (6-12) | 3% |
| 5-HHEB(F,F)-F | (6-12) | 3% |
| 3-HBB(F,F)-F | (6-24) | 20% |
| 5-HBB(F,F)-F | (6-24) | 15% |
| 2-HBEB(F,F)-F | (6-39) | 3% |
| 3-HBEB(F,F)-F | (6-39) | 5% |
| 5-HBEB(F,F)-F | (6-39) | 3% |
| 3-HHBB(F,F)-F | (7-6) | 5% |

Compound (1-3-2) was added to the composition described above at a proportion of 0.8% by weight.

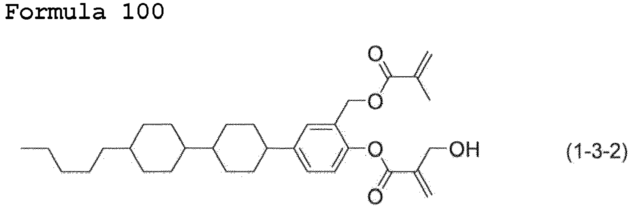

NI = 75.8°C; η = 21.9 mPa·s; Δn = 0.109; Δε = 8.3.

### Use Example 8

| | | |
|---|---|---|
| V2-HHB-1 | (3-1) | 5% |
| 3-HB-CL | (5-2) | 6% |
| 5-HB-CL | (5-2) | 4% |
| 3-HHB-OCF3 | (6-1) | 6% |
| V-HHB(F)-F | (6-2) | 4% |
| 5-HHB(F)-F | (6-2) | 5% |
| 3-H2HB-OCF3 | (6-13) | 5% |
| 5-H2HB(F,F)-F | (6-15) | 7% |
| 5-H4HB-OCF3 | (6-19) | 15% |
| 3-H4HB(F,F)-CF3 | (6-21) | 8% |

| | | |
|---|---|---|
| 5-H4HB(F,F)-CF3 | (6-21) | 10% |
| 5-H4HB(F,F)-F | (6-21) | 7% |
| 2-H2BB(F)-F | (6-26) | 5% |
| 3-H2BB(F)-F | (6-26) | 8% |
| 3-HBEB(F,F)-F | (6-39) | 5% |

Compound (1-3-45) was added to the composition described above at a proportion of 2.5% by weight.

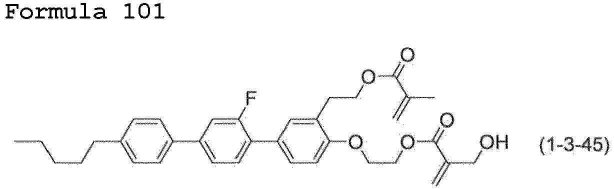

NI = 72.7°C; η = 24.9 mPa·s; Δn = 0.097; Δε = 8.1.

### Use Example 9

| | | |
|---|---|---|
| 3-HH-4 | (2-1) | 12% |
| 3-HH-5 | (2-1) | 5% |
| 3-HB-O2 | (2-5) | 13% |
| 3-HHB-1 | (3-1) | 8% |
| 3-HHB-O1 | (3-1) | 6% |
| 5-HB-CL | (5-2) | 17% |
| 7-HB(F,F)-F | (5-4) | 2% |
| 2-HHB(F)-F | (6-2) | 9% |
| 3-HHB(F)-F | (6-2) | 7% |
| 5-HHB(F)-F | (6-2) | 7% |
| 3-HHB(F,F)-F | (6-3) | 6% |
| 3-H2HB(F,F)-F | (6-15) | 4% |
| 4-H2HB(F,F)-F | (6-15) | 4% |

Compound (1-3-60) was added to the composition described above at a proportion of 3.5% by weight.

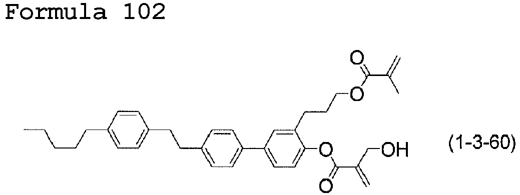

NI = 74.7°C; η = 13.6 mPa·s; Δn = 0.075; Δε = 2.7.

### Use Example 10

| | | |
|---|---|---|
| 3-HH-4 | (2-1) | 9% |
| 3-HH-5 | (2-1) | 10% |
| 4-HH-V | (2-1) | 13% |
| 5-HB-CL | (5-2) | 3% |
| 7-HB(F)-F | (5-3) | 2% |
| 2-HHB(F,F)-F | (6-3) | 4% |
| 3-HHB(F,F)-F | (6-3) | 5% |
| 3-HHEB-F | (6-10) | 12% |
| 5-HHEB-F | (6-10) | 10% |
| 3-HHEB(F,F)-F | (6-12) | 10% |
| 4-HHEB(F,F)-F | (6-12) | 6% |
| 3-GHB(F,F)-F | (6-109) | 5% |
| 4-GHB(F,F)-F | (6-109) | 6% |
| 5-GHB(F,F)-F | (6-109) | 5% |

Compound (1-2-30) was added to the composition described above at a proportion of 4.5% by weight.

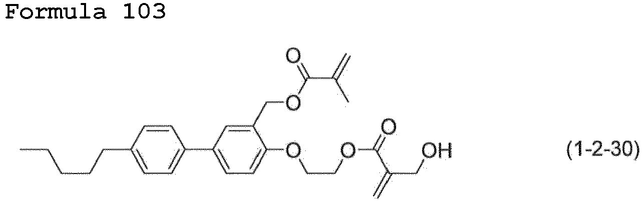

NI = 86.1°C; η = 18.5 mPa·s; Δn = 0.065; Δε = 5.2.

### Use Example 11

| | | |
|---|---|---|
| 3-HH-VFF | (2-1) | 7% |
| 5-HH-VFF | (2-1) | 23% |
| 2-BTB-1 | (2-10) | 10% |
| 3-HHB-1 | (3-1) | 4% |
| VFF-HHB-1 | (3-1) | 8% |
| VFF2-HHB-1 | (3-1) | 11% |
| 3-H2BTB-2 | (3-17) | 5% |
| 3-H2BTB-3 | (3-17) | 4% |
| 3-H2BTB-4 | (3-17) | 4% |
| 3-HB-C | (8-1) | 20% |
| 1V2-BEB(F,F)-C | (8-15) | 4% |

Compound (1-3-31) was added to the composition described above at a proportion of 4% by weight.

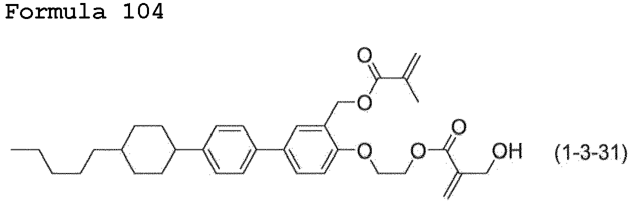

NI = 81.5°C; η = 10.1 mPa·s; Δn = 0.129; Δε = 5.4.

### Use Example 12

| | | |
|---|---|---|
| 3-HH-V | (2-1) | 35% |
| 3-HH-V1 | (2-1) | 7% |
| 5-HH-V | (2-1) | 4% |
| 3-HHB-1 | (3-1) | 4% |
| V-HHB-1 | (3-1) | 5% |
| 2-BB(F)B-3 | (3-6) | 5% |
| 3-HHEH-5 | (3-13) | 3% |
| 1V2-BB-F | (5-1) | 3% |
| 3-BB(F,F)XB(F,F)-F | (6-97) | 11% |
| 3-BB(2F,3F)XB(F,F)-F | (6-114) | 3% |
| 3-HHBB(F,F)-F | (7-6) | 3% |
| 3-HBBXB(F,F)-F | (7-32) | 3% |
| 5-HB(F)B(F,F)XB(F,F)-F | (7-41) | 5% |
| 3-BB(F)B(F,F)XB(F,F)-F | (7-47) | 3% |
| 4-BB(F)B(F,F)XB(F,F)-F | (7-47) | 4% |
| 5-BB(F)B(F,F)XB(F,F)-F | (7-47) | 2% |

Compound (1-3-44) was added to the composition described above at a proportion of 1% by weight.

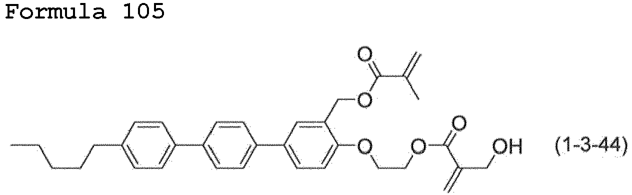

NI = 77.3°C; η = 10.2 mPa·s; Δn = 0.096; Δε = 5.1.

### Use Example 13

| | | |
|---|---|---|
| 3-HH-V | (2-1) | 31% |
| 3-HH-V1 | (2-1) | 7% |
| V-HH-V1 | (2-1) | 8% |
| 3-HHB-1 | (3-1) | 4% |
| V-HHB-1 | (3-1) | 5% |
| 1-BB(F)B-2V | (3-6) | 4% |
| 3-HHEH-5 | (3-13) | 3% |
| 1V2-BB-F | (5-1) | 3% |
| 3-BB(F,F)XB(F,F)-F | (6-97) | 5% |
| 3-HHXB(F,F)-CF3 | (6-100) | 2% |
| 3-GB(F,F)XB(F,F)-F | (6-113) | 4% |
| 3-GB(F)B(F,F)-F | (6-116) | 4% |
| 3-HHBB(F,F)-F | (7-6) | 3% |
| 3-BB(F)B(F,F)XB(F,F)-F | (7-47) | 4% |
| 4-BB(F)B(F,F)XB(F,F)-F | (7-47) | 7% |
| 5-BB(F)B(F,F)XB(F,F)-F | (7-47) | 3% |
| 3-GB(F)B(F,F)XB(F,F)-F | (7-57) | 3% |

Compound (1-3-51) was added to the composition described above at a proportion of 2% by weight.

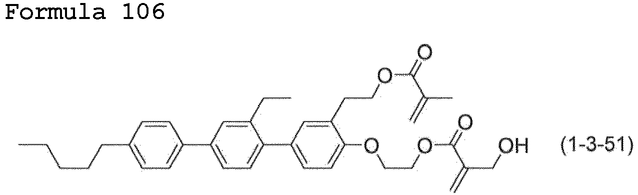

NI = 80.4°C; η = 11.3 mPa·s; Δn = 0.103; Δε = 6.4.

### Use Example 14

| | | |
|---|---|---|
| 3-HH-V | (2-1) | 37% |
| 3-HH-V1 | (2-1) | 5% |
| 3-HHB-1 | (3-1) | 4% |
| V-HHB-1 | (3-1) | 5% |
| 3-HBB-2 | (3-4) | 5% |
| V2-BB(F)B-1 | (3-6) | 5% |
| 3-HHEH-3 | (3-13) | 3% |
| 3-HHEH-5 | (3-13) | 2% |
| 1V2-BB-F | (5-1) | 3% |
| 3-BB(F,F)XB(F,F)-F | (6-97) | 3% |
| 3-GB(F,F)XB(F,F)-F | (6-113) | 2% |
| 3-HHBB(F,F)-F | (7-6) | 4% |
| 3-HBB (F,F)XB (F,F) -F | (7-38) | 3% |
| 3-BB(F)B(F,F)XB(F)-F | (7-46) | 3% |
| 4-BB(F)B(F,F)XB(F,F)-F | (7-47) | 3% |
| 5-BB(F)B(F,F)XB(F,F)-F | (7-47) | 3% |
| 3-GB(F)B(F,F)XB(F,F)-F | (7-57) | 5% |
| 4-GB(F)B(F,F)XB(F,F)-F | (7-57) | 3% |
| 5-GB(F)B(F,F)XB(F,F)-F | (7-57) | 2% |

Compound (1-3-131) was added to the composition described above at a proportion of 3.5% by weight.

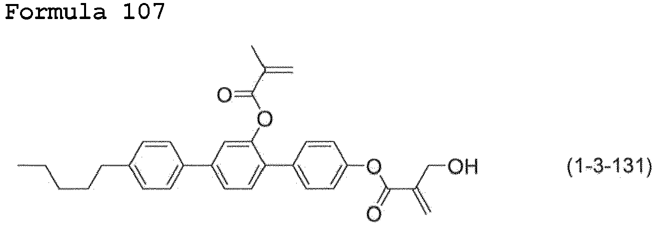

NI = 81.4°C; η = 11.9 mPa·s; Δn = 0.091; Δε = 5.4.

### Use Example 15

| | | |
|---|---|---|
| 3-HH-V | (2-1) | 34% |
| 3-HH-V1 | (2-1) | 4% |
| 3-HHB-1 | (3-1) | 6% |
| V-HHB-1 | (3-1) | 4% |
| V2-BB(F)B-1 | (3-6) | 4% |
| 3-HHEH-5 | (3-13) | 3% |
| 3-HHEBH-3 | (4-6) | 4% |
| 1V2-BB-F | (5-1) | 4% |
| 3-BB(F)B(F,F)-F | (6-69) | 3% |
| 3-BB(F,F)XB(F,F)-F | (6-97) | 5% |
| 3-HHBB(F,F)-F | (7-6) | 3% |
| 5-HB(F)B(F,F)XB(F,F)-F | (7-41) | 4% |
| 4-BB(F)B(F,F)XB(F,F)-F | (7-47) | 5% |
| 5-BB(F)B(F,F)XB(F,F)-F | (7-47) | 2% |
| 2-dhBB (F,F)XB (F,F) -F | (7-50) | 2% |
| 3-dhBB(F,F)XB(F,F)-F | (7-50) | 3% |
| 3-GBB(F)B(F,F)-F | (7-55) | 3% |
| 4-GBB(F)B(F,F)-F | (7-55) | 3% |
| 3-BB(F,F)XB(F)B(F,F)-F | (7-56) | 4% |

Compound (1-3-25) was added to the composition described above at a proportion of 0.5% by weight.

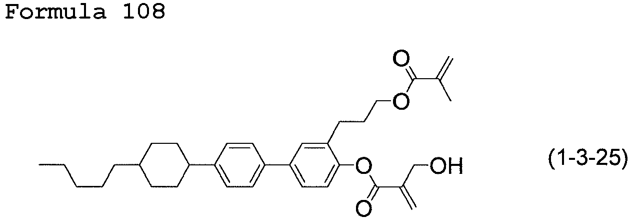

NI = 84.9°C; η = 17.8 mPa·s; Δn = 0.100; Δε = 5.4.

### Use Example 16

| | | |
|---|---|---|
| 3-HH-V | (2-1) | 35% |
| 3-HH-V1 | (2-1) | 6% |
| 3-HHB-1 | (3-1) | 4% |
| V-HHB-1 | (3-1) | 5% |
| V2-BB(F)B-1 | (3-6) | 6% |
| 3-HHEH-5 | (3-13) | 3% |
| 1V2-BB-F | (5-1) | 3% |
| 3-BB(F)B(F,F)-CF3 | (6-69) | 3% |
| 3-BB(F,F)XB(F,F)-F | (6-97) | 5% |
| 3-HHXB(F,F)-F | (6-100) | 5% |
| 3-GB(F,F)XB(F,F)-F | (6-113) | 3% |
| 3-GB(F)B(F)-F | (6-115) | 3% |
| 3-HHBB(F,F)-F | (7-6) | 3% |
| 5-HB(F)B(F,F)XB(F,F)-F | (7-41) | 3% |
| 3-GB(F)B(F,F)XB(F,F)-F | (7-57) | 2% |
| 3-GBB (F,F)XB (F,F) -F | (7-58) | 2% |
| 4-GBB(F,F)XB(F,F)-F | (7-58) | 3% |
| 5-GBB (F,F)XB (F,F) -F | (7-58) | 3% |
| 3-GB(F)B(F)B(F)-F | (7-59) | 3% |

Compound (1-3-2) was added to the composition described above at a proportion of 2.5% by weight.

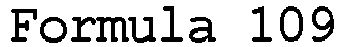

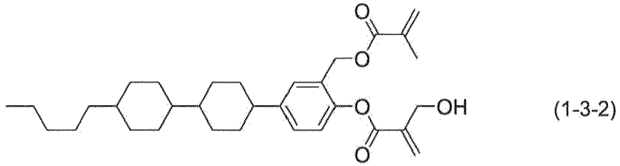

NI = 81.4°C; η = 14.0 mPa·s; Δn = 0.093; Δε = 5.1.

### Industrial Applicability

A liquid crystal composition comprising compound (1) can be used in a display device in the form of a liquid crystal projector, a liquid crystal television or the like.

## Claims

1. A compound, represented by formula (1): 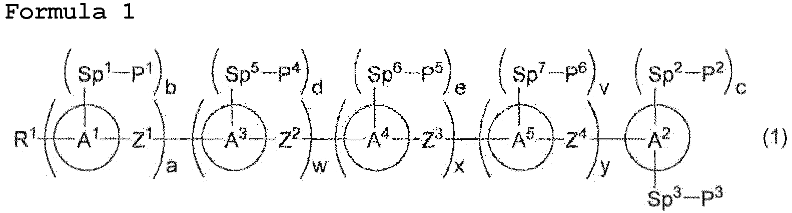 wherein, in formula (1),
R¹ is hydrogen or alkyl having 1 to 15 carbons, and in the alkyl, at least one piece of -CH₂- may be replaced by -O- or -S-, and at least one piece of -(CH₂)₂- may be replaced by -CH=CH- or -C≡C-, and in the groups, at least one hydrogen may be replaced by fluorine or chlorine;
ring A¹, ring A³, ring A⁴, ring A⁵ and ring A² are independently cyclopropyl, cyclobutyl, cyclopentyl, cyclohexyl, cycloheptyl, cyclohexenyl, phenyl, 2-naphthyl, decahydronaphthalene-2-yl, 1,2,3,4-tetrahydronaphthalene-2-yl, 1,2,3,4-tetrahydronaphthalene-6-yl, tetrahydropyran-2-yl, 1,3-dioxane-2-yl, pyrimidine-2-yl or pyridine-2-yl, and in the rings, at least one hydrogen may be replaced by fluorine, chlorine, alkyl having 1 to 10 carbons, alkenyl having 2 to 10 carbons, alkoxy having 1 to 9 carbons or alkenyloxy having 2 to 9 carbons, and in the groups, at least one hydrogen may be replaced by fluorine or chlorine;
a, w, x and y are independently 0 or 1;
b, d, e, v and c are independently 0, 1, 2, 3 or 4, and a sum of b, d, e, v and c is 1, 2, 3 or 4;
Z¹, Z², Z³ and Z⁴ are independently a single bond or alkylene having 1 to 6 carbons, and in the alkylene, at least one piece of -CH₂- may be replaced by -0-, -CO-, -COO-, -OCO- or -OCOO-, and at least one piece of -(CH₂)₂- may be replaced by -CH=CH- or -C≡C-, and in the groups, at least one hydrogen may be replaced by fluorine or chlorine;
Sp¹, Sp⁵, Sp⁶, Sp⁷, Sp² and Sp³ are independently a single bond or alkylene having 1 to 10 carbons, and in the alkylene, at least one piece of -CH₂- may be replaced by -0-, -CO-, -COO-, -OCO- or -OCOO-, and at least one piece of -(CH₂)₂- may be replaced by -CH=CH- or -C≡C-, and in the groups, at least one hydrogen may be replaced by fluorine or chlorine; and
P¹, P⁴, P⁵, P⁶ and P² are independently a group represented by formula (1a) ; 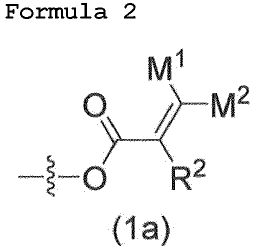
wherein, in formula (1a),
M¹ and M² are independently hydrogen, fluorine, chlorine, alkyl having 1 to 5 carbons, or alkyl having 1 to 5 carbons in which at least one hydrogen is replaced by fluorine or chlorine; and
R² is hydrogen, alkyl having 1 to 10 carbons, alkoxy having 1 to 9 carbons, alkoxyalkyl having 1 to 9 carbons, alkylthio having 1 to 9 carbons or alkylthioalkoxy having 1 to 9 carbons, and in the groups, at least one piece of -(CH₂)₂- may be replaced by -CH=CH- or -C≡C-, and in the groups, at least one hydrogen may be replaced by fluorine or chlorine; and
in formula (1),
P³ is a group represented by formula (1b); 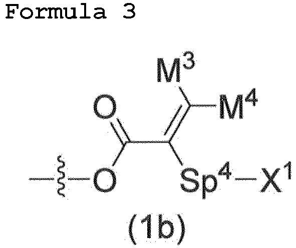
wherein, in formula (1b),
Sp⁴ is a single bond or alkylene having 1 to 10 carbons, and in the alkylene, at least one piece of -CH₂- may be replaced by -0-, -NH-, -CO-, -COO-, -OCO- or -OCOO-, and at least one piece of -(CH₂)₂- may be replaced by -CH=CH- or -C≡C-, and in the groups, at least one hydrogen may be replaced by fluorine or chlorine;
M³ and M⁴ are independently hydrogen, fluorine, chlorine, alkyl having 1 to 5 carbons, or alkyl having 1 to 5 carbons in which at least one hydrogen is replaced by fluorine or chlorine; and
X¹ is -OH, -NH₂, -OR³, -N(R³)₂, -COOH, -SH or -Si(R³)₃; and
in -OR³, -N(R³)₂ and -Si(R³)₃,
R³ is hydrogen or alkyl having 1 to 10 carbons, and in the alkyl, at least one piece of -CH₂- may be replaced by -0-, and at least one piece of -(CH₂)₂- may be replaced by -CH=CH-, and in the groups, at least one hydrogen may be replaced by fluorine or chlorine, and when R³ is plural, each thereof may be identical or different.

2. The compound according to claim 1, wherein, in formula (1), Z¹, Z², Z³ and Z⁴ are independently a single bond, - (CH₂)₂-, - (CH₂)₄-, -CH=CH-, -C≡C-, -COO-, -OCO-, -CF₂O-, -OCF₂-, -CH₂O-, -OCH₂- or -CF=CF-.

3. The compound according to claim 1 or 2, wherein, in formula (1), ring A¹, ring A³, ring A⁴, ring A⁵ and ring A² are independently cyclohexyl, cyclohexenyl, phenyl, 2-naphthyl, tetrahydropyran-2-yl, 1,3-dioxane-2-yl, pyrimidine-2-yl or pyridine-2-yl, and in the rings, at least one hydrogen may be replaced by fluorine, chlorine, alkyl having 1 to 10 carbons, alkenyl having 2 to 10 carbons, alkoxy having 1 to 9 carbons or alkenyloxy having 2 to 9 carbons, and in the groups, at least one hydrogen may be replaced by fluorine or chlorine.

4. The compound according to any one of claims 1 to 3, represented by any one of formula (1-1) to formula (1-4): 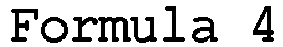 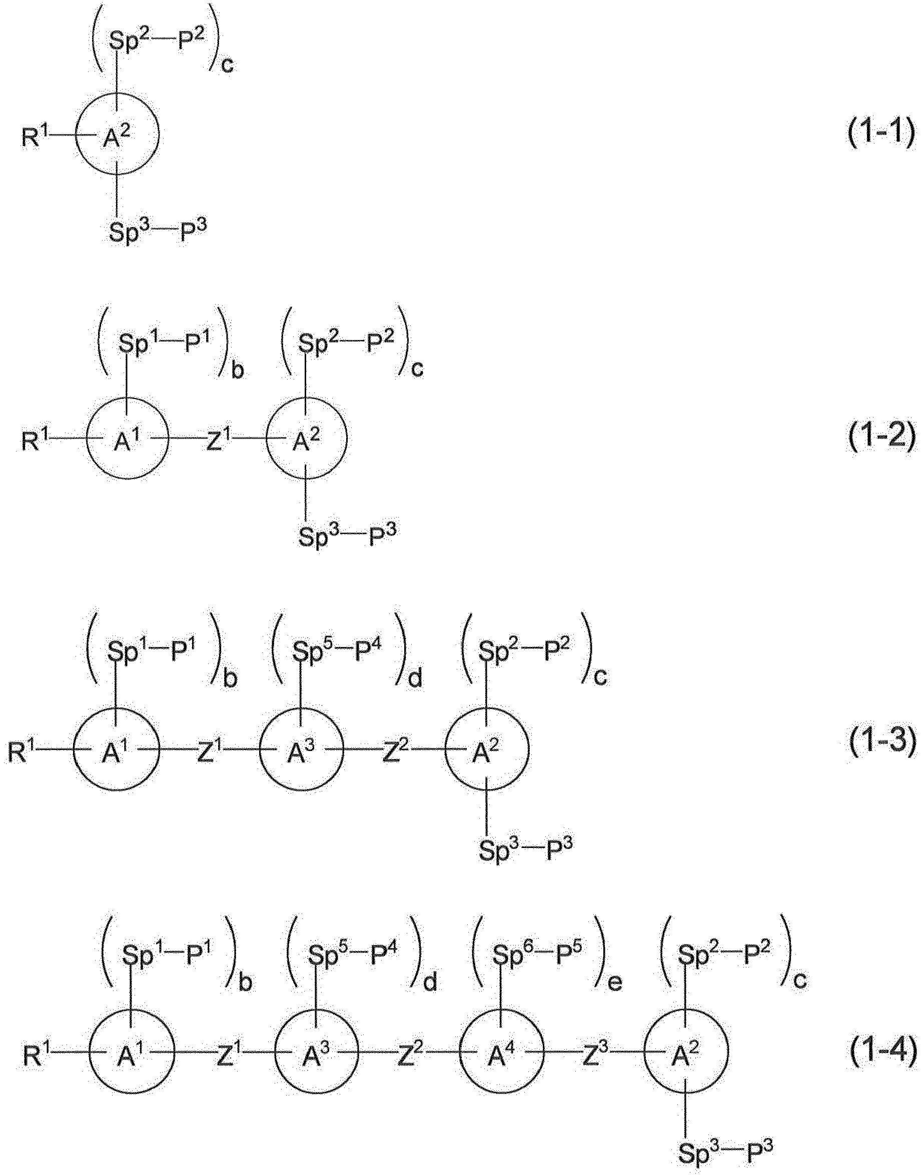 wherein, in formula (1-1) to formula (1-4),
R¹ is alkyl having 1 to 15 carbons, alkenyl having 2 to 15 carbons, alkoxy having 1 to 14 carbons, alkenyloxy having 2 to 14 carbons or alkoxyalkyl having 1 to 14, and in the groups, at least one hydrogen may be replaced by fluorine;
ring A¹, ring A³ and ring A⁴ are independently 1,4-cyclohexylene, 1,4-cyclohexenylene, 1,4-phenylene, naphthalene-2,6-diyl, tetrahydropyran-2,5-diyl or 1,3-dioxane-2,5-diyl, and ring A² is cyclohexyl, cyclohexenyl, phenyl, 2-naphthyl, tetrahydropyran-2-yl or 1,3-dioxane-2-yl, and in the rings, at least one hydrogen may be replaced by fluorine, alkyl having 1 to 10 carbons, alkenyl having 2 to 10 carbons, alkoxy having 1 to 9 carbons or alkenyloxy having 2 to 9 carbons, and in the groups, at least one hydrogen may be replaced by fluorine;
b, c, d and e are independently 0, 1 or 2, and a sum of b, c, d and e is 1 or 2;
Z¹, Z² and Z³ are independently a single bond, - (CH₂)₂-, -CH=CH-, -C≡C-, -COO-, -OCO-, -CF₂O-, -OCF₂-, -CH₂O-, -OCH₂- or -CF=CF-;
Sp¹, Sp², Sp³, Sp⁵ and Sp⁶ are independently a single bond or alkylene having 1 to 7 carbons, and in the alkylene, at least one piece of -CH₂-may be replaced by -0-, -COO- or -OCO-, and at least one piece of - (CH₂)₂-may be replaced by -CH=CH-, and in the groups, at least one hydrogen may be replaced by fluorine; and
P¹, P², P⁴ and P⁵ are independently a group represented by formula (1a); 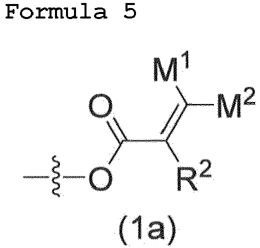
wherein, in formula (1a),
M¹ and M² are independently hydrogen, fluorine, alkyl having 1 to 5 carbons, or alkyl having 1 to 5 carbons in which at least one hydrogen is replaced by fluorine; and
R² is hydrogen, alkyl having 1 to 5 carbons, alkoxy having 1 to 4 carbons, alkoxyalkyl having 1 to 4 carbons, alkylthio having 1 to 4 carbons or alkylthioalkoxy having 1 to 4 carbons, and in the groups, at least one piece of -(CH₂)₂- may be replaced by -CH=CH-, and in the groups, at least one hydrogen may be replaced by fluorine; and
in formula (1-1) to formula (1-4),
P³ is a group represented by formula (1b); 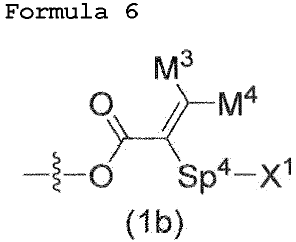
wherein, in formula (1b),
Sp⁴ is a single bond or alkylene having 1 to 7 carbons, and in the alkylene, at least one piece of -CH₂- may be replaced by -0-, -NH-, -CO-, -COO- or -OCO-, and at least one piece of - (CH₂)₂- may be replaced by -CH=CH-, and in the groups, at least one hydrogen may be replaced by fluorine;
M³ and M⁴ are independently hydrogen, fluorine, alkyl having 1 to 5 carbons, or alkyl having 1 to 5 carbons in which at least one hydrogen is replaced by fluorine; and
X¹ is -OH, -NH₂ or -SH.

5. The compound according to any one of claims 1 to 4, represented by any one of formula (1-5) to formula (1-16): 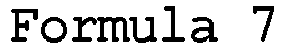 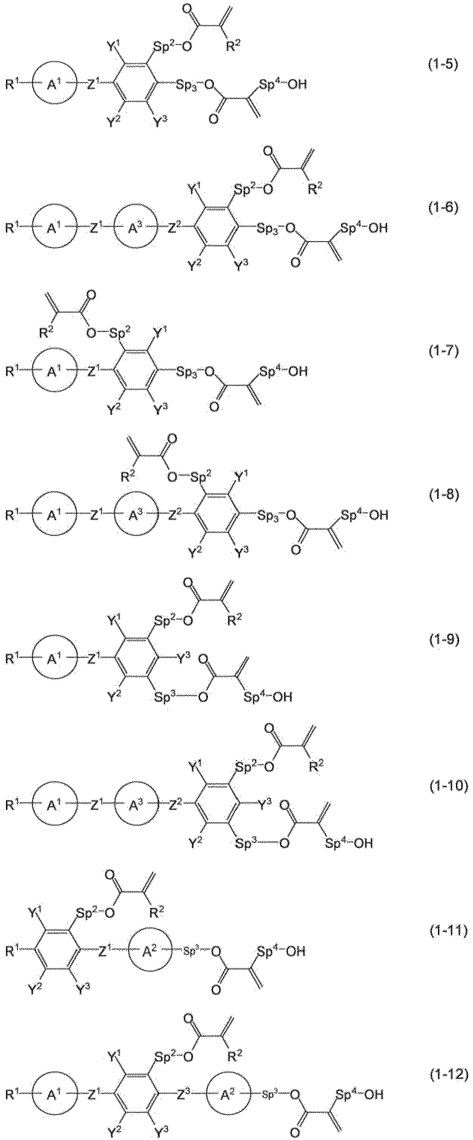 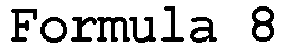 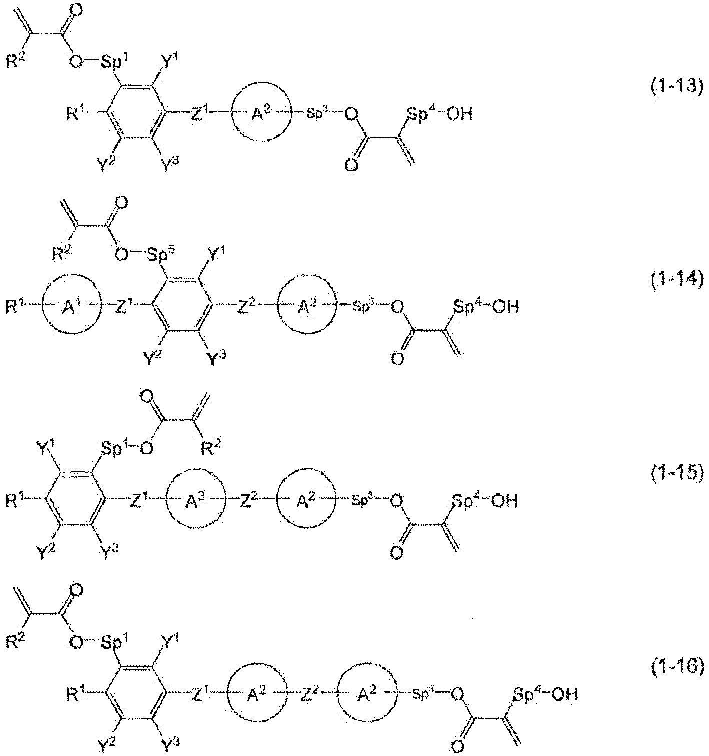 wherein, in formula (1-5) to formula (1-16),
R¹ is alkyl having 1 to 10 carbons, alkenyl having 2 to 10 carbons or alkoxy having 1 to 9 carbons;
ring A¹, ring A² and ring A³ are independently 1,4-cyclohexylene, 1,4-cyclohexenylene, 1,4-phenylene, naphthalene-2,6-diyl, tetrahydropyran-2,5-diyl or 1,3-dioxane-2,5-diyl, and in the rings, at least one hydrogen may be replaced by fluorine, alkyl having 1 to 5 carbons, alkenyl having 2 to 5 carbons or alkoxy having 1 to 4 carbons;
Y¹, Y² and Y³ are independently hydrogen, fluorine, alkyl having 1 to 5 carbons, alkenyl having 2 to 5 carbons or alkoxy having 1 to 4 carbons;
Z¹ and Z² are independently a single bond, - (CH₂)₂-, -CH=CH-, -C≡C-, -CH₂O- or -OCH₂-;
Sp¹, Sp², Sp³ and Sp⁵ are independently a single bond or alkylene having 1 to 5 carbons, and in the alkylene, at least one piece of -CH₂-may be replaced by -0-, and at least one piece of - (CH₂)₂- may be replaced by -CH=CH-;
R² is hydrogen, alkyl having 1 to 5 carbons, alkoxy having 1 to 4 carbons, alkoxyalkyl having 1 to 4 carbons, alkylthio having 1 to 4 carbons or alkylthioalkoxy having 1 to 4 carbons; and
Sp⁴ is a single bond or alkylene having 1 to 5 carbons, and in the alkylene, at least one piece of -CH₂- may be replaced by -0-, -NH-, -CO-, -COO- or -OCO-, and at least one piece of - (CH₂)₂- may be replaced by -CH=CH-.

6. The compound according to any one of claims 1 to 5, represented by any one of formula (1-17) to formula (1-39): 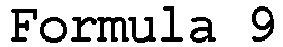 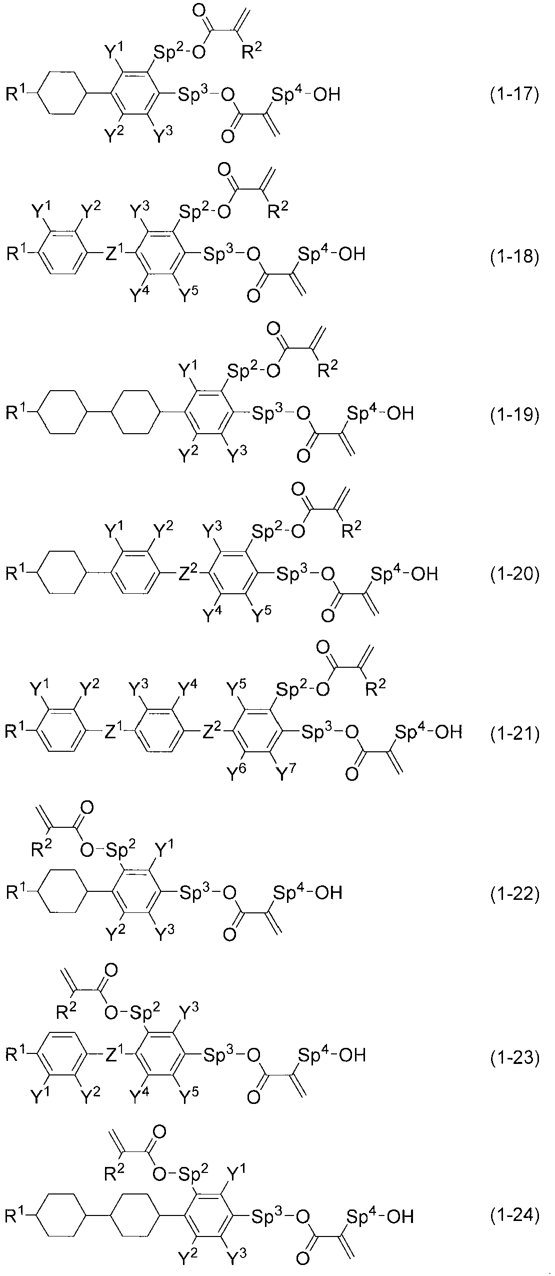 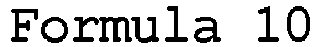 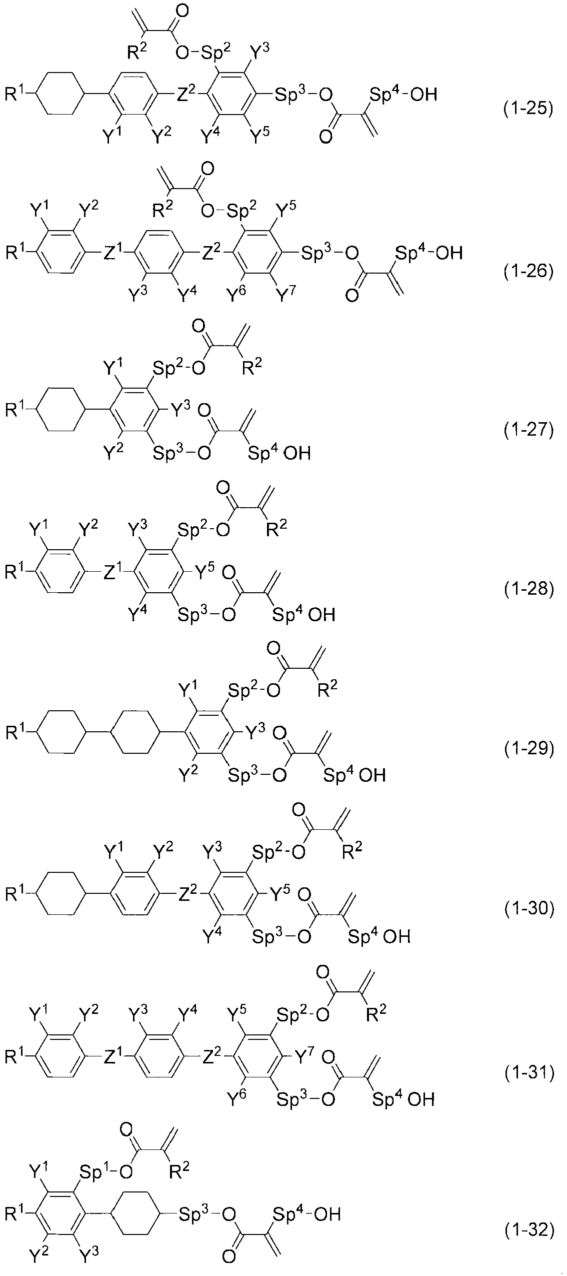 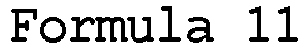 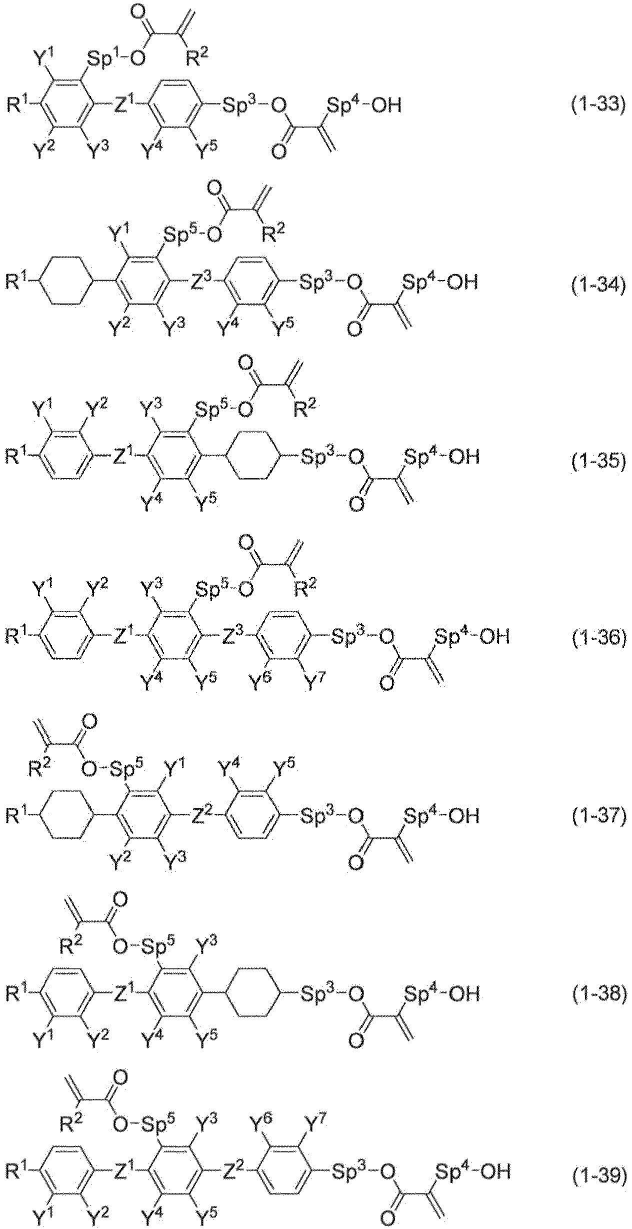 wherein, in formula (1-17) to formula (1-39),
R¹ is alkyl having 1 to 10 carbons, alkenyl having 2 to 10 carbons or alkoxy having 1 to 9 carbons;
Y¹, Y², Y³, Y⁴, Y⁵, Y⁶ and Y⁷ are independently hydrogen, fluorine, or alkyl having 1 to 5 carbons;
Z¹, Z² and Z³ are independently a single bond or -(CH₂)₂-;
Sp¹, Sp², Sp³, Sp⁴ and Sp⁵ are independently a single bond or alkylene having 1 to 5 carbons, and in the alkylene, at least one piece of -CH₂-may be replaced by -0-; and
R² is hydrogen, alkyl having 1 to 3 carbons, alkoxy having 1 or 2 carbons, alkoxyalkyl having 1 or 2 carbons, alkylthio having 1 or 2 carbons or alkylthioalkoxy having 1 or 2 carbons.

7. The compound according to any one of claims 1 to 6, represented by any one of formula (1-40) to formula (1-47): 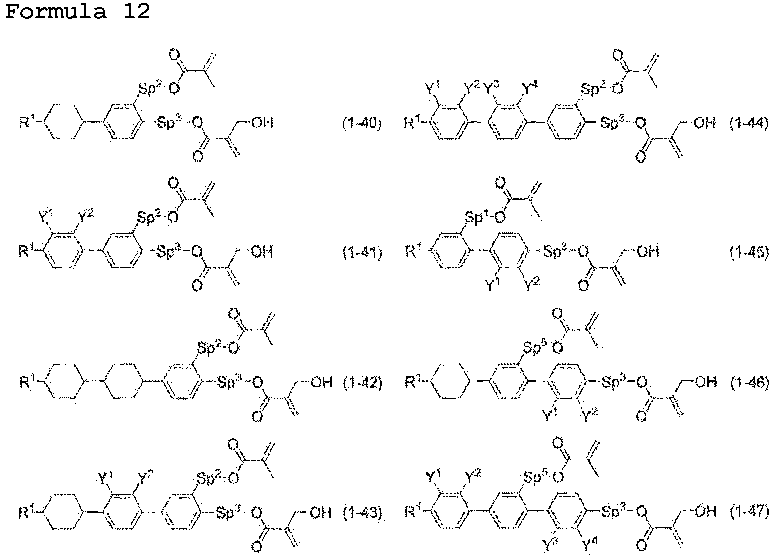 wherein, in formula (1-40) to formula (1-47),
R¹ is alkyl having 1 to 10 carbons; and
Y¹, Y², Y³ and Y⁴ are independently hydrogen, fluorine, methyl or ethyl, and
Sp¹, Sp², Sp³ and Sp⁵ are independently a single bond or alkylene having 1 to 5 carbons, and in the alkylene, at least one piece of -CH₂-may be replaced by -0-.

8. A liquid crystal composition, comprising at least one compound according to any one of claims 1 to 7.

9. The liquid crystal composition according to claim 8, further comprising at least one compound selected from the group of compounds represented by formulas (2) to (4): 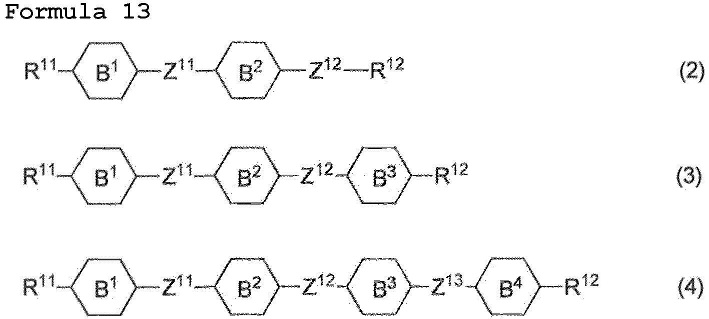 wherein, in formulas (2) to (4),
R¹¹ and R¹² are independently alkyl having 1 to 10 carbons or alkenyl having 2 to 10 carbons, and in the alkyl and the alkenyl, at least one piece of -CH₂- may be replaced by -0-, and in the groups, at least one hydrogen may be replaced by fluorine;
ring B¹, ring B², ring B³ and ring B⁴ are independently 1,4-cyclohexylene, 1,4-phenylene, 2-fluoro-1,4-phenylene, 2,5-difluoro-1,4-phenylene or pyrimidine-2,5-diyl; and
Z¹¹, Z¹² and Z¹³ are independently a single bond, -COO-, -CH₂CH₂-, -CH=CH- or -C≡C-.

10. The liquid crystal composition according to claim 8 or 9, further comprising at least one compound selected from the group of compounds represented by formulas (5) to (7): 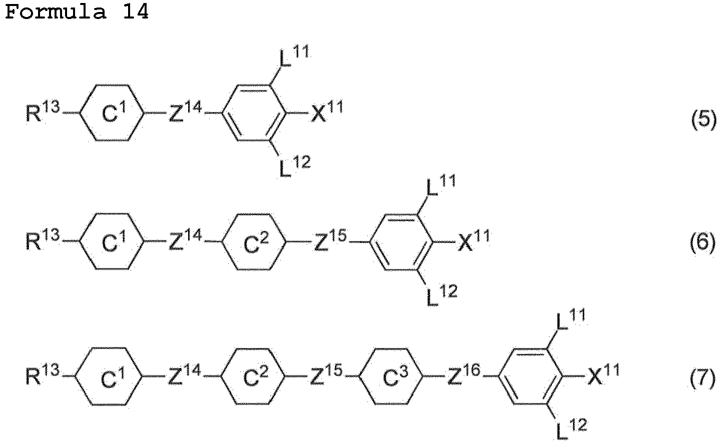 wherein, in formulas (5) to (7),
R¹³ is alkyl having 1 to 10 carbons or alkenyl having 2 to 10 carbons, and in the alkyl and the alkenyl, at least one piece of -CH₂- may be replaced by -0-, and in the groups, at least one hydrogen may be replaced by fluorine;
X¹¹ is fluorine, chlorine, -OCF₃, -OCHF₂, -CF₃, -CHF₂, -CH₂F, -OCF₂CHF₂ or -OCF₂CHFCF₃;
ring C¹, ring C² and ring C³ are independently 1,4-cyclohexylene, 1, 4-phenylene, 1, 4-phenylene in which at least one hydrogen is replaced by fluorine, tetrahydropyran-2,5-diyl, 1,3-dioxane-2,5-diyl or pyrimidine-2,5-diyl;
Z¹⁴, Z¹⁵ and Z¹⁶ are independently a single bond, -COO-, -OCO-, -CH₂O-, -OCH₂-, -CF₂O-, -OCF₂-, -CH₂CH₂-, -CH=CH-, -C≡C- or -(CH₂)₄-; and
L¹¹ and L¹² are independently hydrogen or fluorine.

11. The liquid crystal composition according to any one of claims 8 to 10, further comprising at least one compound represented by formula (8): 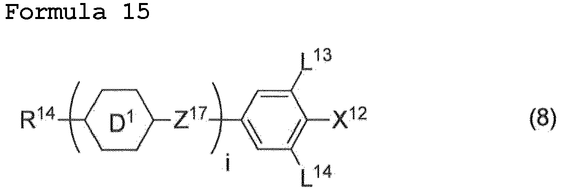 wherein, in formula (8),
R¹⁴ is alkyl having 1 to 10 carbons or alkenyl having 2 to 10 carbons, and in the alkyl and the alkenyl, at least one piece of -CH₂- may be replaced by -0-, and in the groups, at least one hydrogen may be replaced by fluorine;
X¹² is -C≡N or -C≡C-C≡N;
ring D¹ is 1,4-cyclohexylene, 1,4-phenylene, 1,4-phenylene in which at least one hydrogen is replaced by fluorine, tetrahydropyran-2,5-diyl, 1,3-dioxane-2,5-diyl or pyrimidine-2,5-diyl;
Z¹⁷ is a single bond, -COO-, -OCO-, -CH₂O-, -OCH₂-, -CF₂O-, -OCF₂-, -CH₂CH₂- or -C≡C-;
L¹³ and L¹⁴ are independently hydrogen or fluorine; and
i is 1, 2, 3 or 4.

12. The liquid crystal composition according to any one of claims 8 to 11, further comprising at least one compound selected from the group of compounds represented by formulas (11) to (19): 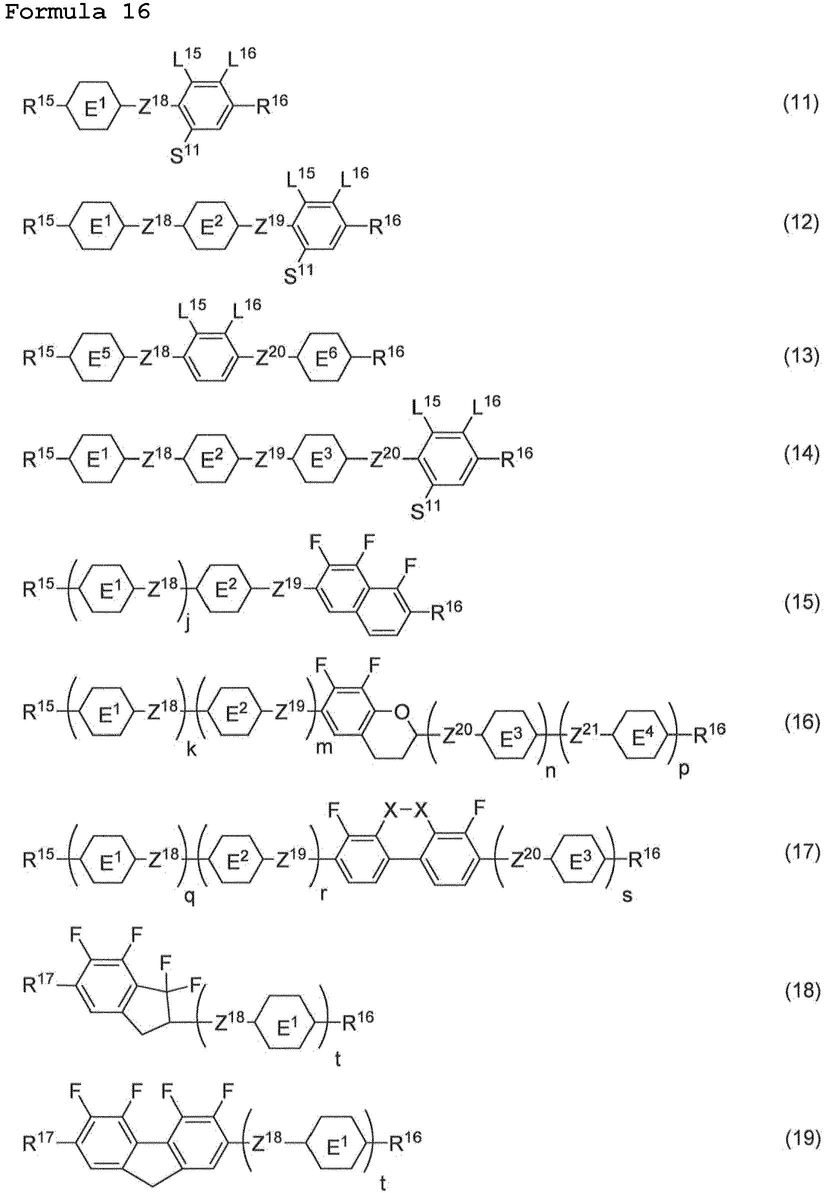 wherein, in formulas (11) to (19),
R¹⁵, R¹⁶ and R¹⁷ are independently alkyl having 1 to 10 carbons or alkenyl having 2 to 10 carbons, and in the alkyl and the alkenyl, at least one piece of -CH₂- may be replaced by -0-, and in the groups, at least one hydrogen may be replaced by fluorine, and R¹⁷ may be hydrogen or fluorine;
ring E¹, ring E², ring E³ and ring E⁴ are independently 1,4-cyclohexylene, 1,4-cyclohexenylene, 1,4-phenylene, 1,4-phenylene in which at least one hydrogen is replaced by fluorine, tetrahydropyran-2,5-diyl or decahydronaphthalene-2,6-diyl;
ring E⁵ and ring E⁶ are independently 1,4-cyclohexylene, 1,4-cyclohexenylene, 1,4-phenylene, tetrahydropyran-2,5-diyl or decahydronaphthalene-2,6-diyl;
Z¹⁸, Z¹⁹, Z²⁰ and Z²¹ are independently a single bond, -COO-, -OCO-, -CH₂O-, -OCH₂-, -CF₂O-, -OCF₂-, -CH₂CH₂-, -CF₂OCH₂CH₂- or -OCF₂CH₂CH₂-;
L¹⁵ and L⁶ are independently fluorine or chlorine;
S¹¹ is hydrogen or methyl;
X is -CHF- or -CF₂-; and
j, k, m, n, p, q, r and s are independently 0 or 1, a sum of k, m, n and p is 1 or 2, a sum of q, r and s is 0, 1, 2 or 3, and t is 1, 2 or 3.

13. The liquid crystal composition according to any one of claims 8 to 12, further comprising at least one compound of polymerizable compounds represented by formula (20): 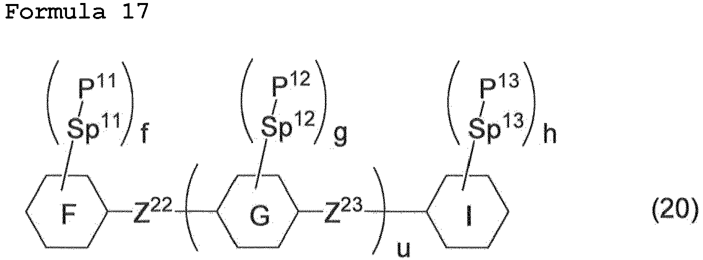 wherein, in formula (20),
ring F and ring I are independently cyclohexyl, cyclohexenyl, phenyl, 1-naphthyl, 2-naphthyl, tetrahydropyran-2-yl, 1,3-dioxane-2-yl, pyrimidine-2-yl or pyridine-2-yl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen;
ring G is 1,4-cyclohexylene, 1,4-cyclohexenylene, 1, 4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl, naphthalene-2,7-diyl, phenanthrene-2,7-diyl, tetrahydropyran-2,5-diyl, 1,3-dioxane-2,5-diyl, pyrimidine-2,5-diyl or pyridine-2,5-diyl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen;
Z²² and Z²³ are independently a single bond or alkylene having 1 to 10 carbons, and in the alkylene, at least one piece of -CH₂- may be replaced by -0-, -CO-, -COO- or -OCO-, and at least one piece of -CH₂CH₂- may be replaced by -CH=CH-, -C(CH₃)=CH-, -CH=C(CH₃)- or -C (CH₃)=C (CH₃)-, and in the groups, at least one hydrogen may be replaced by fluorine or chlorine;
P¹¹, P¹² and P¹³ are independently a polymerizable group;
Sp¹¹, Sp¹² and Sp¹³ are independently a single bond or alkylene having 1 to 10 carbons, and in the alkylene, at least one piece of -CH₂- may be replaced by -0-, -COO-, -OCO- or -OCOO-, and at least one piece of -CH₂CH₂- may be replaced by -CH=CH- or -C≡C-, and in the groups, at least one hydrogen may be replaced by fluorine or chlorine;
u is 0, 1 or 2; and
f, g and h are independently 0, 1, 2, 3 or 4, and a sum of f, g and h is 1 or more.

14. The liquid crystal composition according to claim 13, wherein, in formula (20), P¹¹, P¹² and P¹³ are independently a group selected from the group of polymerizable groups represented by formula (P-1) to formula (P-5): 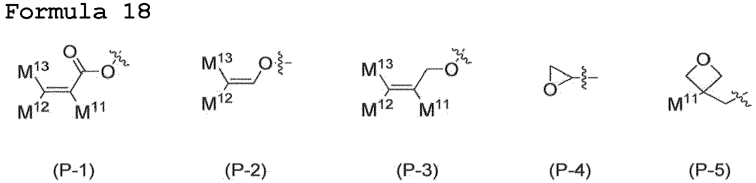 wherein, in formula (P-1) to formula (P-5),
M¹¹, M¹² and M¹³ are independently hydrogen, fluorine, alkyl having 1 to 5 carbons, or alkyl having 1 to 5 carbons in which at least one hydrogen is replaced by halogen.

15. The liquid crystal composition according to claim 13 or 14, wherein a polymerizable compound represented by formula (20) is at least one compound selected from the group of polymerizable compounds represented by formula (20-1) to formula (20-7): 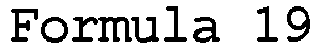 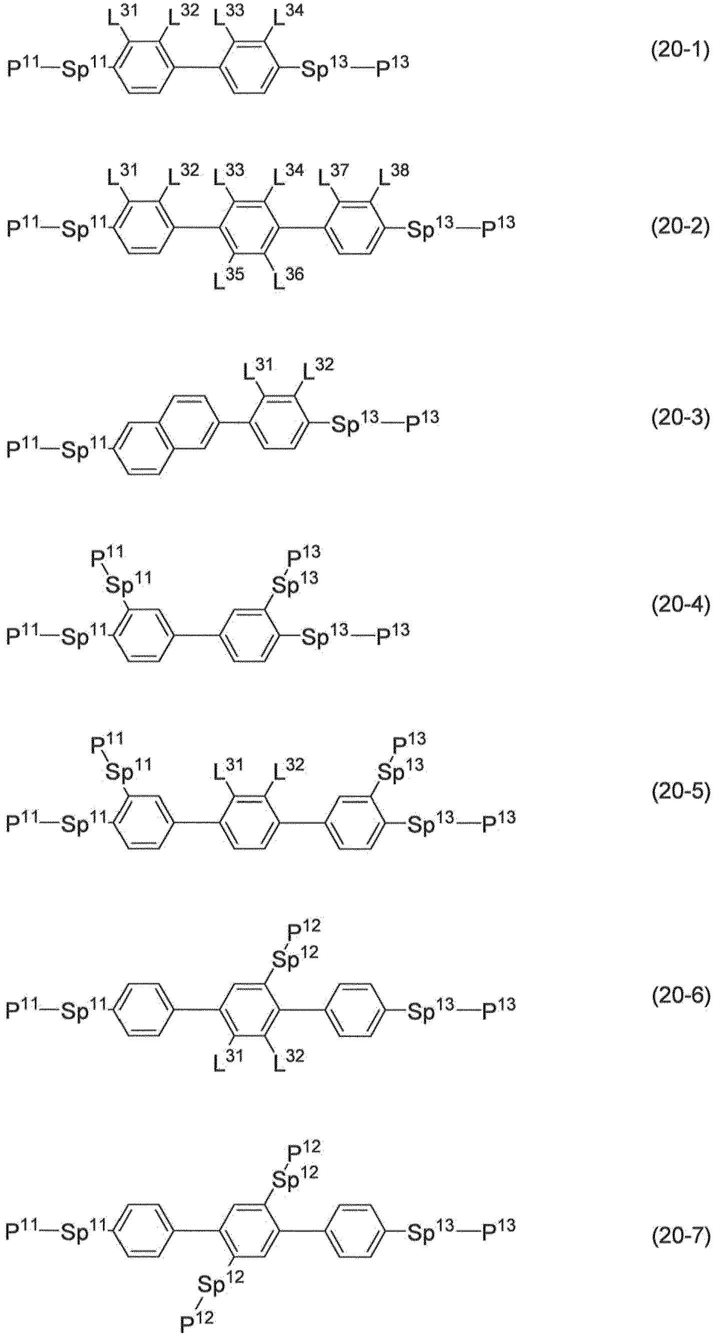 wherein, in formula (20-1) to formula (20-7),
L³¹, L³², L³³, L³⁴, L³⁵, L³⁶, L³⁷ and L³⁸ are independently hydrogen, fluorine or methyl;
Sp¹¹, Sp¹² and Sp¹³ are independently a single bond or alkylene having 1 to 10 carbons, and in the alkylene, at least one piece of -CH₂- may be replaced by -0-, -COO-, -OCO- or -OCOO-, and at least one piece of -CH₂CH₂- may be replaced by -CH=CH- or -C≡C-, and in the groups, at least one hydrogen may be replaced by fluorine or chlorine; and P¹¹, P¹² and P¹³ are independently a group selected from the group of polymerizable groups represented by formula (P-1) to formula (P-3); 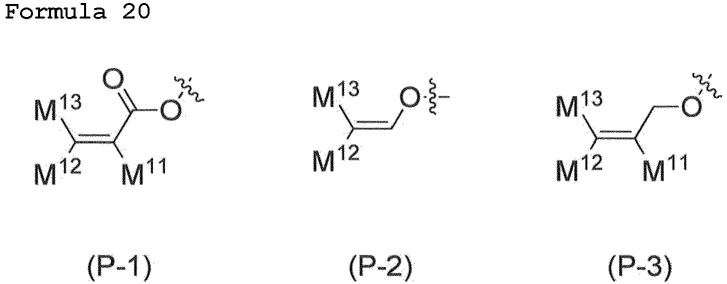 wherein, in formula (P-1) to formula (P-3),
M¹¹, M¹² and M¹³ are independently hydrogen, fluorine, alkyl having 1 to 5 carbons, or alkyl having 1 to 5 carbons in which at least one hydrogen is replaced by halogen.

16. The liquid crystal composition according to any one of claims 8 to 15, further comprising at least a polymerizable compound different from the compound represented by formula (1) or formula (20) described below, a polymerization initiator, a polymerization inhibitor, an optically active compound, an antioxidant, an ultraviolet light absorber, a light stabilizer, a heat stabilizer and an antifoaming agent: 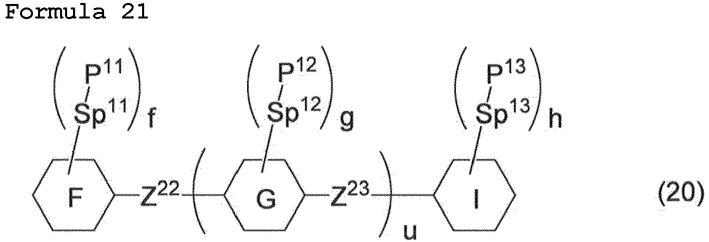 wherein, in formula (20),
ring F and ring I are independently cyclohexyl, cyclohexenyl, phenyl, 1-naphthyl, 2-naphthyl, tetrahydropyran-2-yl, 1,3-dioxane-2-yl, pyrimidine-2-yl or pyridine-2-yl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen;
ring G is 1,4-cyclohexylene, 1,4-cyclohexenylene, 1,4-phenylene, naphthalene-1,2-diyl, naphthalene-1,3-diyl, naphthalene-1,4-diyl, naphthalene-1,5-diyl, naphthalene-1,6-diyl, naphthalene-1,7-diyl, naphthalene-1,8-diyl, naphthalene-2,3-diyl, naphthalene-2,6-diyl, naphthalene-2,7-diyl, phenanthrene-2,7-diyl, tetrahydropyran-2,5-diyl, 1,3-dioxane-2,5-diyl, pyrimidine-2,5-diyl or pyridine-2,5-diyl, and in the rings, at least one hydrogen may be replaced by halogen, alkyl having 1 to 12 carbons, alkoxy having 1 to 12 carbons, or alkyl having 1 to 12 carbons in which at least one hydrogen is replaced by halogen;
Z²² and Z²³ are independently a single bond or alkylene having 1 to 10 carbons, and in the alkylene, at least one piece of -CH₂- may be replaced by -0-, -CO-, -COO- or -OCO-, and at least one piece of -CH₂CH₂- may be replaced by -CH=CH-, -C(CH₃)=CH-, -CH=C(CH₃)- or -C (CH₃)=C (CH₃)-, and in the groups, at least one hydrogen may be replaced by fluorine or chlorine;
P¹¹, P¹² and P¹³ are independently a polymerizable group;
Sp¹¹, Sp¹² and Sp¹³ are independently a single bond or alkylene having 1 to 10 carbons, and in the alkylene, at least one piece of -CH₂- may be replaced by -0-, -COO-, -OCO- or -OCOO-, and at least one piece of -CH₂CH₂- may be replaced by -CH=CH- or -C≡C-, and in the groups, at least one hydrogen may be replaced by fluorine or chlorine;
u is 0, 1 or 2; and
f, g and h are independently 0, 1, 2, 3 or 4, and a sum of f, g and h is 1 or more.

17. A liquid crystal display device, including at least one liquid crystal composition according to any one of claims 8 to 16.
